(19) Europäisches Patentamt
European Patent Office
Office européen des brevets

(11) Numéro de publication: **0 395 528 B1**

# (12) FASCICULE DE BREVET EUROPEEN

(45) Date de publication de fascicule du brevet: **05.07.95**

(51) Int. Cl.[6]: **C07D 209/08**, A61K 31/40, //C07D209/30,C07D405/12

(21) Numéro de dépôt: **90401152.5**

(22) Date de dépôt: **27.04.90**

## (54) Dérivés du 4-phénylméthyl 1H-indole, procédé et intermédiaires de préparation, application à titre de médicaments et compositions les renfermant.

(30) Priorité: **28.04.89 FR 8905650**

(43) Date de publication de la demande:
**31.10.90 Bulletin 90/44**

(45) Mention de la délivrance du brevet:
**05.07.95 Bulletin 95/27**

(84) Etats contractants désignés:
**AT BE CH DE DK ES FR GB GR IT LI LU NL SE**

(56) Documents cités:
**EP-A- 0 209 435**
**EP-A- 0 213 984**

**TETRAHEDRON, vol. 40, no. 12, 1984, pages 2345-2358, Oxford, GB; A.P.KOZIKOWSKI et al.: "The intramolecular nitrile oxide cycloaddition (INOC) route to the ergot alkaloids: use of the isoxazoline to gamma-amino alcohol convertion in the total synthesis of (+)-paliclavine."**

(73) Titulaire: **ROUSSEL-UCLAF**
**35, Boulevard des Invalides**
**F-75007 Paris (FR)**

(72) Inventeur: **Clemence, François**
**2, rue Turgot**
**F-75009 Paris (FR)**
Inventeur: **Guillaume, Jacques**
**62, rue Pixerecourt**
**F-75020 Paris (FR)**
Inventeur: **Hamon, Gilles**
**7, Boulevard de l'Ouest**
**F-93340 Le Raincy (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

EP 0 395 528 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des dérivés du 4-phénylméthyl 1H-indole, le procédé et les intermédiaires de préparation, l'application à titre de médicaments et les compositions les renfermant.

Le document EP-A-0 209 435 décrit des dérivés de l'hydroxy alkoxy 4-phénylpropyl indole qui possèdent les activités anti-arythmique et anticalcique et le document EP-A-0 213 984 décrit des dérivés de l'indole carboxamide qui possèdent une activité anti-arythmique.

L'invention a pour objet les produits de formule générale (I) :

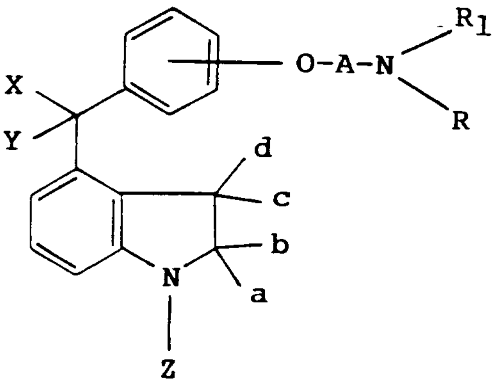

(I)

dans laquelle R et $R_1$ identiques ou différents, représentent :

- soit un atome d'hydrogène,
- soit un radical alkyle, alkényle ou alkynyle linéaire ou ramifié ayant au plus 8 atomes de carbone, éventuellement substitué par un radical hydroxy,
- soit un radical cycloalkyle renfermant de 3 à 7 atomes de carbone, un radical cycloalkylalkyle renfermant de 4 à 7 atomes de carbone ou un radical arylalkyle renfermant de 7 à 14 atomes de carbone, ce dernier radical étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 5 atomes de carbone et les radicaux trifluorométhyle, méthylthio, nitro, amino, monoalkylamino ou dialkylamino,

  ou R et $R_1$ forment ensemble, avec l'atome d'azote auquel ils sont liés, un hétérocycle saturé ou insaturé pouvant renfermer un second hétéroatome choisi parmi les atomes d'oxygène, de soufre ou d'azote, ce second atome d'azote non lié à A étant éventuellement substitué par :
- soit un radical alkyle renfermant de 1 à 5 atomes de carbone,
- soit un radical phényle lui-même éventuellement substitué par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,
- soit un radical naphtyle,
- soit un radical arylalkyle ou diarylalkyle renfermant de 7 à 14 atomes de carbone, chacun de ces radicaux étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,

  A représente :
- soit une chaîne $(CH_2)_n$ dans laquelle n peut prendre les valeurs 2, 3, 4 ou 5
- soit une chaîne

$$-CH_2-\underset{\displaystyle OH}{\underset{|}{CH}}-CH_2-$$

  X et Y sont tels que :
- soit chacun représente un atome d'hydrogène,

- soit l'un représente un atome d'hydrogène et l'autre représente un radical hydroxy, un radical alkoxy renfermant de 1 à 4 atomes de carbone ou un radical alkyle renfermant de 1 à 4 atomes de carbone,
- soit X et Y forment ensemble un radical oxo, un radical alkylidène ayant de 1 à 4 atomes de carbone ou un radical $=N\text{-}OR_5$ dans lequel $R_5$ représente un atome d'hydrogène ou un radical alkyle renfermant de 1 à 4 atomes de carbone,

les substituants a, b, c, d sont tels que :

- soit ils représentent chacun un atome d'hydrogène
- soit a et b forment ensemble une fonction oxo et c et d représentent chacun un atome d'hydrogène
- soit l'un de a ou b forme avec l'un de c ou d une double liaison et les autres substituants représentent chacun un atome d'hydrogène,

Z représente un atome d'hydrogène, un radical alkyle, alkényle ou alkynyle, chacun de ces radicaux linéaire ou ramifié ayant au plus 5 atomes de carbone, un radical cycloalkylalkyle renfermant de 4 à 7 atomes de carbone, un radical arylalkyle renfermant de 7 à 14 atomes de carbone, chacun de ces radicaux étant éventuellement substitué par 1, 2 ou 3 radicaux choisis parmi le groupe constitué par le radical hydroxy, les halogènes, les radicaux alkyle ou alkyloxy linéaires ou ramifiés ayant au plus 5 atomes de carbone,

ou Z représente un groupe aminoalkyle de formule $-R_2\text{-}N\text{-}(R_3)(R_4)$ dans laquelle $R_3$ et $R_4$, identiques ou différents, représentent un atome d'hydrogène, un radical alkyle linéaire ou ramifié renfermant au plus 5 atomes de carbone,

et $R_2$ est un radical alkylène renfermant de 2 à 5 atomes de carbone,

lesdits composés de formule (I) pouvant être sous toutes les formes énantiomères, racémiques ou diastéréoisomères possibles et sous forme de sels d'addition avec les acides minéraux ou organiques.

Dans la formule générale et dans ce qui suit :

- le terme radical alkyle linéaire ou ramifié ayant au plus 8 atomes de carbone désigne, de préférence, un radical méthyle, éthyle, propyle ou isopropyle, butyle, isobutyle, sec-butyle ou tert-butyle, pentyle, isopentyle, sec-pentyle, tert-pentyle ou néo-pentyle,
- le terme radical alkyle renfermant de 1 à 4 atomes de carbone désigne de préférence un radical méthyle, éthyle, propyle ou isopropyle, butyle, isobutyle, sec-butyle ou tert-butyle.

Lorsque l'un de X ou Y représente un radical alkyle ayant de 1 à 4 atomes de carbone, il est choisi de préférence parmi les produits de formule $-CH_2\text{-}R_a$ dans laquelle $R_a$ représente un radical alkyle choisi parmi les radicaux méthyle, éthyle, propyle, isopropyle, c'est-à-dire les radicaux linéaires ou ramifiés ayant de 1 à 3 atomes de carbone.

- le terme radical alkényle linéaire ou ramifié ayant au plus 8 atomes de carbone peut désigner, un radical vinyle, allyle, 1-propényle, butényle, penthényle, hexényle, de préférence ce radical alkényle désigne un radical allyle, butényle, penthényle, hexényle,
- le terme radical alkynyle linéaire ou ramifié ayant au plus 8 atomes de carbone désigne, de préférence, un radical éthynyle, propargyle, butynyle,
- le terme radical alkylidène renfermant de 1 à 4 atomes de carbone désigne de préférence un radical méthylène, éthylidène, propylidène, butylidène,
- le terme radical cycloalkyle renfermant de 3 à 7 atomes de carbone désigne, de préférence, un radical cyclopropyle, cyclobutyle, cyclopentyle, cyclohexyle,
- le terme radical cycloalkylalkyle renfermant de 4 à 12 atomes de carbone désigne, de préférence, un radical cyclopropylméthyle, cyclopropyléthyle, cyclopropylpropyle, cyclobutylméthyle, cyclobutyléthyle, éthylcyclobutylméthyle, cyclobutylpropyle, diisopropylcyclobutyle, cyclopentylméthyle, cyclopentyléthyle, cyclopentylpropyle, diisopropylcyclopentyle, cyclopentylbutyle, cyclopentylpentyle, méthylcyclohexylpentyle,
- les termes radical arylalkyle ou diarylalkyle renfermant de 7 à 14 atomes de carbone désigne, de préférence, un radical benzyle, phénéthyle, alpha-méthyl phénéthyle, phénylpropyle, alpha-méthyl phényl-propyle, béta-méthyl phénylpropyle, phénylbutyle, diphénylméthyle ou 1,1-diphényl éthyle,
- l'expression éventuellement substitué appliquée à la présence d'un ou plusieurs des radicaux précités ainsi qu'aux radicaux phényle et naphtyle désigne de préférence un ou plusieurs substituants choisis parmi les atomes d'halogène, les radicaux alkyle ou alkyloxy linéaires ou ramifiés ayant au plus 5 atomes de carbone, les radicaux hydroxy, trifluorométhyle, méthylthio, nitro, amino, monoalkylamino ou dialkylamino,
- le terme halogène désigne de préférence un atome de fluor, de chlore ou de brome,
- le terme radical alkyloxy linéaire ou ramifié ayant au plus 5 atomes de carbone désigne de préférence un radical méthoxy, éthoxy, propoxy ou tert-butoxy,

- les termes monoalkylamino et dialkylamino désignent, de préférence, des groupes renfermant des radicaux alkyle linéaires ou ramifiés ayant au plus 5 atomes de carbone tels que méthylamino, éthylamino, méthyléthylamino, diméthylamino, diéthylamino ou encore éthylpropylamino,
- le terme hétérocycle saturé ou insaturé que peuvent former $R_1$ et R, d'une part, et $R_3$ et $R_4$, d'autre part, avec l'atome d'azote auquel ils sont liés respectivement, désigne de préférence un radical à 5 ou 6 chaînons pouvant renfermer un second hétéroatome choisi parmi les atomes d'oxygène, d'azote ou de soufre et tout préférentiellement un radical pyrrolidinyle, imidazolidinyle, pyrazolidinyle, pipéridinyle, morpholinyle, pipérazinyle, méthylpipérazinyle, éthylpipérazinyle ou propylpipérazinyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, malonique, maléique, fumarique, succinique, tartrique, citrique, oxalique, glyoxylique, aspartique, alcanesulfoniques tels que les acides méthane ou éthanesulfoniques, arènesulfoniques, tels que les acides benzène ou paratoluène sulfoniques et arylcarboxyliques, tels que benzoïques.

L'invention a particulièrement pour objet les produits de formule (I) telle que définie ci-dessus, dans laquelle Z est un atome d'hydrogène ou un radical alkyle renfermant de 1 à 4 atomes de carbone,
lesdits composés de formule (I) pouvant être sous forme de sels d'addition avec les acides minéraux ou organiques,
les composés de formule (I) telle que définie ci-dessus dans laquelle X et Y représentent un atome d'hydrogène ou forment ensemble un radical oxo ou alkylidène,
lesdits composés de formule (I) pouvant être sous forme de sels d'addition avec les acides minéraux ou organiques,
ainsi que les produits de formule (I) telle que définie ci-dessus, dans laquelle R est un atome d'hydrogène, $R_1$ représente un radical alkyle renfermant de 1 à 4 atomes de carbone ou un radical cycloalkyle renfermant de 3 à 7 atomes de carbone ou R et $R_1$ forment ensemble avec l'atome d'azote auquel ils sont liés un hétérocycle pouvant renfermer un second atome d'azote éventuellement substitué par un radical alkyle renfermant de 1 à 5 atomes de carbone et A est tel que l'entier n a la valeur 2 ou 3 ou A représente le groupe

$$-(CH_2)-\underset{\displaystyle OH}{\underset{|}{CH}}-(CH_2)-$$

lesdits composés de formule (I) pouvant être sous forme de sels d'addition avec les acides minéraux ou organiques.

L'invention a tout aussi particulièrement pour objet les produits de formule (I) telle que définie ci-dessus, dans lesquels a, b, c, d sont tels que l'un de a ou b forme avec l'un de c ou d une seconde liaison carbone-carbone et les autres représentent un atome d'hydrogène, et ceux dans lesquels a et b forment ensemble un radical oxo et c et d représentent chacun un atome d'hydrogène,
lesdits composés de formule (I) pouvant être sous forme de sels d'addition avec les acides minéraux ou organiques.

Parmi les produits de formule (I), on peut citer plus particulièrement les produits décrits ci-aprés dans les exemples et notamment les dérivés de formule (I) telle que définie ci-dessus, dont les noms suivent :

- le [2-[3-[(1,1-diméthyl éthyl) amino] 2-hydroxy propoxy] phényl] (1H-indol-4-yl) méthanone,
- le [2-[3-[(1,1-diméthyl éthyl) amino] propoxy] phényl] (1H-indol-4-yl) méthanone,
- le 1-[(1,1-diméthyl éthyl) amino] 3-[2-[(1H-indol-4-yl) méthyl] phénoxy] 2-propanol,
- le (±) [2-[3-[(1,1-diméthyléthyl) amino] 2-hydroxypropoxy] phényl] (1-méthyl 1H-indol-4-yl) méthanone,
- le 1,3-dihydro 4-[2-[3-[(1,1-diméthyléthyl) amino] 2-hydroxypropoxy] benzoyl] 2H-indol-2-one,
- le (±) 3-[(1,1-diméthyléthyl) amino] 1-[[2-(1H-indol-4-yl) éthényl] phénoxy] 2-propanol,
- le N-(1,1-diméthylethyl) 3-[2-[1-(1H-indol-4-yl) éthényl] phénoxy] propanamine,
- l'alpha [2-[3-[(1,1-diméthyléthyl) amino] 2-hydroxypropoxy] phényl 1H-indol-4-méthanol,

ainsi que leurs sels d'addition avec les acides minéraux ou organiques.

L'invention a également pour objet un procédé de préparation des produits de formule (I) telle que définie ci-dessus, caractérisé en ce que l'on soumet un produit de formule (II) :

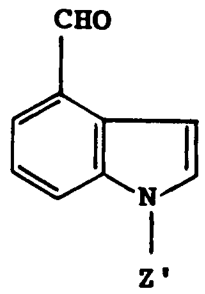

(II)

dans laquelle Z′ représente :

soit les valeurs indiquées ci-dessus pour le radical Z, à l'exception de la valeur hydrogène,

soit lesdites valeurs dans lesquelles les fonctions réactives sont protégées,

soit un groupe protecteur,

à une réaction de condensation avec un organo-métallique de formule (III) :

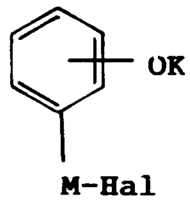

(III)

dans laquelle K représente un groupe protecteur du radical hydroxy, M représente un atome de lithium ou de magnésium, Hal représentant un atome d'halogène, pour obtenir un produit de formule (IV) :

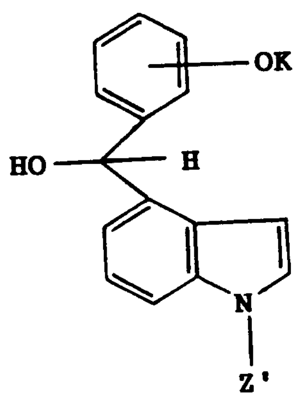

(IV)

que l'on soumet à une réaction d'oxydation pour obtenir le produit de formule (V) :

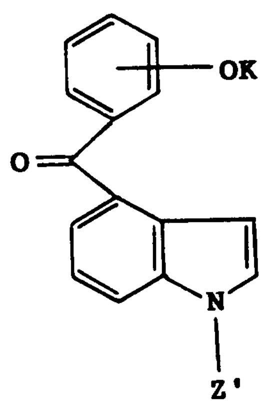

(V)

dont on libère sélectivement le groupe hydroxy pour obtenir le produit de formule (VI) :

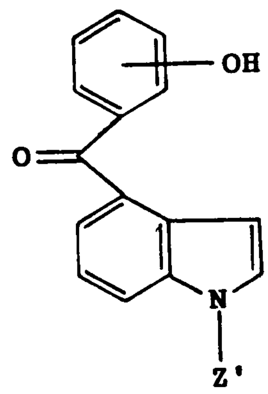

(VI)

et, si désiré, ou bien l'on soumet le produit de formule (VI) à une réaction de réduction de la fonction oxo pour obtenir le produit de formule (VII) :

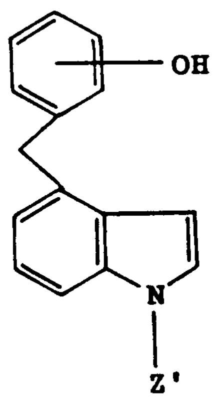

(VII)

ou bien l'on soumet le produit de formule (VI) à l'action d'un réactif organométallique puis à l'action d'un agent de déshydratation pour obtenir un produit de formule (VIII) :

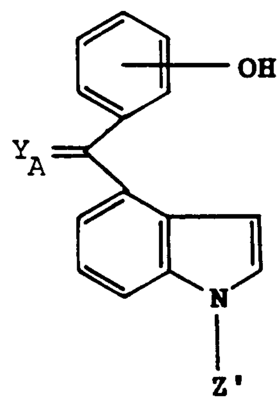

(VIII)

dans laquelle $Y_A$ représente un radical alkylidène renfermant de 1 à 4 atomes de carbone, produit de formule (VIII) que l'on hydrogène si désiré pour obtenir un produit de formule (VIII') :

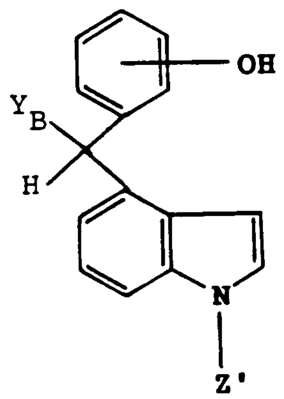

(VIII')

dans laquelle $Y_B$ représente un radical alkyle renfermant de 1 à 4 atomes de carbone, ou bien l'on transforme le produit de formule (VI) en l'oxime correspondante de formule (IX) :

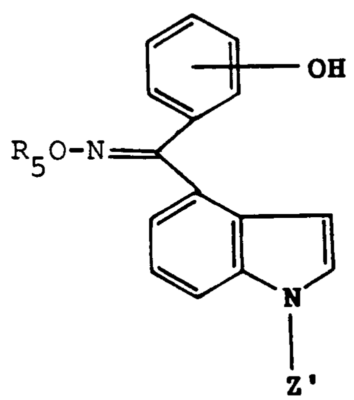

(IX)

dans laquelle $R_5$ a la signification indiquée précédemment, produits de formule (VI), (VII), (VIII), (VIII') ou (IX) que l'on soumet à une réaction de condensation sur le groupe hydroxy,

- soit par le produit de formule (X) :

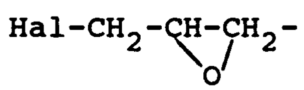

(X)

pour obtenir le produit de formule (XI) :

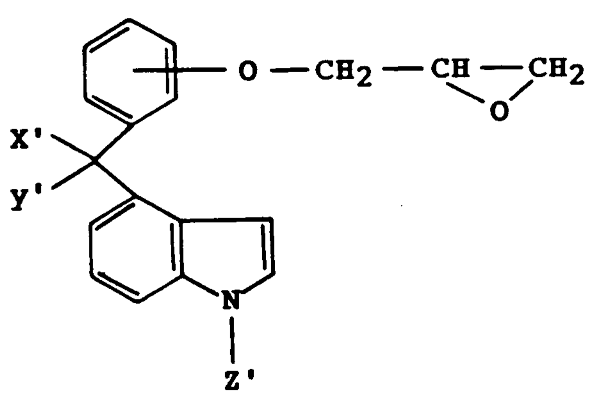

(XI)

dans laquelle X' et Y' représentent un atome d'hydrogène ou forment ensemble une fonction oxo, un radical alkylidène ou un radical $=N-OR_5$, ou l'un représente un atome d'hydrogène et l'autre

représente un radical alkyle renfermant de 1 à 4 atomes de carbone,

- soit par le produit de formule (XII) :

Hal-A'-Hal (XII)

dans laquelle A' représente $-(CH_2)_n$ où n a la signification indiquée ci-dessus pour obtenir le produit de formule (XIII) :

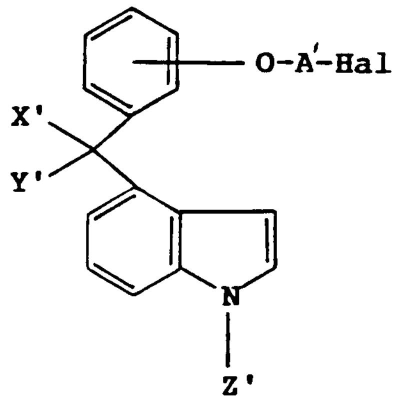

(XIII)

dans laquelle A', Z', X' et Y' ont la signification donnée ci-dessus et soumet les produits de formule (XI) ou (XIII) à une réaction d'addition par l'amine de formule (XIV) :

NH-(R)($R_1$) (XIV)

dans laquelle R et $R_1$ ont la signification indiquée ci-dessus, pour obtenir les produits de formule (XV) :

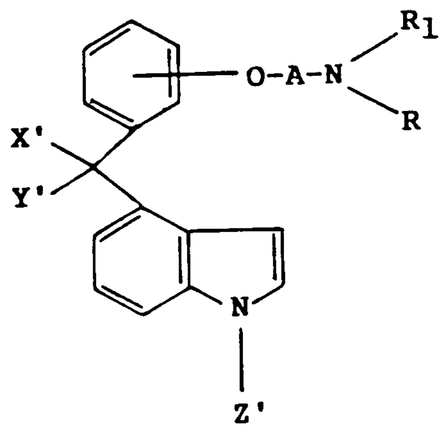

(XV)

que l'on soumet, si nécessaire et si désiré, à l'une ou plusieurs des réactions suivantes, dans un ordre quelconque :

a) coupure éventuelle de Z' ou des groupes protecteurs que porte Z' si nécessaire pour obtenir Z,

b) réduction de la fonction oxo formée par X' et Y' en une fonction alcool, suivie si nécessaire et si désiré d'une alkylation de la fonction hydroxy formée par X ou Y pour obtenir les produits de formule (I) dans laquelle X ou Y représente un radical alkoxy,

c) réduction de la double liaison du noyau pyrrole du radical indole afin d'obtenir les produits de formule (I) dans laquelle a, b, c et d représentent chacun un atome d'hydrogène,

d) halogénation en béta de l'atome d'azote sur le noyau pyrrole du radical indole suivie d'une hydrolyse en milieu acide afin d'obtenir les produits de formule (I) dans laquelle a et b forment une fonction oxo,

e) dédoublement des produits racémiques par des procédés classiques pour obtenir les produits optiquement actifs et salification, si désiré, des produits de formule (I) pour obtenir les sels

correspondants.

Dans le produit de formule (II), Z′ peut représenter un groupe protecteur de l'atome d'azote obtenu par addition d'un halogénure en présence d'un agent basique : dans ce cas, de préférence, ce groupe protecteur est un radical Tosyle (4-méthylbenzène sulfonyle) obtenu par addition de chlorure de Tosyle en milieu basique tel que de la soude à 50 % et de l'hydrogénosulfate de tétrabutylammonium en présence de benzène à la température ambiante pendant environ 1 heure 30.

Les groupes protecteurs des radicaux amino-N($R_3$)($R_4$) que comporte Z, lorsque l'un au moins de $R_3$ ou $R_4$ est un atome d'hydrogène, ou encore d'un radical amino ou monoalkylamino lorsqu'il s'agit d'un substituant de radical arylalkyle, diarylalkyle, phényle ou naphtyle, tels que définis ci-dessus que peut porter le cycle formé, le cas échéant, par $R_3$ ou $R_4$, peuvent être par exemple :

- un groupe acyle éventuellement substitué par exemple par un ou plusieurs atomes d'halogène tel que le chloroacétyle, dichloroacétyle ou trichloroacétyle ou le trifluoroacétyle,
- un groupe alkyloxy ou cycloalkyloxycarbonyle tel que par exemple méthoxycarbonyle ou tert-butoxycarbonyle,
- un groupe aromatique, arylalkyle tel que par exemple benzyle.

La liste ci-dessus n'est pas limitative et d'autres exemples de groupes protecteurs peuvent être trouvés dans le brevet français n° 2 499 995 dont le contenu est incorporé par voie de référence dans la présente demande.

Dans le produit de formule (III) qui représente indifféremment un organolithien ou un organomagnésien, le radical hydroxy protégé par le groupe K peut être un groupe alkyloxy tel que méthoxy, benzyloxy ou encore triméthylsilyloxy.

L'atome d'halogène que représente Hal peut être indifféremment un atome de chlore ou de brome.

La réaction de condensation du produit de formule (II) ou dérivé d'indole-4-carboxaldéhyde avec l'organométallique de formule (III) s'opère, par exemple, dans un solvant qui peut être du THF (Tétrahydrofuranne) à la température de 0 à 5 °C pendant 1 heure.

L'alcool de formule (IV) est soumis à une réaction d'oxydation par exemple dans du chlorochromate de pyridinium ou préférentiellement dans du dichromate de pyridinium dans un solvant inerte tel qu'un halogéno ou un polyhalogénoalcane et préférentiellement du chlorure de méthylène (dichlorométhane) pendant environ 20 heures à la température ambiante.

Le groupe hydroxy protégé du produit de formule (V) peut être libéré, de façon sélective afin de conserver les groupes protecteurs tels que définis ci-dessus, par clivage du groupe protecteur K pour donner le produit de formule (VI) par exemple en présence d'un acide de LEWIS qui peut être du tribromure de bore ou du diméthylsulfure de tribromure de bore dans du dichlorométhane à basse température ou encore, préférentiellement du chlorhydrate de pyridinium à haute température de préférence environ 180 °C.

Le produit de formule (VI) peut être soumis à une réaction de réduction de la fonction oxo pour donner le produit de formule (VII) par exemple par du lithium dans de l'ammoniac liquide ou préférentiellement par l'hydrate d'hydrazine dans du diéthylène glycol en présence de potasse à une température de l'ordre de 130 °C pendant environ 1 heure 30.

- Pour obtenir un produit de formule (VIII), on soumet le produit de formule (VI) à l'action d'un réactif organo-métallique tel qu'un halogénure de méthyl magnésium au sein d'un solvant organique et en opérant par exemple au reflux de ce solvant, puis à une réaction de déshydratation obtenue par exemple en chauffant le milieu réactionnel à 50 °C et en l'acidifiant.
- Le produit de formule (VIII′) peut être obtenu par hydrogénation catalytique en présence de palladium, du produit de formule (VIII).
- Le produit de formule (VI) peut être transformé en oxime de formule (IX) selon des méthodes classiques, par condensation avec l'hydroxylamine ou un dérivé de ce produit de formule $H_2N\text{-}OR_5$.
- Les produits de formule (VI), (VII), (VIII), (VIII′) ou (IX) peuvent être soumis à une réaction d'addition sur le groupe hydroxy du phénol par un dérivé halogéné de formule (X) ou (XII) dans lequel le ou les atomes d'halogène peuvent être indifféremment des atomes de chlore ou de brome :
  soit par une réaction de condensation avec le dérivé halogéné de formule (X) tel que l'épichlorhydrine en présence d'un agent alcalin tel que la soude, la potasse, le carbonate de soude ou préférentiellement le carbonate de potassium au reflux d'un solvant organique tel que l'acétone ou encore une dialkylcétone telle que la méthyléthylcétone ou la méthylisobutylcétone pendant 24 heures environ pour donner les produits de formule (XI),
  soit par une réaction de substitution avec le dérivé dihalogéné de formule (XII) tel que le 1-bromo 3-chloro propane au reflux d'un solvant polaire qui peut être indifféremment un alcool tel que l'éthanol, un dérivé oxo tel que l'acétone ou encore le DMF (diméthylformamide) et en présence d'un agent

basique tel que le carbonate de sodium ou de potassium pour donner les produits de formule (XIII).

L'addition de l'amine de formule (XIV), telle que par exemple la tert-butylamine, sur les composés de formule (XI) ou (XIII) s'opère au reflux pendant environ 2 heures d'un solvant polaire qui peut être un alcool tel que l'éthanol ou encore le DMF en présence ou non d'un agent basique tel que, par exemple, le carbonate de sodium ou de potassium.

Les produits de formule (XV) obtenus, peuvent ou non constituer des produits de formule (I). Ces produits de formule (XV) constituent des produits de formule (I) quand Z′ ne représente pas un groupe protecteur ou ne porte pas de groupe protecteur. Ces produits de formule (XV) peuvent donc être soumis, si nécessaire et si désiré, à l'une ou plusieurs des réactions suivantes, dans un ordre quelconque :

a) coupure éventuelle de Z′ ou des groupes protecteurs que porte Z′ si nécessaire pour obtenir Z par exemple par toute réaction de saponification par un agent tel que le cyanure de sodium en présence de DMSO (Diméthylsulfoxyde) à une température de l'ordre de 100 °C ou préférentiellement la potasse alcoolique.

b) réduction de la fonction oxo formée par X′ et Y′ en une fonction alcool par exemple par un hydroborure alcalin tel que le tétrahydroborure de potassium ou préférentiellement de sodium en milieu alcoolique tel que le méthanol ou préférentiellement le butanol au reflux ou encore par l'hydrure d'aluminium et de lithium dans du THF ou de l'éther.

c) l'alkylation éventuelle de la fonction hydroxyle que l'un de X′ ou Y′ peut représenter est effectuée de préférence par action d'un dérivé réactif du radical alkyle, par exemple le sulfate de méthyle.

d) réduction de la double liaison du noyau pyrrole du radical indole par des procédés classiques afin d'obtenir les produits de formule (I) dans laquelle a, b, c et d représentent chacun un atome d'hydrogène. Cette réduction peut être obtenue :

- par hydrogénation catalytique en présence de platine sous légère pression d'hydrogène,
- par le diborane dans un solvant polaire tel que le THF suivi par un traitement dans une solution d'alcoolate de sodium ou de potassium dans un alcool, de préférence du méthylate de sodium dans le méthanol,
- ou, également, par des complexes du diborane qui peuvent être par exemple un aminoborane tel que le pyridineborane ou le triméthylaminoborane dans un alcool tel que l'éthanol ou encore le dioxanne en présence d'acide chlorhydrique.

e) halogénation en béta de l'atome d'azote sur le noyau pyrrole du radical indole par les procédés classiques suivie d'une hydrolyse en milieu acide afin d'obtenir les produits de formule (I) dans laquelle a et b forment une fonction oxo : l'halogénation peut être obtenue dans un N-halosuccinimide tel que le N-chlorosuccinimide (NCS) ou le N-bromosuccinimide dans un solvant organique tel que le dioxanne ou l'acide acétique et l'hydrolyse peut être réalisée en milieu d'acide chlorhydrique ou d'acide phosphorique aqueux.

f) dédoublement des produits racémiques par des procédés classiques pour obtenir les produits optiquement actifs et salification, si désiré, également par des méthodes connues, des produits de formule (I) pour obtenir les sels correspondants.

Parmi ces méthodes de salification, peuvent être citées, par exemple, celles qui utilisent le caractère basique des produits de formule (I) : ainsi on peut avantageusement préparer les sels d'addition des dérivés de formule (I) en faisant réagir, en proportions sensiblement stoéchiométriques, un acide minéral ou organique avec ledit dérivé de formule (I). Les sels peuvent être préparés sans isoler les bases correspondantes.

Les produits de formule (II) telle que définie ci-dessus sont connus ou peuvent être préparés comme indiqué, notamment, dans J. Org. Chem. (1980), 45 p. 3350 et suivantes ou encore sont nouveaux et peuvent être préparés par addition d'un halogénure de formule (XVI) :

Hal-Z′ (XVI)

dans laquelle Z′ a la signification indiquée ci-dessus et Hal représente préférentiellement un atome d'iode sur le produit connu de formule (XVII) ou (1H)-indole-4-carboxaldéhyde :

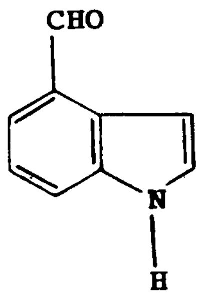

(XVII)

dans un mélange biphasique d'halogénure d'alkyle tel que le dichlorométhane et d'un milieu alcalin par exemple la soude aqueuse et d'un sel d'ammonium quaternaire tel que l'hydrogénosulfate de tétrabutyleammonium. Un exemple de cette préparation est donnée dans la partie expérimentale.

Les produits organo-métalliques de formule (III) sont des produits connus qui peuvent être préparés, par exemple, à partir du 1-bromo 4-méthoxy benzène ou 4-bromo anisole commercialisé sur lequel est ajouté un atome de magnésium en présence de THF.

Les dérivés, objet de la présente invention, possèdent de très intéressantes propriétés pharmacologiques ; ils sont doués notamment de remarquables propriétés anti-arythmiques et bloquantes des canaux calcisodiques lents (propriété anti-calcique). Certains possèdent en outre des propriétés anti-agrégantes plaquettaires et béta-bloquantes.

Ces propriétés sont illustrées plus loin dans la partie expérimentale.

Ces propriétés justifient l'utilisation des dérivés du 4-phénylméthyl 1H-indole de formule (I), ainsi que de leurs sels pharmaceutiquement acceptables à titre de médicaments.

Les médicaments selon l'invention trouvent leur emploi dans le traitement de l'insuffisance cardiaque et des arythmies, des douleurs migraineuses et de l'angine de poitrine sous toutes ses formes aussi bien dans le cas d'angor spastique que d'angor instable. Les médicaments selon l'invention sont également utilisables dans le cas de traitement anti-thrombotique.

La présente demande a également pour objet l'application, à titre de médicaments, des dérivés du 4-phénylméthyl 1H-indole, tels que définis par la formule (I), ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du 4-phénylméthyl 1H-indole répondant à la formule (I) dans laquelle R et $R_1$, identiques ou différents, représentent :

- soit un atome d'hydrogène,
- soit un radical alkyle, alkényle ou alkynyle linéaire ou ramifié ayant au plus 8 atomes de carbone, éventuellement substitué par un radical hydroxy,
- soit un radical cycloalkyle renfermant de 3 à 7 atomes de carbone, un radical cycloalkylalkyle renfermant de 4 à 7 atomes de carbone ou un radical arylalkyle renfermant de 7 à 14 atomes de carbone, ce dernier radical étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 5 atomes de carbone et les radicaux trifluorométhyle, méthylthio, nitro, amino, monoalkylamino ou dialkylamino,

  ou R et $R_1$ forment ensemble, avec l'atome d'azote auquel ils sont liés, un hétérocycle saturé ou insaturé pouvant renfermer un second hétéroatome choisi parmi les atomes d'oxygène, de soufre ou d'azote, ce second atome d'azote non lié à A étant éventuellement substitué par :
- soit un radical alkyle renfermant de 1 à 5 atomes de carbone,
- soit un radical phényle lui-même éventuellement substitué par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,
- soit un radical naphtyle
- soit un radical arylalkyle ou diarylalkyle renfermant de 7 à 14 atomes de carbone, chacun de ces radicaux étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,

  et dans laquelle Z représente un atome d'hydrogène, un radical alkyle, alkényle ou alkynyle, chacun

de ces radicaux linéaire ou ramifié ayant au plus 5 atomes de carbone, un radical cycloalkylalkyle renfermant de 4 à 7 atomes de carbone, un radical arylalkyle renfermant de 7 à 14 atomes de carbone, chacun de ces radicaux étant éventuellement substitué sur le noyau aromatique par 1, 2 ou 3 radicaux choisis parmi le groupe constitué par le radical hydroxy, les halogènes, les radicaux alkyle ou alkyloxy linéaires ou ramifiés ayant au plus 5 atomes de carbone, ou Z représente un groupe amino-alkyle de formule $-R_2-N-(R_3)(R_4)$ dans laquelle $R_3$ et $R_4$, identiques ou différents, représentent un atome d'hydrogène, un radical alkyle linéaire ou ramifié renfermant au plus 5 atomes de carbone, et $R_2$ a la signification indiquée ci-dessus, ainsi que leurs sels d'addition avec les acides pharmaceutiquement acceptables.

Parmi les médicaments, objet de l'invention, on retient également les médicaments caractérisés en ce qu'ils sont constitués par les nouveaux dérivés du 4-phénylméthyl 1H-indole répondant à la formule (I) dans laquelle Z est un atome d'hydrogène ou un radical alkyle renfermant de 1 à 4 atomes de carbone, ceux dans laquelle X et Y représentent un atome d'hydrogène ou forment ensemble un radical oxo ou alkylidène, ceux dans laquelle R est un atome d'hydrogène, $R_1$ représente un radical alkyle renfermant de 1 à 4 atomes de carbone ou un radical cycloalkyle renfermant de 3 à 7 atomes de carbone ou R et $R_1$ forment ensemble avec l'atome d'azote auquel ils sont liés un hétérocycle pouvant renfermer un second atome d'azote éventuellement substitué par un radical alkyle renfermant de 1 à 5 atomes de carbone et dans laquelle R est un atome d'hydrogène, $R_1$ représente un radical alkyle renfermant de 1 à 4 ato- mes de carbone et A est tel que l'entier n a la valeur 2 ou 3 ou A représente le groupe

$$-(CH_2)-\underset{\displaystyle OH}{\underset{|}{CH}}-(CH_2)-,$$

ainsi que leurs sels d'addition avec les acides pharmaceutiquement acceptables.

Parmi les médicaments, objet de l'invention, on retient particulièrement les médicaments caractérisés en ce qu'ils sont constitués par les nouveaux dérivés du 4-phénylméthyl 1H-indole répondant à la formule (I) dans lesquels a, b, c, d sont tels que l'un de a ou b forme avec l'un de c ou d une seconde liaison carbone-carbone et les autres représentent un atome d'hydrogène, et ceux dans lesquels a et b forment ensemble un radical oxo et c et d représentent chacun un atome d'hydrogène, ainsi que leurs sels d'addition avec les acides pharmaceutiquement acceptables.

Parmi les médicaments, objet de l'invention, on retient tout particulièrement les nouveaux dérivés du 4-phénylméthyl 1H-indole répondant à la formule (I), dont les noms suivent :

- le [2-[3-[(1,1-diméthyl éthyl) amino] 2-hydroxy propoxy] phényl] (1H-indol-4-yl) méthanone
- le [2-[3-[(1,1-diméthyl éthyl) amino] propoxy] phényl] (1H-indol-4-yl) méthanone
- le 1-[(1,1-diméthyl éthyl) amino] 3-[2-[(1H-indol-4-yl) méthyl] phénoxy] 2-propanol,
- le (±) [2-[3-[(1,1-diméthyléthyl) amino] 2-hydroxypropoxy] phényl] (1-méthyl 1H-indol-4-yl) méthanone,
- le 1,3-dihydro 4-[2-[3-[(1,1-diméthyléthyl) amino] 2-hydroxypropoxy] benzoyl] 2H-indol-2-one,
- le (±) 3-[(1,1-diméthyléthyl) amino] 1-[[2-(1H-indol-4-yl) éthényl] phénoxy] 2-propanol,
- le N-(1,1-diméthyléthyl) 3-[2-[1-(1H-indol-4-yl) éthényl] phénoxy] propanamine,
- l'alpha [2-[3-[(1,1-diméthyléthyl) amino] 2-hydroxypropoxy] phényl 1H-indol-4-méthanol,

ainsi que leurs sels d'addition avec les acides pharmaceutiquement acceptables.

La dose usuelle, variable selon le dérivé utilisé, le sujet et l'affection en cause peut être par exemple, de 50 mg à 1 g par jour. Par voie orale, chez l'homme, le dérivé de l'exemple 4 peut être administré à la dose quotidienne de 300 mg à 600 mg, par exemple pour le traitement des troubles du rythme cardiaque, soit environ de 5 mg à 10 mg par kilogramme de poids corporel.

L'invention a enfin pour objet les compositions pharmaceutiques qui renfermant au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux

ou non, les corps gras d'origine animale ou végétale, les dérivés paraffiniques, les glycols, les divers agents mouillants, dispersants ou émulsifiants, les conservateurs.

L'invention s'étend également aux produits industriels nouveaux, utiles notamment pour la préparation des dérivés répondant à la formule (I), les produits intermédiaires répondant aux formules (II), (IV), (V), (VI), (VII), (VIII), (VIII'), (IX), (XI) et (XIII) tels que définis ci-dessus.

Les exemples qui suivent illustrent la présente invention.

**EXEMPLE 1 : Fumarate neutre de [4-[3-[(1,1-diméthyl éthyl) amino] propoxy] phényl] (1H-indol-4-yl) méthanone**

STADE A : 1-[(4-méthyl phényl) sulfonyl] 1H-indol-4-carboxaldehyde.

A une suspension de 20 g d'indole 4-carboxaldéhyde dans 600 $cm^3$ de benzène, on ajoute 150 $cm^3$ de soude à 50 %, 4,75 g d'hydrogénosulfate de tétrabutylammonium et 30,24 g de chlorure de paratoluène sulfonyl. On agite pendant 1 h 30 à température ambiante. On décante, extrait avec du benzène, lave à l'eau, sèche et évapore à sec sous pression réduite. Le résidu, 42,1 g, est dissous dans 210 $cm^3$ de benzène au reflux. On laisse refroidir 16 heures à température ambiante et essore. On obtient 23,96 g de produit recherché. F = 144-146 °C.

Le produit est utilisé tel quel pour le stade suivant.

Spectre IR ($CHCl_3$)
Absence de NH
C=O aldéhyde 1691 $cm^{-1}$
C-H aldéhyde 2740 $cm^{-1}$

STADE B : alpha-(4-méthoxy phényl) 1-[(4-méthyl phényl) sulfonyl] 1H-indole-4-méthanol

A 226 $cm^3$ d'une solution de magnésien de 4-bromo anisole dans le tétrahydrofuranne titrant 1,77 M/L on ajoute lentement à 0/+5 °C une solution de 60 g du produit obtenu au stade précédent dans 600 $cm^3$ de tétrahydrofuranne. On agite 1 heure à 0/+5 °C, ajoute 250 $cm^3$ d'une solution saturée de chlorure d'ammonium en maintenant la température à 5 °C, extrait avec du chlorure de méthylène et amène à sec sous pression réduite. On obtient 114 g de produit que l'on empâte dans 150 $cm^3$ d'éther isopropylique et recueille 78,2 g de produit recherché F = 90 °C.

Un échantillon analytique à été obtenu en recristallisant 1 g du produit obtenu ci-dessus dans 10 $cm^3$ d'isopropanol on a obtenu 0,93 g de produit. F = 90 °C.

SPECTRE IR
Absence de C=O
OH à 3601 $cm^{-1}$

| | |
|---|---|
| | (1612 $cm^{-1}$ |
| C=C | )1599 " |
| aromatique | (1586 " |
| | (1528 " |
| | )1511 " |
| | (1495 " |

STADE C : (4-méthoxy phényl) [1-[(4-méthyl phényl) sulfonyl] 1H-indol-4-yl] méthanone.

A une solution de 78 g d'alcool obtenu au stade B dans 1500 $cm^3$ de chlorure de méthylène on ajoute 15,6 g de siliporite NK 30 et 105,4 g de dichromate de pyridinium. On agite 20 h à température ambiante, filtre et concentre à sec sous pression réduite. On chromatographie le résidu sur silice (éluant : chlorure de méthylène) et obtient 71,9 g de produit recherché, F = 135 °C, utilisé tel quel pour le stade suivant.

SPECTRE IR
C=O 1646 $cm^{-1}$
C=C et aromatique : 1600, 1573, 1510, 1495 $cm^{-1}$.

STADE D : (1H-indol-4yl) (4-hydroxy phényl) méthanone

On chauffe pendant 16 h à 140 °C une solution comprenant 56,1 g du produit obtenu au stade précédent, 66,16 g de cyanure de sodium et 370 $cm^3$ de diméthylsufoxyde. On laisse revenir à température ambiante et ajoute 1500 $cm^3$ d'acide chlorhydrique 2 N, extrait avec de l'éther sulfurique et concentre à sec sous pression réduite. On chromatographie le résidu (70,4 g) sur silice (éluant : chlorure de méthylène-acétate d'éthyle (85-15)). On obtient 20,1 g du produit recherché F = 177 °C. Ce produit est utilisé tel quel pour le stade suivant.

Après chromatographie sur silice de 5,5 g du produit ci-dessus (éluant : acétate d'éthyle-n hexane (4-6)) et recristallisation du résidu dans l'acétate d'éthyle-n hexane, on a recueilli 3,96 g de produit pur. F = 177 °C.

| Analyse pour $C_{15}H_{11}NO_2$ | | | | | | |
|---|---|---|---|---|---|---|
| Calculés<br>Trouvés | C% | 75,94<br>75,7 | H% | 4,67<br>4,6 | N% | 5,9<br>5,8 |

STADE E : [4-(3-chloro propoxy) phényl] (1H-indol-4-yl) méthanone

On agite au reflux pendant 4 h 30 un mélange de 5,93 g du produit obtenu au stade précédent, 6,91 g de carbonate de potassium, 150 $cm^3$ d'acétone et 9,84 g de 1-bromo 3-chloro propane. On laisse revenir à température ambiante, filtre, lave à l'acétone et concentre à sec sous pression réduite. On chromatographie le résidu (14,2 g) sur silice (éluant : chlorure de méthylène) et obtient 7,8 g de produit recherché, F = 86 °C, que l'on utilise tel quel pour le stade suivant.

SPECTRE IR
pas d' OH
NH indole 3479 $cm^{-1}$
>C=O 1640 $cm^{-1}$
aromatique : 1601, 1575, 1509 $cm^{-1}$

STADE F : [4-[3-[(1,1-diméthyl éthyl) amino] propoxy] phényl] (1H-indol-4-yl) méthanone

On chauffe à 140 °C pendant 48 h en autoclave un mélange de 7,75 g du produit obtenu au stade précédent, 6,83 g de carbonate de potassium, 25,8 $cm^3$ de tert-butylamine et 150 $cm^3$ d'éthanol. On filtre, amène à sec sous pression réduite et chromatographie le résidu (11,7 g) sur silice (éluant : acétate d'éthyle-triéthylamine (95/05)). On obtient 6,32 g de produit recherché.

STADE G : Fumarate neutre de [4-[3-[(1,1-diméthyl éthyl) amino] propoxy] phényl] (1H-indol-4-yl) méthanone

On dissout 5,45 g du produit obtenu précédemment dans 60 $cm^3$ d'éthanol, filtre la solution puis ajoute une solution de 1,8 g d'acide fumarique dans 40 $cm^3$ d'éthanol. On glace, essore 4,24 g de produit cristallisé que l'on purifie par recristallisation par chaud et froid dans 600 $cm^3$ d'éthanol, on filtre, concentre à demi volume, glace, essore, rince à l'éther, sèche à 80 °C sous pression réduite et obtient 3,62 g de produit pur. F = 210 °C.

| Analyse pour : $2[C_{22}H_{26}N_2O_2]C_4H_4O_4$ | | | | | | |
|---|---|---|---|---|---|---|
| Calculés<br>Trouvés | C% | 70,56<br>70,50 | H% | 6,91<br>6,90 | N% | 6,86<br>6,90 |

**<u>EXEMPLE 2</u> : Fumarate neutre de [4-[3-[(1,1-diméthyl éthyl) amino] 2-hydroxy propoxy] phényl] (1H-indol-4-yl) méthanone**

<u>STADE A</u> : (1H-indol-4-yl) [4-[(2-oxirannyl) méthoxy] phényl] méthanone

On agite pendant 3 heures au reflux un mélange de 2,37 g du produit obtenu au stade D de l'exemple 1 avec 2,76 g de carbonate de potassium, 1,18 $cm^3$ d'épichlorhydrine et 40 $cm^3$ d'acétone. On ajoute alors 1,18 $cm^3$ d'épichlorhydrine et agite au reflux 5 h supplémentaires. On ajoute alors 1,18 $cm^3$ d'épichlorhydrine et agite au reflux. Après 24 h de reflux, on filtre et amène à sec sous pression réduite. On chromatographie le résidu (5,34g) sur silice (éluant : acétate d'éthyle-hexane (1-1)) et obtient 2,82 g de produit recherché.
F = 96°C.
SPECTRE IR
NH indole 3480 $cm^{-1}$
Cétone 1640 $cm^{-1}$
Aromatiques : 1600, 1576, 1509, 1497 $cm^{-1}$

<u>STADE B</u> : [4-[3-[(1,1-diméthyl éthyl) amino] 2-hydroxy propoxy] phényl] (1H-indol-4-yl) méthanone

On agite 2 heures au reflux une solution de 4,65 g d'époxyde obtenu au stade précédent, 100 $cm^3$ d'éthanol et 9,94 $cm^3$ de tert-butylamine. 0n distille à sec et chromatographie le résidu (6,83 g) sur silice (éluant : acétate d'éthyle-triéthylamine (9-1)), on obtient 4,77 g de produit recherché.

<u>STADE C</u> : Fumarate neutre de [4-[3-[(1,1-diméthyl éthyl) amino] 2-hydroxy propoxy] phényl] (1H-indol-4-yl) méthanone

On opère comme au stade G de l'exemple 1 à partir de 4,6 g du produit obtenu précédemment et 1,46 g d'acide fumarique.
On obtient 3,4 g de produit attendu.
F = 210°C.

| Analyse pour $2[C_{22}H_{26}N_2O_3]C_4H_4O_4$ | | | | | | |
|---|---|---|---|---|---|---|
| Calculés<br>Trouvés | C% | 67,9<br>67,2 | H% | 6,65<br>6,6 | N% | 6,6<br>6,2 |

**<u>EXEMPLE 3</u> : Benzoate de [2-[3-[(1,1-diméthyl éthyl) amino] propoxy] phényl] (1H-indol-4-yl) méthanone**

<u>STADE A</u> : 4-[[hydroxy (2-méthoxy phényl) méthyl] 1-[(4-méthyl phényl) sulfonyl] 1H-indole

On opère comme au stade B de l'exemple 1 à partir de 80 g du produit obtenu au stade A de l'exemple 1 en utilisant 207 $cm^3$ d'une solution de magnésien de 2-bromo anisole dans le tétrahydrofuranne titrant 2,28 M/L. On recueille 109 g de produit attendu utilisé tel quel pour le stade suivant.
SPECTRE IR
Absence de >C=O
OH à 3600 $cm^{-1}$
Aromatiques : 1600, 1589, 1529, 1491, 1484 $cm^{-1}$
$SO_2$ 1773, 1178 $cm^{-1}$

<u>STADE B</u> : 4-(2-méthoxy benzoyl) 1-[(4-méthyl phényl) sulfonyl] 1H-indole

On opère comme au stade C de l'exemple 1 à partir de 108,9 g d'alcool obtenu au stade A avec 150,8 g de dichromate de pyridinium. On obtient 101,4 g de produit recherché, F = 154°C, utilisé tel quel pour le stade suivant.
SPECTRE IR
pas d'OH

>C=O 1657 $cm^{-1}$
C=C et aromatiques : 1599, 1580, 1521, 1488 $cm^{-1}$

STADE C : 4-(2-hydroxy benzoyl) 1-[(4-méthyl phényl) sulfonyl] 1H-indole

On chauffe à 180°C pendant 4 heures un mélange de 85 g du produit obtenu au stade précédent et 600 g de chlorhydrate de pyridine. On refroidit à 80°C et ajoute 700 $cm^3$ d'une solution saturée de carbonate de sodium, extrait avec de l'acétate d'éthyle, lave avec 2 fois 300 $cm^3$ d'acide chlorhydrique N puis à l'eau, sèche et évapore à sec sous pression réduite. On chromatographie le résidu (100,3 g) sur silice (éluant : chlorure de méthylène-hexane (8-2)). On obtient 78,8 g de produit attendu utilisé tel quel pour le stade suivant.
F = 110°C.
SPECTRE IR
Absorption générale du type -OH chélaté
>C=O 1626 $cm^{-1}$
C=C + aromatiques : 1606, 1594, 1583, 1524, 1485 $cm^{-1}$

STADE D : (2-hydroxy phényl) (1H-indol-4-yl) méthanone

On agite au reflux pendant 1 h une solution de 84,2 g du produit obtenu au stade précédent et 850 $cm^3$ de potasse éthanolique à 10 %. On évapore le méthanol et reprend le résidu avec une solution aqueuse saturée de chlorure de sodium ; on extrait avec de l'acétate d'éthyle, lave à l'eau, sèche et évapore à sec. On chromatographie le résidu (63,8 g) sur silice et recueille 49,6 g de produit que l'on empâte dans 150 $cm^3$ d'éther éthylique. On obtient 40,9 g de produit recherché. F = 165°C.
SPECTRE IR
NH indole 3479 $cm^{-1}$
Absorption générale type -OH chélaté
>C=O 1626 $cm^{-1}$
aromatiques : 1613, 1598, 1575, 1503, 1425 $cm^{-1}$

STADE E : [2-(3-chloro propoxy) phényl] (1H-indol-4-yl) méthanone

On opère comme au stade E de l'exemple 1 à partir de 10,9 g de produit obtenu au stade précédent en utilisant 18,17 $cm^3$ de 1-bromo 3-chloro propane.
On chromatographie sur silice (éluant : chlorure de méthylène-hexane (75-25)) et obtient 14,4 g de produit recherché, F = 94°C, que l'on utilise tel quel pour le stade suivant.
SPECTRE IR
C=O 1648 $cm^{-1}$
NH indole 3479 $cm^{-1}$
aromatiques : 1609, 1599, 1581, 1571, 1489 $cm^{-1}$

STADE F : [2-[3-[(1,1-diméthyl éthyl) amino] propoxy] phényl] (1H-indol-4-yl) méthanone

On opère comme au stade F de l'exemple 1 à partir de 14,3 g du produit obtenu au stade précédent.
On recueille 12,12 g de produit attendu. F = 130°C.
SPECTRE IR
NH indole 3479 $cm^{-1}$
>C=O 1649 $cm^{-1}$
aromatiques et conjugués : 1609, 1599, 1581, 1572, 1488 $cm^{-1}$

STADE G : Benzoate de [2-[3-[(1,1-diméthyl éthyl) amino] propoxy] phényl] (1H-indol-4-yl) méthanone

On opère comme au stade G de l'exemple 1 à partir de 3,0 g du produit obtenu au stade précédent en utilisant 1,05 g d'acide benzoïque. On obtient 3,42 g de produit, F = 162°C que l'on recristallise dans l'isopropanol. On recueille 2,73 g de produit recherché pur. F = 162°C.

| Analyse pour : $C_{22}H_{26}N_2O_2$, $C_7H_6O_2$ | | | | | | |
|---|---|---|---|---|---|---|
| Calculés<br>Trouvés | C % | 73,71<br>73,7 | H % | 6,82<br>6,9 | N % | 5,93<br>5,9 |

**EXEMPLE 4 : Fumarate neutre de [2-[3-[(1,1-diméthyl éthyl) amino] 2-hydroxy propoxy] phényl] (1H-indol-4-yl) méthanone**

STADE A : (1H-indol-4-yl) [2-[(2-oxirannyl) méthoxy] phényl] méthanone

On opère comme au stade A de l'exemple 2 à partir de 10,9 g du produit obtenu au stade D de l'exemple 3 en utilisant 3 fois 18 $cm^3$ d'épichlorhydrine. On obtient 12,3 g du produit recherché que l'on utilise tel quel pour le stade suivant.

SPECTRE IR
NH indole 3479 $cm^{-1}$
>C=O 1649 $cm^{-1}$
aromatiques : 1609, 1600, 1581, 1571 $cm^{-1}$

STADE B : 2-[3-[(1,1-diméthyl éthyl) amino] 2-hydroxy propoxy] phényl] (1H-indol-4-yl) méthanone

On opère comme au stade B de l'exemple 2 à partir de 12,3 g du produit obtenu au stade précédent. On obtient 12,45 g du produit recherché, F = 130 °C, que l'on utilise tel quel pour le stade suivant.

STADE C : Fumarate neutre de [2-[3-[(1,1-diméthyl éthyl) amino] 2-hydroxy propoxy] phényl] (1H-indol-4-yl) méthanone

On opère comme au stade G de l'exemple 1 à partir de 3,0 g du produit obtenu au stade précédent et 1,19 g d'acide fumarique en opérant dans l'éthanol. On obtient 2,05 g du produit recherché. F = 245 °C.

| Analyse pour $[C_{22}H_{26}N_2O_3]_2$, $C_4H_4O_4$ | | | | | | |
|---|---|---|---|---|---|---|
| Calculés<br>Trouvés | C % | 67,91<br>67,8 | H % | 6,65<br>6,5 | N % | 6,6<br>6,4 |

**EXEMPLE 5 : Benzoate de 1-[(1,1-diméthyl éthyl) amino] 3-[2-[(1H-indol-4-yl) méthyl] phénoxy] 2-propanol**

STADE A : 2-[(1H-indol-4-yl) méthyl] phénol

On agite 30 minutes à 140 °C un mélange de 22,74 g du produit obtenu au stade D de l'exemple 3 avec 49,4 $cm^3$ de monohydrate d'hydrazine, 96 $cm^3$ de diéthylène glycol et 38,4 $cm^3$ de lessive de potasse à 38 %. On chauffe à 210 °C, distille l'eau et l'hydrate d'hydrazine et agite encore 1 h à 210 °C. On laisse revenir à température ambiante et verse dans 300 $cm^3$ d'une solution aqueuse saturée de chlorure de sodium, extrait à l'acétate d'éthyle et évapore à sec sous pression réduite. On chromatographie le résidu sur silice (éluant : chlorure de méthylène), on recueille 20,55 g de produit recherché, F = 139 °C, utilisé tel quel pour le stade suivant.

SPECTRE IR
NH indole 3480 $cm^{-1}$
OH + associés 3598 $cm^{-1}$
bandes aromatiques type : 1617, 1585, 1489 $cm^{-1}$
2 alkylphénol
autre aromatique : 1501 $cm^{-1}$

STADE B : 4-[[2-[(2-oxirannyl) méthoxy] phényl] méthyl] 1H-indole

On opère comme au stade A de l'exemple 2 à partir de 10,0 g du produit obtenu au stade précédent en utilisant 3 fois 17,56 $cm^3$ d'épichlorhydrine. On obtient 12,5 g du produit recherché, F = 78 °C, que l'on utilise tel quel pour le stade suivant.
SPECTRE IR
pas d'OH
=C-NH- 3482 $cm^{-1}$
aromatiques : 1611, 1600, 1587, 1492 $cm^{-1}$

STADE C : 1-[(1,1-diméthyl éthyl) amino] 3-[2-[(1H-indol-4-yl) méthyl] phénoxy] 2-propanol

On opère comme au stade B de l'exemple 2 à partir de 13,06 g du produit obtenu au stade précédent. On obtient 16,45 g du produit recherché que l'on utilise tel quel pour le stade suivant.

STADE D : Benzoate de 1-[(1,1-diméthyl éthyl) amino] 3-[2-[(1H-indol-4-yl) méthyl] phénoxy] 2-propanol

On opère comme au stade G de l'exemple 3 à partir de 16,3 g du produit obtenu au stade précédent en utilisant 5,65 g d'acide benzoïque. On obtient 8,29 g du produit recherché.
F = 195 °C (Ethanol).

| Analyse pour $C_{22}H_{28}N_2O_2$, $C_7O_2H_6$ | | | | | | |
|---|---|---|---|---|---|---|
| Calculés<br>Trouvés | C % | 73,39<br>73,0 | H % | 7,22<br>7,3 | N % | 5,9<br>5,8 |

**EXEMPLE 6 : Benzoate de N-(1,1-diméthyl éthyl) 3-[2-[(1H-indol-4-yl) méthyl] phénoxy] propanamine**

STADE A : 4-[[2-(3-chloro propoxy) phényl] méthyl] 1H-indole

On opère comme au stade E de l'exemple 1 à partir de 8,0 g du produit obtenu au stade A de l'exemple 5. On obtient après chromatographie sur silice (éluant : chlorure de méthylène-hexane (1-1)), 10,8 g du produit recherché, F = 75 °C, que l'on utilise tel quel pour le stade suivant.
SPECTRE IR
pas d'OH
=C-NH- type indole 3484 $cm^{-1}$
C=C et aromatiques : 1612, 1600, 1588, 1492 $cm^{-1}$

STADE B : N-(1,1-diméthyl éthyl) 3-[2-[(1H-indol-4-yl) méthyl] phénoxy] propanamine

On opère comme au stade F de l'exemple 1 à partir de 10,55 g du produit obtenu au stade précédent. On obtient 8,65 g du produit recherché, F = 96 °C, que l'on utilise tel quel pour le stade suivant.

STADE C : Benzoate de N-(1,1-diméthyl éthyl) 3-[2-[(1H-indol-4-yl) méthyl] phénoxy] propanamine

On opère comme au stade G de l'exemple 3 à partir de 8,12 g du produit obtenu au stade précédent en utilisant 2,95 g d'acide benzoïque. On obtient 7,42 g du produit recherché, F = 183 °C, après recristallisation dans l'éthanol.

| Analyse pour $C_{22}H_{28}N_2O$, $C_7H_6O_2$ | | | | | | |
|---|---|---|---|---|---|---|
| Calculés<br>Trouvés | C % | 75,95<br>76,1 | H % | 7,47<br>7,5 | N % | 6,11<br>5,8 |

**EXEMPLE 7 : Benzoate de alpha-[2-[3-[(1,1-diméthyl éthyl) amino] propoxy] phényl] 1H-indole-4-méthanol**

STADE A : alpha-[2-[3-[(1,1-diméthyl éthyl) amino] propoxy] phényl] 1H-indole-4-méthanol

On agite au reflux 1 heure un mélange de 5,25 g de produit obtenu au stade F de l'exemple 3 avec 70 $cm^3$ de butanol normal et 1,7 g d'hydroborure de sodium. On refroidit et ajoute 200 $cm^3$ d'une solution saturée de carbonate de sodium, extrait avec un mélange acétate d'éthyle-tétrahydrofuranne et amène à sec sous pression réduite. On chromatographie sur silice (éluant : acétate d'éthyle-triéthylamine (95-05)) on recueille 5,0 g de produit F = 205 ° C. On recristallise 4,3 g de ce produit dans 580 $cm^3$ d'acétonitrile. On obtient 3,78 g de produit recherché F = 205 ° C.
SPECTRE IR
absence de >C=O
absorptions complexes région NH/OH
C=C et aromatiques : 1602, 1590, 1496 $cm^{-1}$

| Analyse pour $C_{22}H_{28}N_2O_2$ | | | | | | |
|---|---|---|---|---|---|---|
| Calculés<br>Trouvés | C % | 74,97<br>75,0 | H % | 8,00<br>7,9 | N % | 7,95<br>8,2 |

STADE B : Benzoate de alpha-[2-[3-[(1,1-diméthyl éthyl) amino] propoxy] phényl] 1H-indole-4-méthanol

A un mélange chauffé à 70 ° C de 2,15 g de produit obtenu au stade A et 400 $cm^3$ d'acétate d'éthyle on ajoute une solution de 0,745 g d'acide benzoïque dans 11 $cm^3$ d'acétate d'éthyle, filtre à chaud la solution obtenue, refroidit et obtient 2,62 g de produit recherché. F = 185 ° C.

| Analyse pour : $C_{22}H_{28}N_2O_2$, $C_7H_6O_2$ | | | | | | |
|---|---|---|---|---|---|---|
| Calculés<br>Trouvés | C % | 73,39<br>73,1 | H % | 7,22<br>7,3 | N % | 5,90<br>5,8 |

**EXEMPLE 8 : Benzoate de alpha-[2-[3-[1,1-diméthyl éthyl) amino] 2-hydroxy propoxy] phényl] 1H-indole-4-méthanol (Isomère A)**

STADE A : alpha-[2-[3-[(1,1-diméthyl éthyl) amino] 2-hydroxy propoxy] phényl] 1H-indole-4-méthanol

On opère comme au stade A de l'exemple 7 à partir de 6,9 g du produit obtenu au stade B de l'exemple 4. Après extraction à l'acétate d'éthyle et concentration à sec, on chromatographie le résidu sur silice (éluant : acétate d'éthyle-triéthylamine (95-05)) on recueille 1,71 g d'isomère A et 4,4 g de mélange d'isomères A+B. On chromatographie le mélange sur silice (éluant : acétate d'éthyle-triéthylamine-méthanol (80-15-5)) et obtient au total, 3,29 g d'isomère A (F = 205 ° C) et 2,51 g d'isomère B que l'on chromatographie à nouveau pour obtenir 1,6 g d'isomère B utilisable pour l'exemple suivant.

STADE B : Benzoate de alpha-[2-[3-[(1,1-diméthyl éthyl) amino] 2-hydroxy propoxy] phényl] 1H-indole-4-méthanol (Isomère A)

On recristallise 3,29 g d'isomère A obtenu au stade A dans 400 $cm^3$ d'acétonitrile et recueille 2,66 g de produit. F = 205 ° C. On dissout 2,6 g d'isomère A recristallisé dans 300 $cm^3$ d'acétate d'éthyle et ajoute 0,86 g d'acide benzoïque en solution dans 30 $cm^3$ d'acétate d'éthyle, on filtre, glace pendant 12 heures, essore et obtient 3 g de produit recherché F = 191 ° C.

| Analyse pour $C_{22}H_{28}N_2O_3$, $C_7H_6O_2$ | | | | | | |
|---|---|---|---|---|---|---|
| Calculés<br>Trouvés | C % | 71,00<br>70,9 | H % | 6,98<br>7,1 | N % | 5,71<br>5,6 |

**EXEMPLE 9 : Benzoate de alpha-[2-[3-[(1,1-diméthyl éthyl) amino] 2-hydroxy propoxy] phényl] 1H-indole-4-méthanol (Isomère B)**

A une solution de 1,25 g d'isomère B (obtenu au stade A de l'exemple 8) dans 50 $cm^3$ d'acétate d'éthyle, on ajoute une solution de 0,41 g d'acide benzoïque dans 10 $cm^3$ d'acétate d'éthyle. On recueille 1,1 g de produit attendu que l'on recristallise dans 200 $cm^3$ d'acétonitrile. On obtient 0,95 g de produit recherché. F = 196°C.

| Analyse pour $C_{22}H_{28}N_2O_3$, $C_7H_6O_2$ | | | | | | |
|---|---|---|---|---|---|---|
| Calculés<br>Trouvés | C % | 71,00<br>70,9 | H % | 6,98<br>7,1 | N % | 5,71<br>5,9 |

**EXEMPLE 10 : (±) [2-[3-[(1,1-diméthyléthyl) amino] 2-hydroxy-propoxy] phényl] (1-méthyl 1H-indol-4-yl) méthanone et son benzoate.**

STADE A : 1-méthyl 1H-indol 4-carboxaldéhyde.

On mélange 4 heures à température ambiante 34 g d'indol 4-carboxaldéhyde dans 800 $cm^3$ de chlorure de méthylène avec 79,5 g d'hydrogénosulfate de tétrabutylammonium et 16,04 $cm^3$ d'iodure de méthyle dans 400 $cm^3$ de soude 5N. On ajoute 300 $cm^3$ de chlorure de méthylène, décante, extrait au chlorure de méthylène, lave la phase organique avec une solution aqueuse saturée en chlorure de sodium, sèche, concentre à sec, chromatographie le résidu sur silice (éluant : chlorure de méthylène) et récupère 36,8 g de produit attendu. F < 40°C.

STADE b : alpha-(2-méthoxyphényl) 1-méthyl 1H-indol 4-méthanol.

On opère comme au stade B de l'exemple 1 à partir de 36,8 g de produit obtenu au stade A en utilisant 104 $cm^3$ d'une solution de magnésien de 2-bromoanisole dans le tétrahydrofuranne titrant 1,94N. On extrait à l'acétate d'éthyle, lave à l'eau salée, amène à sec, chromatographie le résidu sur silice (éluant : chlorure de méthylène) et obtient 46,5 g de produit attendu. F = 95°C.

STADE C : (2-méthoxyphényl) (1-méthyl 1H-indol-4-yl) méthanone.

On opère comme au stade C de l'exemple 1 à partir de 43,7 g de l'alcool obtenu au stade B avec 92,4 g de dichromate de pyridinium. On obtient 30,1 g de produit attendu (F = 74°C) utilisé tel quel pour le stade suivant.

STADE D: (2-hydroxyphényl) (1-méthyl 1H-indol-4-yl) méthanone.

On opère comme au stade C de l'exemple 3 en utilisant au départ 29,9 g de produit obtenu au stade C, 300 g de chlorhydrate de pyridine et en utilisant le chlorure de méthylène comme éluant de la chromatographie. On recueille 17,2 g de produit attendu (F = 80°C) utilisé tel quel pour le stade suivant.

STADE E : (1-méthyl 1H-indol-4-yl) [2-(2-oxiranylméthoxy) phényl] méthanone.

On opère comme au stade A de l'exemple 2 en utilisant 3 g de produit obtenu au stade D, 6,6 g de carbonate de potassium et en ajoutant à 5 reprises 4,7 $cm^3$ d'épichlorhydrine. Après un total de 48 heures de reflux, on filtre, concentre à sec sous pression réduite et chromatographie le résidu comme indiqué à l'exemple 2. On obtient 3,7 g de produit attendu.

Spectre IR :
=O : 1650 $cm^{-1}$
aromatiques : 1599, 1581, 1567, 1508, 1481 $cm^{-1}$

STADE F : (±) [2-[3-[(1,1-diméthyléthyl) amino] 2-hydroxy-propoxy] phényl] (1-méthyl 1H-indol-4-yl) méthanone et son benzoate.

On agite 3 heures et demie au reflux une solution de 3,7 g d'époxyde obtenu au stade précédent, 80 $cm^3$ d'éthanol et 7,5 $cm^3$ de tert-butylamine. On distille à sec et chromatographie le résidu sur silice (éluant : acétate d'éthyle-triéthylamine 95-5). On obtient 3,82 g de produit recherché sous forme de base. On dissout 3,80 g du produit obtenu précédemment dans 50 $cm^3$ d'éthanol, puis ajoute une solution de 1,22 g d'acide benzoïque dans 12 $cm^3$ d'éthanol. On glace pendant 12 heures, essore, lave à l'éthanol, sèche à 80 °C sous pression réduite, recueille 4,05 g de benzoate que l'on recristallise dans l'éthanol. F = 166 °C.

| Analyse : $C_{23}H_{28}N_2O_3$, $C_7H_{16}O_2$ | | | | | | |
|---|---|---|---|---|---|---|
| Calculé<br>Trouvé | C% | 71,69<br>71,7 | H% | 6,82<br>6,8 | N% | 5,57<br>5,5 |

**EXEMPLE 11 : (±) 1-[(1,1-diméthyléthyl) amino] 3-[2-[(1-méthyl 1H-indol-4-yl) méthyl] phénoxy] 2-propanol et son benzoate.**

STADE A : 2-[(1-méthyl 1H-indol-4-yl) méthyl] phénol.

On opère comme au stade A de l'exemple 5 en utilisant 2,6 g de produit préparé au stade D de l'exemple 10, 5,33 $cm^3$ d'hydrate d'hydrazine, 10 $cm^3$ de diéthylène glycol et 4 $cm^3$ de lessive de soude à 38% et en utilisant le mélange acétate d'éthyle-hexane 5-5 comme éluant de chromatographie. On obtient 2,5 g de produit attendu utilisé tel quel pour le stade suivant.

STADE B : 1-méthyl 4-[[2-(2-oxirannylméthoxy) phényl] méthyl] 1H-indole.

On opère comme au stade A de l'exemple 2 à partir de 2,45 g du produit obtenu au stade précédent en utilisant 3 fois 4,05 $cm^3$ d'épichlorhydrine. Après chromatographie sur silice (éluant : chlorure de méthylène), on obtient 2,95 g du produit recherché (F = 72 °C) que l'on utilise tel quel pour le stade suivant.
Spectre IR :
pas d'OH
aromatiques : 1601, 1587, 1514, 1494 $cm^{-1}$

STADE C : (±) 1-[(1,1-diméthyl) amino] 3-[2-[1-méthyl 1H-indol-4-yl) méthyl] phénoxy] 2-propanol et son benzoate.

On opère comme au stade F de l'exemple 10 en utilisant 2,95 g de l'époxyde obtenu au stade B, 65 $cm^3$ d'éthanol et 6,3 $cm^3$ de tert-butylamine. On obtient 3,6 g de produit attendu sous forme de base. On dissout 3,80 g de base préparée comme ci-dessus dans 70 $cm^3$ d'isopropanol et ajoute 1,33 g d'acide benzoïque en solution dans 15 $cm^3$ d'isopropanol. On glace pendant 12 heures, essore, lave à l'isopropanol puis à l'éther, sèche à 80 °C sous pression réduite et récupère 2,61 g de produit attendu. F = 136 °C.

| Analyse : $C_{23}H_{30}N_2O_2$, $C_7H_6O_2$ | | | | | | |
|---|---|---|---|---|---|---|
| Calculé<br>Trouvé | C% | 73,74<br>73,6 | H% | 7,43<br>7,4 | N% | 5,73<br>5,7 |

**EXEMPLE 12 : [2-[3-[(1,1-diméthyléthyl) amino] propoxy] phényl] (1-méthyl 1H-indol-4-yl) méthanone.**

STADE A : [2-(3-chloropropoxy) phényl] (1-méthyl 1H-indol-4-yl) méthanone.

On agite au reflux pendant 24 heures un mélange de 8 g du produit obtenu au stade D de l'ex. 10, 8,80 g de carbonate de potassium, 215 $cm^3$ d'acétone et 12,53 $cm^3$ de 1-bromo 3-chlorochloropropane. On laisse revenir à température ambiante, filtre, lave à l'acétone et concentre à sec sous pression réduite. On chromatographie le résidu sur silice (éluant : chlorure de méthylène-hexane 7-3) et obtient 10,4 g de produit recherché, F = 60 ° C, que l'on utilise tel quel pour le stade suivant.
Spectre IR :
pas d'OH
C = O : 1649 $cm^{-1}$

système conjugué
+ : 1599, 1582, 1568, 1508 $cm^{-1}$
aromatique

STADE B : [2-[3-[(1,1-diméthyléthyl) amino] propoxy] phényl] (1-méthyl 1H-indol-4-yl) méthanone.

On chauffe à 150 ° C pendant 24 heures en autoclave un mélange de 10,30 g du produit obtenu au stade précédent, 8,68 g de carbonate de potassium, 13,13 $cm^3$ de tert-butylamine et 180 $cm^3$ d'éthanol. On filtre, amène à sec sous pression réduite et chromatographie le résidu sur silice (éluant : acétate d'éthyle-triéthylamine 95-05). On obtient 11,06 g de produit recherché sous forme de base. On opère ensuite comme au stade C de l'exemple 11 en utilisant 3 g de base ci-dessus et 1 g d'acide benzoïque. On obtient 3,15 g de benzoate. F = 172 ° C.

| Analyse : $C_{23}H_{28}N_2O_2$, $C_7H_6O_2$ | | | | | | |
|---|---|---|---|---|---|---|
| Calculé<br>Trouvé | C% | 74,05<br>74,0 | H% | 7,04<br>7,0 | N% | 5,76<br>5,8 |

**EXEMPLE 13 : (±) alpha-[2-[3-[(1,1-diméthyléthyl) amino] propoxy] phényl] 1-méthyl 1H-indol-4-méthanol et son fumarate.**

STADE A : (±) alpha-[2-[3-[(1,1-diméthyléthyl) amino] propoxy] phényl] 1-méthyl 1H-indol-4-yl) méthanone.

On agite 5 heures à température ambiante 0,4 g de [2-[3-[(1,1-diméthyléthyl) amino] propoxy] phényl] (1H-indol-4-yl) méthanone préparée au stade F de l'exemple 3, 8 $cm^3$ de chlorure de méthylène, 0,07 $cm^3$ d'iodure de méthyle, 4 $cm^3$ de soude 5N et 0,39 g d'hydrogénosulfate de tétrabutylammonium. On dilue avec de l'eau, extrait au chlorure de méthylène, lave la phase organique à l'eau salée, sèche et amène à sec sous pression réduite. On chromatographie le résidu sur silice (eluant : acétate d'éthyle-triéthylamine 95-5) et obtient 0,4 g de produit attendu. F = 60 ° C.

STADE B : (±) alpha-[2-[3-[(1,1-diméthyléthyl) amino] propoxy] phényl] 1-méthyl 1H-indol-4-méthanol et son fumarate.

On chauffe 1 heure au reflux 3,30 g de produit préparé comme au stade A dans 33 $cm^3$ de butanol, en présence de 1,027 g de borohydrure de sodium. On refroidit à température ambiante, verse dans 100 $cm^3$ d'une solution aqueuse saturée en carbonate de sodium et extrait à l'acétate d'éthyle. On concentre à sec sous pression réduite, chromatographie le résidu sur silice (éluant : acétate d'éthyle-triéthylamine 95-5) et recueille 3,04 g de produit attendu sous forme de base. F = 124 ° C. On prépare ensuite le fumarate comme indiqué au stade G de l'exemple 1 en utilisant 3,4 g de base obtenue comme ci-dessus et 1,07 g d'acide fumarique. On recueille 3,48 g de fumarate attendu. F = 208 ° C.

| Analyse : $C_{23}H_{30}N_2O_2$, $C_4H_4O_4$ | | | | | | |
|---|---|---|---|---|---|---|
| Calculé<br>Trouvé | C% | 67,2<br>67,5 | H% | 7,10<br>7,2 | N% | 5,80<br>5,8 |

**EXEMPLE 14 : 1,3-dihydro 4-[2-[3-(1,1-diméthyléthyl) amino] propoxy] benzoyl] 2H-indol-2-one et son fumarate.**

STADE A : [2-[3-[(1,1-diméthyléthyl) amino] propoxy] phényl] (3-chloro 1H-indol-4-yl) méthanone.

On agite pendant 2 heures à température ambiante 6,3 g de [2-[3-[1,1-diméthyléthyl) amino] propoxy] phényl] (1H-indol-4-yl) méthanone préparée au stade F de l'exemple 3 dans 50 cm$^3$ d'acide acétique en présence de 2,64 g de N-chloro succinimide. On verse le mélange dans 300 cm$^3$ d'une solution aqueuse saturée de carbonate de sodium, extrait à l'acétate d'éthyle, lave la phase organique à l'eau salée, sèche et concentre à sec. On obtient, après chromatographie du résidu sur silice (éluant : acétate d'éthyle-triéthylamine 95-5), 5,52 g de produit attendu utilisé tel quel pour le stade suivant.

STADE B : 1,3-dihydro 4-[2-[3-(1,1-diméthyléthyl) amino] propoxy] benzoyl] 2H-indol-2-one et son fumarate.

On chauffe au reflux pendant 1 heure et demie 5,50 g de produit préparé au stade A dans 77 cm$^3$ d'éthanol en présence de 154 cm$^3$ d'acide chlorhydrique N. On laisse revenir à température ambiante, verse sur 170 cm$^3$ de soude N et extrait à l'acétate d'éthyle. On lave la phase organique, sèche et concentre à sec. On obtient 5,7 g de produit brut que l'on chromatographie sur silice (éluant : acétate d'éthyle-méthanol-triéthylamine8-1-1). Après empâtage à l'éther isopropylique et séchage sous pression réduite, on recueille 3,80 g de produit attendu. F = 132 °C. On prépare le fumarate comme indiqué au stade G de l'exemple 1 en utilisant 2,5 g de base préparée ci-dessus et 0,79 g d'acide fumarique, en opérant dans l'éthanol. On obtient après recristallisation 1,51 g de fumarate attendu. F = 258 °C.

| Analyse : $C_{22}H_{26}N_2O_3$, $C_4H_4O_4$ | | | | | | |
|---|---|---|---|---|---|---|
| Calculé<br>Trouvé | C% | 67,91<br>67,9 | H% | 6,65<br>6,6 | N% | 6,60<br>6,3 |

**EXEMPLE 15 : 1,3-dihydro 4-[2-[3-[(1,1-diméthyléthyl) amino] 2-hydroxypropoxy] benzoyl] 2H-indol-2-one et son fumarate neutre.**

On chauffe 1 heure au reflux une solution comprenant 4 g de fumarate neutre préparé comme indiqué au stade C de l'exemple 4, 160 cm$^3$ d'éthanol et 1,57 g de N-chlorosuccinimide. On refroidit à température ambiante et ajoute 50 cm$^3$ de soude N et 100 cm$^3$ d'eau. On extrait à l'acétate d'éthyle, lave à l'eau salée la phase organique, sèche et amène à sec. On obtient 3,6 g d'intermédiaire chloré auquel on ajoute 57,6 cm$^3$ d'éthanol et 113 cm$^3$ d'acide chlorhydrique. On agite 24 heures à température ambiante puis 45 minutes au reflux. On laisse revenir à température ambiante, ajoute 150 cm$^3$ de soude N, extrait à l'acétate d'éthyle, sèche, concentre à sec, chromatographie le résidu sur silice (éluant : acétate d'éthyle-méthanol-triéthylamine 80-10-10). On obtient 2,37 g de produit attendu sous forme de base. F = 158 °C. On opère ensuite comme au stade G de l'exemple 1 à partir de 2,3 g du produit obtenu précédemment et 1,46 g d'acide fumarique. On obtient 1,78 g de produit attendu. F = 236 °C.

**EXEMPLE 16 : [2-[2-[(1,1-diméthyléthyl) amino] éthoxy] phényl] (1H-indol-4-yl) méthanone et son fumarate.**

STADE A : [2-(2-chloroéthoxy] phényl] (1H-indol-4-yl) méthanone.

On agite pendant 72 heures à 50 °C 6 g de produit obtenu comme au stade D de l'exemple 3, 192 cm$^3$ de toluène, 90 cm$^3$ d'acétonitrile, 2,16 g d'hydrogénosulfate de tétrabutylammonium, 9,18 cm$^3$ de tosylate de 2-chloroéthanol et 90 cm$^3$ de soude 5N. On dilue avec de l'eau, extrait à l'acétate d'éthyle, lave la phase

organique, sèche, concentre à sec sous pression réduite et chromatographie le résidu sur silice (éluant : chlorure de méthylène). On obtient 5,39 g de produit attendu utilisé tel quel pour le stade suivant.

STADE B : [2-[2-[(1,1-diméthyléthyl) amino] éthoxy] phényl] (1H-indol-4-yl) méthanone et son fumarate.

On opère comme au stade F de l'exemple 1 à partir de 5,46 g du dérivé chloré préparé comme ci-dessus, 5,03 g de carbonate de potassium, 7,61 $cm^3$ de terbutylamine dans 70 $cm^3$ d'éthanol. On obtient 4,95 g de produit attendu sous forme de base. F = 140 °C. On prépare le fumarate à partir de 4,73 g de base et 1,63 g d'acide fumarique en opérant comme au stade G de l'exemple 1. On obtient 3,34 g de fumarate attendu après recristallisation dans l'éthanol. F = 180 °C.

| Analyse : $C_{21}H_{24}N_2O_2$, 1/2 $C_4H_4O_4$ | | | | | | |
|---|---|---|---|---|---|---|
| Calculé<br>Trouvé | C% | 70,03<br>70,3 | H% | 6,64<br>6,7 | N% | 7,10<br>7,1 |

**EXEMPLE 17 : (±) [2-[2-hydroxy 3-[(1-méthyléthyl) amino] propoxy] phényl] (1H-indol-4-yl) méthanone et son fumarate.**

On opère comme au stade B de l'exemple 2 en utilisant 3,7 g de l'époxyde préparé au stade A de l'exemple 4 et 6,45 $cm^3$ d'isopropylamine dans 45 $cm^3$ d'éthanol. On obtient 3,26 g de produit attendu sous forme de base. En opérant comme au stade G de l'exemple 1 à partir de 3,19 g de la base ci-dessus et 1,05 g d'acide fumarique, on obtient 1,62 g de fumarate attendu. F = 193 °C.

| Analyse : $C_{21}H_{24}N_2O_3$, 1/2 $C_4H_4O_4$ | | | | | | |
|---|---|---|---|---|---|---|
| Calculé<br>Trouvé | C% | 67,30<br>67,4 | H% | 6,38<br>6,6 | N% | 6,82<br>6,7 |

**EXEMPLE 18 : (±) [2-[3-[[bis (1-méthyléthyl) amino] 2-hydroxy-propoxy] phényl] (1H-indol-4-yl) méthanone et son chlorhydrate.**

On chauffe au reflux pendant 4 heures 1 g de l'époxyde préparé au stade A de l'exemple 4 dans 40 $cm^3$ d'éthanol et ajoute 2,4 $cm^3$ de diisopropylamine. On élimine le solvant sous pression réduite, refroidit à 4 °C, empâte à l'éther et recueille 880 mg de produit attendu sous forme de base. F = 100 °C. On dissout 2,1 g de base dans 100 $cm^3$ d'acétate d'éthyle à température ambiante, ajoute 3 $cm^3$ d'une solution d'acétate d'éthyle saturée en acide chlorhydrique, glace 1 heure, essore et sèche sous pression réduite 2,25 g de chlorhydrate attendu. F = 210-212 °C.

| Analyse : $C_{24}H_{30}N_2O_3$, HCl | | | | | | | | |
|---|---|---|---|---|---|---|---|---|
| Calculé<br>Trouvé | C% | 66,89<br>66,6 | H% | 7,25<br>7,4 | Cl% | 8,23<br>8 | N% | 6,5<br>6,2 |

**EXEMPLE 19 : (±) [2-[2-hydroxy 3-(4-méthyl 1-pipérazinyl) propoxy] phényl] (1H-indol-4-yl) méthanone et son fumarate.**

On opère comme au stade B de l'exemple 2 en utilisant 2,5 g de l'époxyde préparé au stade A de l'exemple 4 et 4,73 $cm^3$ de N-méthylpipérazine. Après chromatographie sur silice (éluant : chloroforme-méthanol-triéthylamine 8-1-1), on obtient 3 g de produit attendu sous forme de base. En opérant comme au stade G de l'exemple 1 à partir de 3,3 g de base ci-dessus et 0,97 g d'acide fumarique, on obtient, après recristallisation dans l'éthanol, 3,25 g de fumarate attendu. F = 186 °C.

| Analyse : $C_{23}H_{27}N_3O_3$, 1/2 $C_4H_4O_4$ | | | | | | |
|---|---|---|---|---|---|---|
| Calculé<br>Trouvé | C% | 66,5<br>66,4 | H% | 6,47<br>6,5 | N% | 9,31<br>9,3 |

**EXEMPLE 20 : (±) [2-[2-hydroxy 3-(propylamino) propoxy] phényl] (1H-indol-4-yl) méthanone et son fumarate.**

On opère comme à l'exemple 19 en utilisant 3 g de l'époxyde préparé au stade A de l'exemple 4 et 5,22 $cm^3$ de N-propylamine. On obtient 2,4 g de produit attendu sous forme de base que l'on fait réagir avec 0,79 g d'acide fumarique pour obtenir 1,67 g de fumarate attendu. F = 175 °C.

| Analyse : $C_{21}H_{24}N_2O_3$, 1/2 $C_4H_4O_4$ | | | | | | |
|---|---|---|---|---|---|---|
| Calculé<br>Trouvé | C% | 67,3<br>67,2 | H% | 6,38<br>6,3 | N% | 6,82<br>6,8 |

**EXEMPLE 21 : (±) [2-[(3-cyclohexylamino) 2-hydroxypropoxy] phényl] (1H-indol-4-yl) méthanone.**

On opère comme à l'exemple 19 en utilisant 3 g de l'époxyde préparé au stade A de l'exemple 4 et 5,8 $cm^3$ de cyclohexylamine. On obtient 2 g de produit attendu sous forme de base que l'on recristallise dans l'acétonitrile. On obtient 1,6 g de produit pur. F≃ 136-138 °C.

| Analyse : $C_{24}H_{28}N_2O_3$ | | | | | | |
|---|---|---|---|---|---|---|
| Calculé<br>Trouvé | C% | 73,44<br>73,5 | H% | 7,19<br>7,5 | N% | 7,14<br>7,2 |

**EXEMPLE 22 : (±) 3-[(1,1-diméthyléthyl) amino] 1-[2-[1-(1H-indol-4-yl) éthényl] phénoxy] 2-propanol et son chlorhydrate.**

STADE A : 2-[1-(1H-indol-4-yl) éthényl] phénol.

**- Préparation de l'iodure de méthyl magnésium.**

A 20 g de magnésium en tournures dans 70 $cm^3$ d'éther, on ajoute lentement 52 $cm^3$ d'iodure de méthyle dans 400 $cm^3$ d'éther. On chauffe 1 heure au reflux et obtient le magnésien attendu titrant 1,48 M/l.

**- Condensation :**

Dans 236 $cm^3$ de solution de magnésien obtenue ci-dessus, on ajoute à température ambiante, 20 g de (2-hydroxyphényl) (1H-indol-4-yl) méthanone préparée comme au stade D de l'exemple 3 en solution dans 400 $cm^3$ de tétrahydrofuranne. On élimine l'éther, le remplace par du tétrahydrofuranne, chauffe 1 heure au reflux, refroidit, détruit l'excès de magnésien par addition d'une solution aqueuse saturée en chlorure d'ammonium, dilue à l'eau et extrait à l'acétate d'éthyle. On sèche la phase organique, élimine les solvants sous pression réduite à 50 °C et obtient 21,9 g de produit brut que l'on chromatographie sur silice (éluant : chlorure de méthylène-acétate d'éthyle 98-2) et obtient 17,8 g de produit attendu que l'on cristallise dans l'éther isopropylique. On recueille en 2 jets 17,4 g de produit pur. F = 117-118 °C.

STADE B : 4-[1-[2-[(2-oxyrannyl) méthoxy] phényl] éthényl] 1H-indole.

On opère comme au stade A de l'exemple 2 en utilisant 7 g de produit obtenu au stade A, 4,2 g de carbonate de potassium et en ajoutant à 2 reprises 24 $cm^3$ d'épichlorhydrine. Après total de 44 heures de reflux, on filtre, concentre à sec sous pression réduite à 50 °C et chromatographie le résidu sur silice

(éluant : chlorure de méthylène-acétate d'éthyle 98-2), récupère les fractions dont le rf = 0,35 et obtient après cristallisation dans l'éther isopropylique 7,35 g de produit attendu. F = 87-88 ° C.

STADE C : (±) 3-[(1,1-dimethyléthyl) amino] 1-[2-[1-(1H-indol-4-yl) éthényl] phénoxy] 2-propanol et son chlorhydrate.

On opère comme au stade B de l'exemple 2 à partir de 7 g de produit obtenu au stade B, 70 $cm^3$ d'éthanol et 35 $cm^3$ de tert-butylamine. On obtient 7,7 g de produit attendu sous forme de base. F = 127-128 ° C.

Spectre IR : ($CHCl_3$)
OH : 3535 $cm^{-1}$
=C-NH : 3490 $cm^{-1}$
C=C + aromatique : 1620, 1600, 1580, 1490 $cm^{-1}$

On dissout à température ambiante 3,7 g de la base ci-dessus dans 200 $cm^3$ d'acétate d'éthyle et forme le chlorhydrate par addition d'une solution d'acide chlorhydrique dans l'acétate d'éthyle. On obtient après recristallisation dans l'éthanol, 2,7 g de chlorhydrate attendu. F = 204-205 ° C.

| Analyse : $C_{23}H_{28}N_2O_2$, HCl | | | | | | | | |
|---|---|---|---|---|---|---|---|---|
| Calculé<br>Trouvé | C% | 68,90<br>69,1 | H% | 7,29<br>7,5 | Cl% | 8,84<br>8,8 | N% | 6,99<br>6,8 |

**EXEMPLE 23 : (±) 3-[1,1-diméthyléthyl) amino] 1-[2-[1-(1H-indol-4-yl) éthyl] phénoxy] 2-propanol et son chlorhydrate.**

On hydrogène pendant 30 minutes à 50 ° C 4,1 g de la base obtenue comme indiqué au stade C de l'exemple 22 dans 400 $cm^3$ d'éthanol, en présence de 1,3 g de charbon actif à 10% de palladium. On filtre le catalyseur, élimine le solvant à 50 ° C sous pression réduite et obtient 4,6 g de produit brut que l'on recristallise dans l'éther isopropylique. On obtient 3,3 g de produit attendu sous forme de base. F = 135-137 ° C.

Spectre IR : ($CHCl_3$)
OH 3630 $cm^{-1}$
=C-NH 3486 $cm^{-1}$
C=C + aromatique 1610, 1599, 1586, 1492 $cm^{-1}$

On dissout 3,3 g de la base obtenue ci-dessus dans 400 $cm^3$ d'éther isopropylique et forme le chlorhydrate par addition d'une solution saturée d'acide chlorhydrique dans l'acétate d'éthyle. Après recristallisation dans l'acétonitrile, on obtient 2,6 g de chlorhydrate attendu. F = 208-209 ° C.

| Analyse : $C_{23}H_{30}N_2O_2$, HCl | | | | | | | | |
|---|---|---|---|---|---|---|---|---|
| Calculé<br>Trouvé | C% | 68,56<br>68,3 | H% | 7,75<br>7,9 | Cl% | 8,80<br>8,8 | N% | 6,95<br>7,0 |

**EXEMPLE 24 : N-(1,1-diméthyléthyl) 3-[2-[1-(1H-indol-4-yl) éthényl] phénoxy] propanamide et son chlorhydrate.**

STADE A : 4-[-1-[2-(3-chloropropoxy) phényl] éthényl] 1H-indole.

On opère comme indiqué au stade E de l'exemple 1 en utilisant 6,7 g de 2-[1-(1H-indol-4-yl) éthényl] phénol préparé comme au stade A de l'exemple 22, 70 $cm^3$ d'acétone, 4 g de carbonate de potassium et 11,5 $cm^3$ de 1-chloro 3-bromopropane et en chauffant 20 heures au reflux. On obtient après chromatographie et cristallisation dans l'éther de pétrole (Eb = 40-70 ° C) 7,05 g de produit attendu. F = 70-72 ° C.

STADE B : N-(1,1-diméthyléthyl) 3-[2-[1-(1H-indol-4-yl) éthényl] phénoxy] propanamide et son chlorhydrate.

On opère comme au stade F de l'exemple 1 en chauffant 6 heures à 120 °C, 7 g de produit obtenu au stade A, 50 $cm^3$ de tert-butylamine, 2,9 g de carbonate de potassium dans 50 $cm^3$ d'éthanol. On obtient après chromatographie et cristallisation dans l'éther isopropylique, 7,4 g de produit attendu sous forme de base. F = 112 °C.
Spectre IR : (Nujol)
NH indole : 3630 $cm^{-1}$
C=C, C=N et aromatique 1615, 1597, 1578 $cm^{-1}$

On prépare le chlorhydrate comme indiqué au stade C de l'exemple 22 à partir de 4,6 g de la base obtenue ci-dessus. On obtient 3,35 g de chlorhydrate attendu. F = 238-240 °C.

| Analyse : $C_{23}H_{28}N_2O$, HCl | | | | | | | | |
|---|---|---|---|---|---|---|---|---|
| Calculé<br>Trouvé | C% | 71,76<br>71,8 | H% | 7,59<br>7,6 | Cl% | 9,21<br>9,4 | N% | 7,28<br>7,0 |

**EXEMPLE 25 : (±) O-méthyloxime de [2-[3-[(2,2-diméthyléthyl) amino] 2-hydroxypropoxy] phényl] (1H-indol-4-yl) méthanone et son chlorhydrate.**

STADE A : (±) O-méthyloxime de (2-hydroxyphényl) (1H-indol-4-yl) méthanone.

On chauffe 7 heures au reflux 4,8 g de (2-hydroxyphényl) (1H-indol-4-yl) méthanone préparée comme au stade D de l'exemple 3 dans 100 $cm^3$ de méthanol avec 8,5 g de chlorhydrate de méthylhydroxylamine, 4,8 g d'acétate de sodium dans 40 $cm^3$ d'eau distillée. On élimine le méthanol sous pression réduite, dilue à l'eau et extrait à l'acétate d'éthyle. On concentre à sec, chromatographie le résidu sur silice (éluant : chlorure de méthylène-acétate d'éthyle 98-2) et obtient d'une part 4,08 g d'isomère A (F = 228 °C) (correspondant au produit cristallisé dans le mélange de chromatographie et à la fraction de rf = 0,50) et d'autre part 900 mg d'isomère B (correspondant à la fraction de rf = 0,25).

| Analyse : $C_{16}H_{14}N_2O_2$ | | | | | | |
|---|---|---|---|---|---|---|
| Calculé<br>Trouvé<br>Isomère A<br>Isomère B | C% | 72,17<br><br>72,2<br>72,4 | H% | 5,30<br><br>5,1<br>5,3 | N% | 10,52<br><br>10,4<br>10,3 |

STADE B : O-méthyloxime de [2-(2-oxirannylméthoxy) phényl] (1H-indol-4-yl) méthanone.

On opère comme au stade A de l'exemple 2 en utilisant 3,57 g de produit obtenu au stade A, 7,5 g de carbonate de potassium et en ajoutant à 4 reprises 5,4 $cm^3$ d'épichlorhydrine. Après un total de 96 heures de reflux, on filtre, concentre à sec sous pression réduite et chromatographie le résidu sur silice (éluant : hexane-acétate d'éthyle 7-3). On obtient 2,7 g de produit attendu. F = 140 °C.

STADE C : (±) O-méthyloxime de [2-[3-[(2,2-diméthyléthyl) amino] 2-hydroxypropoxy] phényl] (1H-indol-4-yl) méthanone et son chlorhydrate.

On chauffe 5 heures au reflux 2,7 g de produit obtenu au stade A dans 270 $cm^3$ d'éthanol en présence de 4,42 $cm^3$ de tert-butylamine. On refroidit à température ambiante, concentre à sec, chromatographie le résidu sur silice (éluant : chloroforme-méthanol-triéthylamine 90-5-5). On obtient 2 g de produit brut que l'on empâte à l'éther et recueille 1,67 g de produit attendu sous forme de base. F = 153-155 °C.
Spectre IR : ($CHCl_3$)
-OH 3542 $cm^{-1}$
=C-NH 3480 $cm^{-1}$
C=N + aromatique 1610, 1600, 1589 $cm^{-1}$

On prépare le chlorhydrate comme indiqué au stade C de l'exemple 22 en utilisant 1,7 g de la base préparée ci-dessus. On obtient après cristallisation dans l'isopropanol 1,4 g de chlorhydrate attendu. F = 246-248 ° C.

| Analyse : $C_{23}H_{29}N_3O_3$, HCl | | | | | | | | |
|---|---|---|---|---|---|---|---|---|
| Calculé<br>Trouvé | C% | 63,95<br>63,7 | H% | 7,00<br>7,1 | Cl% | 8,21<br>8,1 | N% | 9,73<br>9,6 |

**EXEMPLE 26 :**

On a préparé des comprimés répondant à la formule :

| | |
|---|---|
| - produit de l'exemple 4<br>- excipient q.s. pour un comprimé terminé à | 100 mg<br>150 mg |

(détail de l'excipient : lactose, amidon, talc, stéarate de magnésium).

**ETUDE PHARMACOLOGIOUE**

1) Action antiarythmique chez le rat.

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse ou par voie orale.

Au bout de cinq minutes dans le cas d'administration par voie intraveineuse et d'une heure dans le cas d'administration par voie orale, on perfuse la veine jugulaire des rats avec 10 microgrammes/mn sous 0,2 ml d'une solution d'aconitine et on note le temps d'apparition des premières extrasystoles ventriculaires. La quantité d'aconitine perfusée est calculée puis exprimée en fonction du poids corporel de l'animal.

Le pourcentage d'augmentation de la dose d'aconitine nécessaire pour déclencher des extrasystoles ventriculaires après traitement est calculé par rapport aux animaux témoins.

Les résultats figurant sur le tableau ci-après montrent que les produits de la présente demande sont doués de remarquables propriétés anti-arythmiques.

| Produit de l'exemple | Voie | Dose mg/kg | Pourcentage de protection |
|---|---|---|---|
| 4 | IV | 5 | > 217 |
| | PO | 25 | 73 |
| 9 | IV | 5 | > 165 |
| | PO | 25 | 91 |
| 7 | IV | 5 | > 201 |
| 3 | IV | 5 | 202 |
| | PO | 25 | 52 |
| 2 | IV | 5 | 93 |
| | PO | 25 | 82 |
| 10 | IV | 1 | 133 |
| | PO | 25 | 124 |
| 11 | IV | 5 | 257 |
| | PO | 25 | 81 |
| 12 | IV | 1 | 99 |
| 13 | IV | 5 | 140 |
| 22 | IV | 5 | 84 |
| | PO | 25 | 33 |
| 23 | IV | 5 | 88 |
| 18 | IV | 5 | 135 |
| 19 | IV | 5 | 142 |
| 21 | IV | 5 | 124 |
| 25 | IV | 5 | 186 |

2) Test d'activité anticalcique in vitro

Artère caudale isolée du rat.

Des anneaux d'artère caudale de rats Wistar ont été suspendus dans une solution de Krebs-bicarbonate (NaCl : 120,8 mM, Kcl : 5,9 mM, Mg $Cl_2$ : 1,2 mM, $NaH_2PO_4$ : 1,2 mM, $NaHCO_3$ : 15,5 mM, glucose : 12,6 mM) sans calcium en présence de phentolamine $10^{-5}$M, oxygéné par un mélange gazeux contenant 5 % de $CO_2$ dans l'oxygène et maintenu à 37 °C.

Après 30 minutes d'équilibre, les préparations ont été contractées toutes les 15 minutes à l'aide d'une solution dépolarisante (NaCl : 26,7 mM, Kcl : 100 mM, $MgCl_2$ : 1,2 mM, $NaH_2PO_4$ : 1,2 mM, $NaHCO_3$ : 15,5 mM, glucose : 12,6 mM) contenant 2,5 mM de $CaCl_2$.

Quand les réponses contractiles sont devenues reproductibles, le produit à tester est ajouté à des concentrations croissantes 15 mn avant chaque contraction.

La concentration qui inhibe de 50 % la contraction induite par le chlorure de calcium est calculée ($Cl_{50}$).

On constate d'après les résultats figurant sur le tableau ci-après que les produits de la présente demande possèdent une forte activité anticalcique.

| Produit de l'exemple | $CI_{50}$ en micromoles |
|---|---|
| 6 | 0,4 |
| 1 | 0,46 |
| 2 | 1,9 |
| 5 | 2 |
| 4 | 6,2 |
| 3 | 1,9 |
| 11 | 3 |
| 12 | 4,8 |
| 16 | 1,1 |
| 22 | 3,8 |
| 23 | 4,7 |
| 24 | 3,1 |
| 18 | 7,2 |

3) Test de l'activité anti-agrégante plaquettaire. Agrégation plaquettaire, in vitro, sur plasma riche en plaquettes (PRP)

La mesure de l'agrégation plaquettaire est faite selon la méthode turbidimétrique de Born G.V. et Croos M.J. 1963, J. Physiol 168 178. Le sang de lapin est prélevé sur citrate de NA à 3,2 % par ponction cardiaque. Le plasma riche en plaquettes est obtenu par centrifugation et ajustement du nombre des plaquettes à 300 000 ml.

L'agrégation est induite par le collagène (40 microgrammes/ml de PRP) ou le PAF acéther (0,05 micromoles/l de PRP).

Les molécules à tester sont mises à incuber à différentes concentrations dans le PRP 2 minutes avant l'agent agrégant.

Les résultats sont exprimés en $CI_{50}$ (concentration inhibant l'agrégation de 50 % par rapport aux courbes témoins).

| Produit de l'exemple | $CI_{50}$ en $10^{-5}$ M | |
|---|---|---|
| | Induction par le COLLAGENE | Induction par le PAF ACETHER |
| 3 | 3,1 | 4,7 |
| 4 | 0,95 | 5,5 |
| 5 | 1,8 | 4,2 |
| 6 | 1,4 | 3,7 |
| 10 | 2,1 | 7 |
| 11 | 1 | 2,4 |
| 12 | 4 | 4 |
| 13 | 6 | 7 |
| 16 | 5,7 | 10 |
| 22 | 0,9 | 1,4 |
| 23 | 0,8 | 2,5 |
| 24 | 0,8 | 1,4 |

4) Etude de la toxicité aigüe

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants :

| Produit de l'exemple | $DL_0$ en mg/kg |
|---|---|
| 3 | 100 |
| 5 | > 200 |
| 7 | 100 |
| 8 | > 200 |
| 9 | > 200 |
| 15 | > 200 |
| 22 | 200 |

**Revendications**

**Revendications pour les Etats contractants suivants : AT, BE, CH, DE, DK, FR, GB, IT, LI, LU, NL, SE**

**1.** Les produits de formule générale (I) :

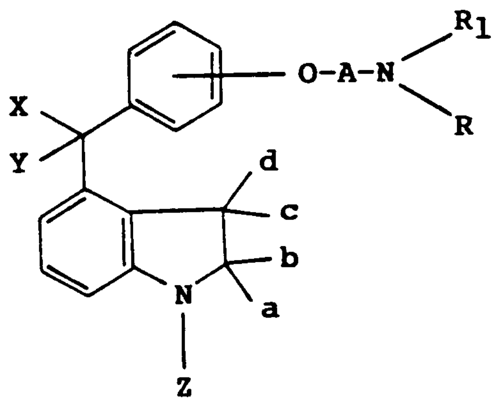

(I)

dans laquelle R et $R_1$ identiques ou différents, représentent : - soit un atome d'hydrogène,

- soit un radical alkyle, alkényle ou alkynyle linéaire ou ramifié ayant au plus 8 atomes de carbone, éventuellement substitué par un radical hydroxy,
- soit un radical cycloalkyle renfermant de 3 à 7 atomes de carbone, un radical cycloalkylalkyle renfermant de 4 à 7 atomes de carbone ou un radical arylalkyle renfermant de 7 à 14 atomes de carbone, ce dernier radical étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 5 atomes de carbone et les radicaux trifluorométhyle, méthylthio, nitro, amino, monoalkylamino ou dialkylamino,

  ou R et $R_1$ forment ensemble, avec l'atome d'azote auquel ils sont liés, un hétérocycle saturé ou insaturé pouvant renfermer un second hétéroatome choisi parmi les atomes d'oxygène, de soufre ou d'azote, ce second atome d'azote non lié à A étant éventuellement substitué par :
- soit un radical alkyle renfermant de 1 à 5 atomes de carbone,
- soit un radical phényle lui-même éventuellement substitué par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,
- soit un radical naphtyle,
- soit un radical arylalkyle ou diarylalkyle renfermant de 7 à 14 atomes de carbone, chacun de ces radicaux étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,

  A représente :
- soit une chaîne $(CH_2)_n$ dans laquelle n peut prendre les valeurs 2, 3, 4 ou 5

- soit une chaîne

$$-CH_2-\underset{\displaystyle OH}{\underset{|}{CH}}-CH_2-$$

X et Y sont tels que :
- soit chacun représente un atome d'hydrogène,
- soit l'un représente un atome d'hydrogène et l'autre représente un radical hydroxy, un radical alkoxy renfermant de 1 à 4 atomes de carbone ou un radical alkyle renfermant de 1 à 4 atomes de carbone,
- soit X et Y forment ensemble un radical oxo, un radical alkylidène ayant de 1 à 4 atomes de carbone ou un radical $=N\text{-}OR_5$ dans lequel $R_5$ représente un atome d'hydrogène ou un radical alkyle renfermant de 1 à 4 atomes de carbone,

les substituants a, b, c, d sont tels que :
- soit ils représentent chacun un atome d'hydrogène
- soit a et b forment ensemble une fonction oxo et c et d représentent chacun un atome d'hydrogène
- soit l'un de a ou b forme avec l'un de c ou d une double liaison et les autres substituants représentent chacun un atome d'hydrogène,

Z représente un atome d'hydrogène, un radical alkyle, alkényle ou alkynyle, chacun de ces radicaux linéaire ou ramifié ayant au plus 5 atomes de carbone, un radical cycloalkylalkyle renfermant de 4 à 7 atomes de carbone, un radical arylalkyle renfermant de 7 à 14 atomes de carbone, chacun de ces radicaux étant éventuellement substitué par 1, 2 ou 3 radicaux choisis parmi le groupe constitué par le radical hydroxy, les halogènes, les radicaux alkyle ou alkyloxy linéaires ou ramifiés ayant au plus 5 atomes de carbone,

ou Z représente un groupe aminoalkyle de formule $-R_2-N-(R_3)(R_4)$ dans laquelle $R_3$ et $R_4$, identiques ou différents, représentent un atome d'hydrogène, un radical alkyle linéaire ou ramifié renfermant au plus 5 atomes de carbone,

et $R_2$ est un radical alkylène renfermant de 2 à 5 atomes de carbone,

lesdits composés de formule (I) pouvant être sous toutes les formes énantiomères, racémiques ou diastéréoisomères possibles et sous forme de sels d'addition avec les acides minéraux ou organiques.

**2.** Les produits de formule (I) telle que définie à la revendication 1, dans laquelle Z est un atome d'hydrogène ou un radical alkyle renfermant de 1 à 4 atomes de carbone, lesdits composés de formule (I) pouvant être sous forme de sels d'addition avec les acides minéraux ou organiques.

**3.** Les produits de formule (I) telle que définie à l'une quelconque des revendications 1 et 2, dans laquelle X et Y représentent un atome d'hydrogène ou forment ensemble un radical oxo ou un radical alkylidène,
lesdits composés de formule (I) pouvant être sous forme de sels d'addition avec les acides minéraux ou organiques.

**4.** Les produits de formule (I) telle que définie à l'une quelconque des revendications 1 à 3, dans laquelle R est un atome d'hydrogène, $R_1$ représente un radical alkyle renfermant de 1 à 4 atomes de carbone ou un radical cycloalkyle renfermant de 3 à 7 atomes de carbone ou R et $R_1$ forment ensemble avec l'atome d'azote auquel ils sont liés un hétérocycle pouvant renfermer un second atome d'azote éventuellement substitué par un radical alkyle renfermant de 1 à 5 atomes de carbone et A est tel que l'entier n a la valeur 2 ou 3 ou A représente le groupe

$$-(CH_2)-\underset{\displaystyle OH}{\underset{|}{CH}}-(CH_2)-$$

lesdits composés de formule (I) pouvant être sous forme de sels d'addition avec les acides minéraux ou organiques.

**5.** Les produits de formule (I) telle que définie aux revendications 1 à 4, dans laquelle a, b, c, d sont tels que l'un de a ou b forme avec l'un de c ou d une seconde liaison carbone-carbone et les autres représentent un atome d'hydrogène, et ceux dans lesquels a et b forment ensemble un radical oxo et c et d représentent chacun un atome d'hydrogène,
lesdits composés de formule (I) pouvant être sous forme de sels d'addition avec les acides minéraux ou organiques.

**6.** Les dérivés de formule (I) telle que définie à la revendication 1, dont les noms suivent :
- le [2-[3-[(1,1-diméthyl éthyl) amino] 2-hydroxy propoxy] phényl] (1H-indol-4-yl) méthanone,
- le [2-[3-[(1,1-diméthyl éthyl) amino] propoxy) phényl] (1H-indol-4-yl) méthanone,
- le 1-((1,1-diméthyl éthyl) amino] 3-[2-[(1H-indol-4-yl) méthyl] phénoxy] 2-propanol,
- le (±) [2-[3-[(1,1-diméthyléthyl) amino] 2-hydroxypropoxy] phényl] (1-méthyl 1H-indol-4-yl) méthanone,
- le 1,3-dihydro 4-[2-[3-[(1,1-diméthyléthyl) amino] 2-hydroxypropoxy] benzoyl] 2H-indol-2-one,
- le (±) 3-[(1,1-diméthyléthyl) amino] 1-[[2-(1H-indol-4-yl) éthényl] phénoxy] 2-propanol,
- le N-(1,1-diméthyléthyl) 3-[2-[1-(1H-indol-4-yl) éthényl] phénoxy] propanamine,
- l'alpha [2-[3-[(1,1-diméthyléthyl) amino] 2-hydroxypropoxy] phényl 1H-indol-4-méthanol,

ainsi que leurs sels d'addition avec les acides minéraux ou organiques.

**7.** Procédé de préparation des produits de formule (I) telle que définie à la revendication 1, caractérisé en ce que l'on soumet un produit de formule (II) :

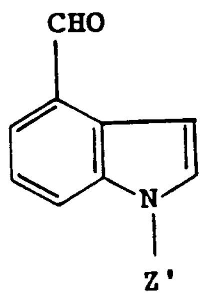

(II)

dans laquelle Z' représente :
soit les valeurs indiquées ci-dessus pour le radical Z, à l'exception de la valeur hydrogène,
soit lesdites valeurs dans lesquelles les fonctions réactives sont protégées,
soit un groupe protecteur,
à une réaction de condensation avec un organo-métallique de formule (III) :

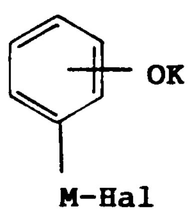

(III)

dans laquelle K représente un groupe protecteur du radical hydroxy, M représente un atome de lithium ou de magnésium, Hal représentant un atome d'halogène, pour obtenir un produit de formule (IV) :

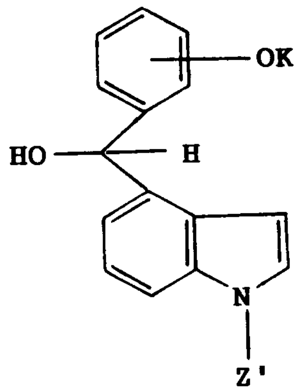

(IV)

que l'on soumet à une réaction d'oxydation pour obtenir le produit de formule (V) :

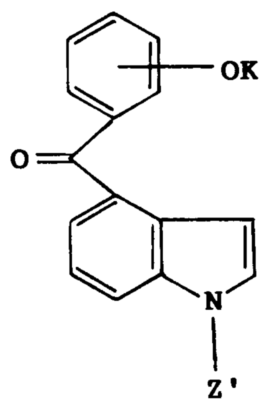

(V)

dont on libère sélectivement le groupe hydroxy pour obtenir le produit de formule (VI) :

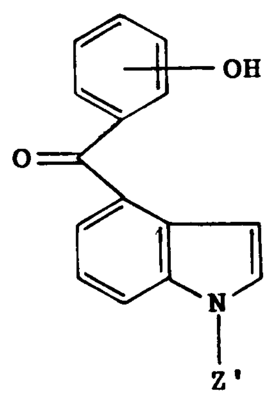

(VI)

et, si désiré, ou bien l'on soumet le produit de formule (VI) à une réaction de réduction de la fonction oxo pour obtenir le produit de formule (VII) :

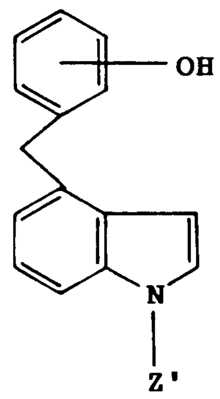

(VII)

ou bien l'on soumet le produit de formule (VI) à l'action d'un reactif organométallique puis à l'action d'un agent de déshydratation pour obtenir un produit de formule (VIII) :

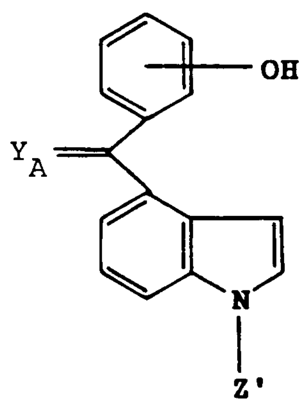

(VIII)

dans laquelle $Y_A$ représente un radical alkylidène renfermant de 1 à 4 atomes de carbone, produit de formule (VIII) que l'on hydrogène si désiré pour obtenir un produit de formule (VIII') :

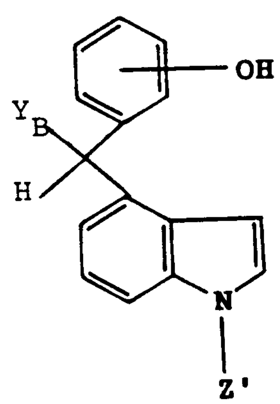

(VIII')

dans laquelle $Y_B$ représente un radical alkyle renfermant de 1 à 4 atomes de carbone,
ou bien l'on transforme le produit de formule (VI) en l'oxime correspondante de formule (IX) :

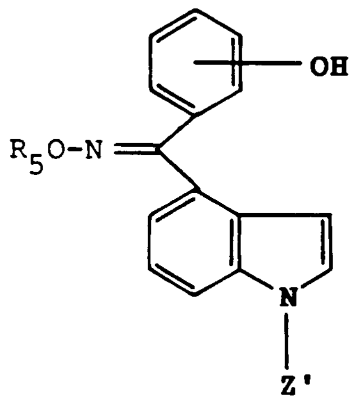

(IX)

dans laquelle $R_5$ a la signification indiquée précédemment, produits de formule (VI), (VII), (VIII), (VIII') ou (IX) que l'on soumet à une réaction de condensation sur le groupe hydroxy,

- soit par le produit de formule (X) :

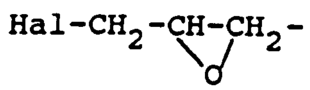

(X)

pour obtenir le produit de formule (XI) :

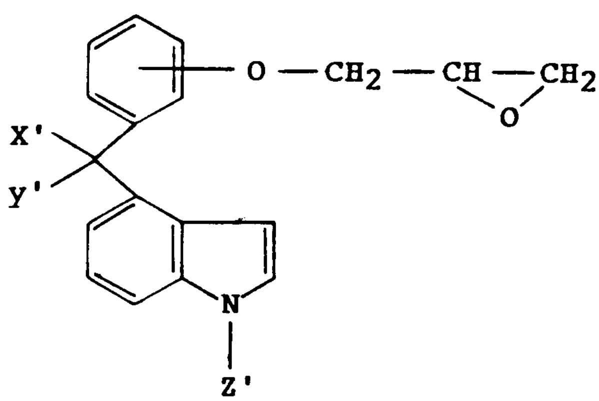

(XI)

dans laquelle X' et Y' représentent un atome d'hydrogène ou forment ensemble une fonction oxo, un radical alkylidène ou un radical $=N\text{-}OR_5$, ou l'un représente un atome d'hydrogène et l'autre représente un radical alkyle renfermant de 1 à 4 atomes de carbone,

- soit par le produit de formule (XII) :

Hal-A'-Hal (XII)

dans laquelle A' représente $-(CH_2)_n$ où n a la signification indiquée à la revendication 1 pour obtenir le produit de formule (XIII) :

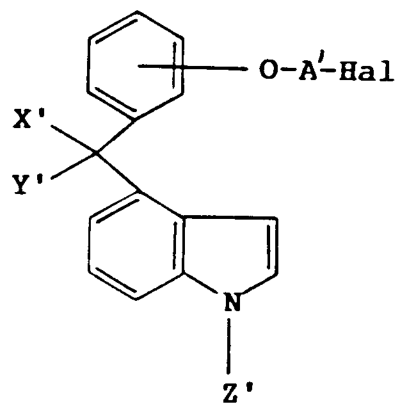

(XIII)

dans laquelle A', Z', X' et Y' ont la signification donnée ci-dessus et soumet les produits de formule (XI) ou (XIII) à une réaction d'addition par l'amine de formule (XIV) :

$NH-(R)(R_1)$ (XIV)

dans laquelle R et $R_1$ ont la signification indiquée ci-dessus, pour obtenir les produits de formule (XV) :

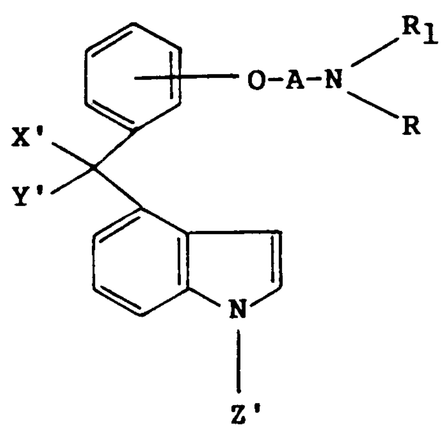

(XV)

que l'on soumet, si nécessaire et si désiré, à l'une ou plusieurs des réactions suivantes, dans un ordre quelconque :
a) coupure éventuelle de Z' ou des groupes protecteurs que porte Z' si nécessaire pour obtenir Z,
b) réduction de la fonction oxo formée par X' et Y' en une fonction alcool, suivie si nécessaire et si désiré d'une alkylation de la fonction hydroxy formée par X ou Y pour obtenir les produits de formule (I) dans laquelle X ou Y représente un radical alkoxy,
c) réduction de la double liaison du noyau pyrrole du radical indole afin d'obtenir les produits de formule (I) dans laquelle a, b, c et d représentent chacun un atome d'hydrogène,
d) halogénation en béta de l'atome d'azote sur le noyau pyrrole du radical indole suivie d'une hydrolyse en milieu acide afin d'obtenir les produits de formule (I) dans laquelle a et b forment une fonction oxo,
e) dédoublement des produits racémiques par des procédés classiques pour obtenir les produits optiquement actifs et salification, si désiré, des produits de formule (I) pour obtenir les sels correspondants.

**8.** A titre de médicaments, les produits de formule (I) tels que définis à l'une quelconque des revendications 1 à 5, ainsi que leurs sels d'addition avec les acides pharmaceutiquement acceptables.

**9.** A titre de médicaments, les produits définis à la revendication 6, ainsi que leurs sels d'addition avec les acides pharmaceutiquement acceptables.

**10.** Les compositions pharmaceutiques contenant à titre de principe actif l'un au moins des médicaments tels que définis par la revendication 8 ou 9.

**11.** Les produits intermédiaires répondant aux formules (IV), (V), (VI), (VIII), (VIII'), (IX), (XI) et (XIII) tels que définis à la revendication 9.

**Revendications pour l'Etat contractant suivant : ES**

**1.** Procédé pour préparer les produits de formule générale (I) :

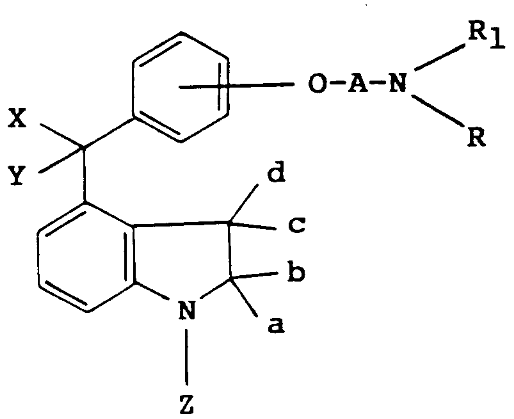

(I)

dans laquelle R et $R_1$ identiques ou différents, représentent : - soit un atome d'hydrogène,

- soit un radical alkyle, alkényle ou alkynyle linéaire ou ramifié ayant au plus 8 atomes de carbone, éventuellement substitué par un radical hydroxy,
- soit un radical cycloalkyle renfermant de 3 à 7 atomes de carbone, un radical cycloalkylalkyle renfermant de 4 à 7 atomes de carbone ou un radical arylalkyle renfermant de 7 à 14 atomes de carbone, ce dernier radical étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 5 atomes de carbone et les radicaux trifluorométhyle, méthylthio, nitro, amino, monoalkylamino ou dialkylamino,

ou R et $R_1$ forment ensemble, avec l'atome d'azote auquel ils sont liés, un hétérocycle saturé ou insaturé pouvant renfermer un second hétéroatome choisi parmi les atomes d'oxygène, de soufre ou d'azote, ce second atome d'azote non lié à A étant éventuellement substitué par :

- soit un radical alkyle renfermant de 1 à 5 atomes de carbone,
- soit un radical phényle lui-même éventuellement substitué par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,
- soit un radical naphtyle,
- soit un radical arylalkyle ou diarylalkyle renfermant de 7 à 14 atomes de carbone, chacun de ces radicaux étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,

A représente :

- soit une chaîne $(CH_2)_n$ dans laquelle n peut prendre les valeurs 2, 3, 4 ou 5
- soit une chaîne

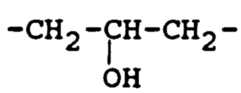

X et Y sont tels que :

- soit chacun représente un atome d'hydrogène,
- soit l'un représente un atome d'hydrogène et l'autre représente un radical hydroxy, un radical alkoxy renfermant de 1 à 4 atomes de carbone ou un radical alkyle renfermant de 1 à 4 atomes de carbone,
- soit X et Y forment ensemble un radical oxo, un radical alkylidène ayant de 1 à 4 atomes de carbone ou un radical =N-$OR_5$ dans lequel $R_5$ représente un atome d'hydrogène ou un radical alkyle renfermant de 1 à 4 atomes de carbone,

les substituants a, b, c, d sont tels que :

- soit ils représentent chacun un atome d'hydrogène
- soit a et b forment ensemble une fonction oxo et c et d représentent chacun un atome d'hydrogène
- soit l'un de a ou b forme avec l'un de c ou d une double liaison et les autres substituants représentent chacun un atome d'hydrogène,

Z représente un atome d'hydrogène, un radical alkyle, alkényle ou alkynyle, chacun de ces radicaux linéaire ou ramifié ayant au plus 5 atomes de carbone, un radical cycloalkylalkyle renfermant de 4 à 7 atomes de carbone, un radical arylalkyle renfermant de 7 à 14 atomes de carbone, chacun de ces radicaux étant éventuellement substitué par 1, 2 ou 3 radicaux choisis parmi le groupe constitué par le radical hydroxy, les halogènes, les radicaux alkyle ou alkyloxy linéaires ou ramifiés ayant au plus 5 atomes de carbone,

ou Z représente un groupe aminoalkyle de formule -$R_2$-N-($R_3$)($R_4$) dans laquelle $R_3$ et $R_4$, identiques ou différents, représentent un atome d'hydrogène, un radical alkyle linéaire ou ramifié renfermant au plus 5 atomes de carbone,

et $R_2$ est un radical alkylène renfermant de 2 à 5 atomes de carbone,

lesdits composés de formule (I) pouvant être sous toutes les formes énantiomères, racémiques ou diastéréoisomères possibles et sous forme de sels d'addition avec les acides minéraux ou organiques, caractérisé en ce que l'on soumet un produit de formule (II) :

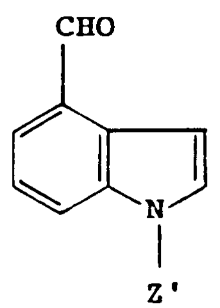

(II)

dans laquelle Z' représente :

soit les valeurs indiquées ci-dessus pour le radical Z, à l'exception de la valeur hydrogène,

soit lesdites valeurs dans lesquelles les fonctions réactives sont protégées,

soit un groupe protecteur,

à une réaction de condensation avec un organo-métallique de formule (III) :

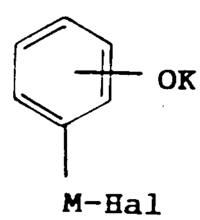

(III)

dans laquelle K représente un groupe protecteur du radical hydroxy, M représente un atome de lithium ou de magnésium, Hal représentant un atome d'halogène, pour obtenir un produit de formule (IV) :

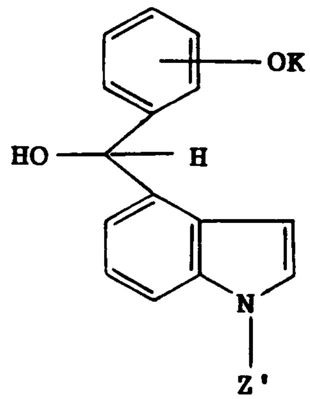

(IV)

que l'on soumet à une réaction d'oxydation pour obtenir le produit de formule (V) :

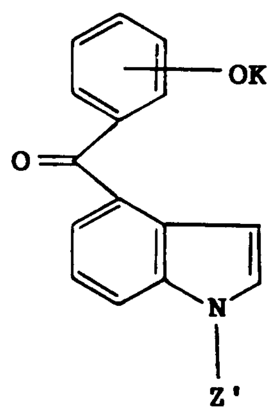

(V)

dont on libère sélectivement le groupe hydroxy pour obtenir le produit de formule (VI) :

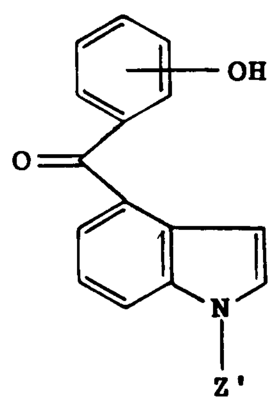

(VI)

et, si désiré, ou bien l'on soumet le produit de formule (VI) à une réaction de réduction de la fonction oxo pour obtenir le produit de formule (VII) :

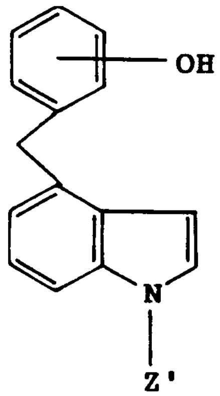

(VII)

ou bien l'on soumet le produit de formule (VI) à l'action d'un réactif organométallique puis à l'action d'un agent de déshydratation pour obtenir un produit de formule (VIII) :

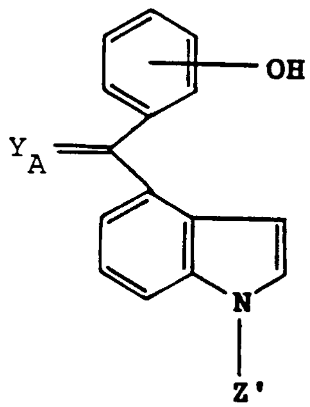

(VIII)

dans laquelle $Y_A$ représente un radical alkylidène renfermant de 1 à 4 atomes de carbone, produit de formule (VIII) que l'on hydrogène si désiré pour obtenir un produit de formule (VIII') :

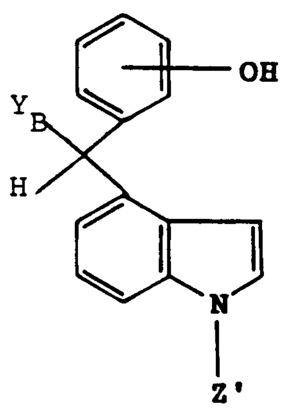

(VIII')

dans laquelle $Y_B$ représente un radical alkyle renfermant de 1 à 4 atomes de carbone,
ou bien l'on transforme le produit de formule (VI) en l'oxime correspondante de formule (IX) :

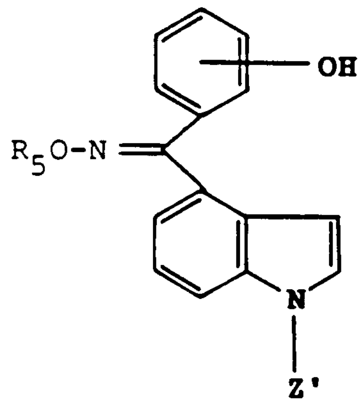

(IX)

dans laquelle $R_5$ a la signification indiquée précédemment, produits de formule (VI), (VII), (VIII), (VIII') ou (IX) que l'on soumet à une réaction de condensation sur le groupe hydroxy,

- soit par le produit de formule (X) :

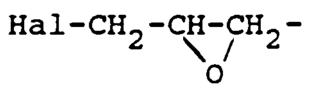

(X)

pour obtenir le produit de formule (XI) :

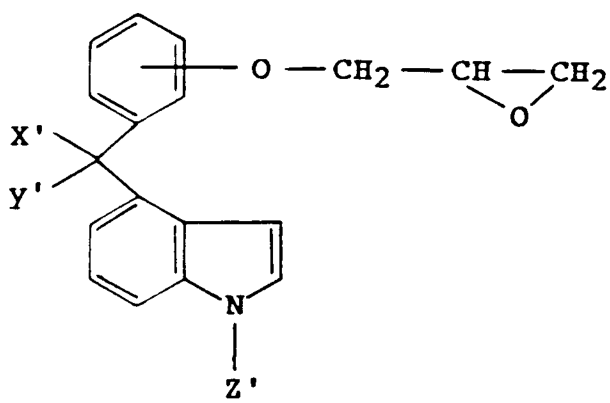

(XI)

dans laquelle X' et Y' représentent un atome d'hydrogène ou forment ensemble une fonction oxo, un radical alkylidène ou un radical $=N\text{-}OR_5$, ou l'un représente un atome d'hydrogène et l'autre représente un radical alkyle renfermant de 1 à 4 atomes de carbone,

- soit par le produit de formule (XII) :

Hal-A'-Hal (XII)

dans laquelle A' représente $-(CH_2)_n$ où n a la signification indiquée précédemment pour obtenir le produit de formule (XIII) :

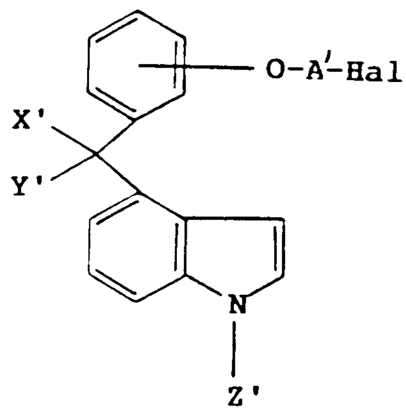

(XIII)

dans laquelle A', Z', X' et Y' ont la signification donnée ci-dessus et soumet les produits de formule (XI) ou (XIII) à une réaction d'addition par l'amine de formule (XIV) :

$NH-(R)(R_1)$ (XIV)

dans laquelle R et $R_1$ ont la signification indiquée ci-dessus, pour obtenir les produits de formule (XV) :

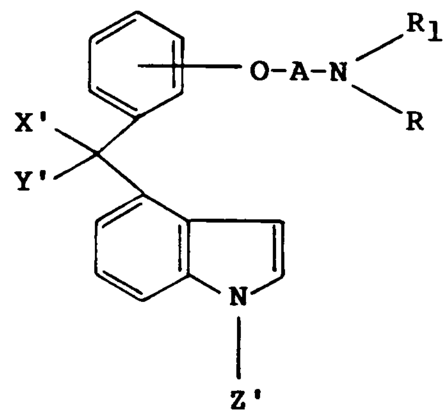

(XV)

que l'on soumet, si nécessaire et si désiré, à l'une ou plusieurs des réactions suivantes, dans un ordre quelconque :
a) coupure éventuelle de Z' ou des groupes protecteurs que porte Z' si nécessaire pour obtenir Z,
b) réduction de la fonction oxo formée par X' et Y' en une fonction alcool, suivie si nécessaire et si désiré d'une alkylation de la fonction hydroxy formée par X ou Y pour obtenir les produits de formule (I) dans laquelle X ou Y représente un radical alkoxy,
c) réduction de la double liaison du noyau pyrrole du radical indole afin d'obtenir les produits de formule (I) dans laquelle a, b, c et d représentent chacun un atome d'hydrogène,
d) halogénation en béta de l'atome d'azote sur le noyau pyrrole du radical indole suivie d'une hydrolyse en milieu acide afin d'obtenir les produits de formule (I) dans laquelle a et b forment une fonction oxo,
e) dédoublement des produits racémiques par des procédés classiques pour obtenir les produits optiquement actifs et salification, si désiré, des produits de formule (I) pour obtenir les sels correspondants.

**2.** Procédé selon la revendication 1 pour préparer des produits de formule (I) telle que définie à la revendication 1 répondant à la formule (I') :

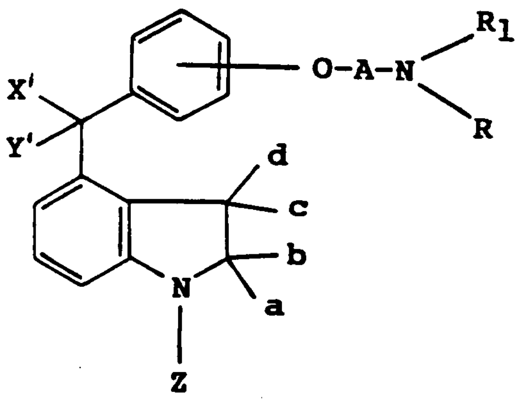

(I')

dans laquelle R, $R_1$, A, a, b, c, d et Z ont la signification indiquée à la revendication 1 et X' et Y' sont tels que :

- soit chacun représente un atome d'hydrogène,
- soit l'un représente un atome d'hydrogène et l'autre représente un radical hydroxy,
- soit X et Y forment ensemble un radical oxo, caractérisé en ce que l'on soumet le produit de formule (VI) ou de formule (VII) telle que définie à la revendication 1 à une réaction de condensation sur le groupe hydroxy,
- soit par le produit de formule (X) :

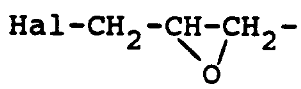

(X)

pour obtenir le produit de formule (XI') :

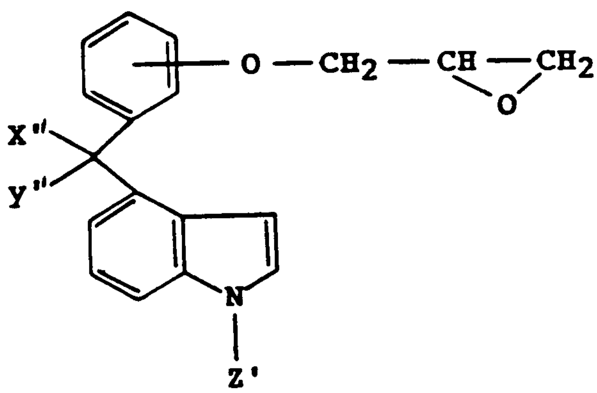

(XI')

dans laquelle X'' et Y'' représentent un atome d'hydrogène ou forment ensemble une fonction oxo,

- soit par le produit de formule (XII) :

Hal-A'-Hal (XII)

dans laquelle A' représente $-(CH_2)_n$ pour obtenir le produit de formule (XIII') :

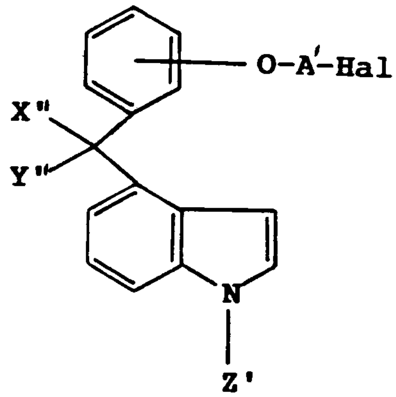

(XIII')

dans laquelle A', Z', X' et Y' ont la signification donnée précédemment et soumet les produits de formule (XI) ou (XIII) à une réaction d'addition par l'amine de formule (XIV) :

NH-(R)($R_1$) (XIV)

dans laquelle R et $R_1$ ont la signification indiquée ci-dessus, pour obtenir les produits de formule (XV') :

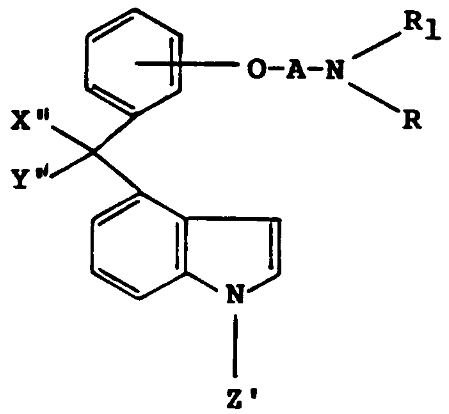

(XV')

que l'on soumet, si nécessaire et si désiré, à l'une ou plusieurs des réactions suivantes, dans un ordre quelconque :
a) coupure de Z' ou des groupes protecteurs que porte Z' pour obtenir Z,
b) réduction de la fonction oxo formée par X' et Y' en une fonction alcool,
c) réduction de la double liaison du noyau pyrrole du radical indole afin d'obtenir les produits de formule (I) dans laquelle a, b, c et d représentent chacun un atome d'hydrogène,
d) halogénation en béta de l'atome d'azote sur le noyau pyrrole du radical indole suivie d'une hydrolyse en milieu acide afin d'obtenir les produits de formule (I) dans laquelle a et b forment une fonction oxo,
e) dédoublement des produits racémiques par des procédés classiques pour obtenir les produits optiquement actifs et salification, si désiré, des produits de formule (I) pour obtenir les sels correspondants.

**3.** Procédé selon la revendication 1 caractérisé en ce que l'on utilise au départ un produit de formule (II) telle que définie à la revendication 1 dans laquelle Z' représente un groupement protecteur ou un radical alkyle renfermant de 1 à 4 atomes de carbone et en ce que l'on soumet le produit de formule (XV) correspondant, le cas échéant à une réaction de coupure de Z'.

**4.** Procédé selon la revendication 1 pour la préparation des produits de formule (I) telle que définie à la revendication 1 dans laquelle R est un atome d'hydrogène, $R_1$ représente un radical alkyle renfermant de 1 à 4 atomes de carbone ou un radical cycloalkyle renfermant de 3 à 7 atomes de carbone ou R et

$R_1$ forment ensemble avec l'atome d'azote auquel ils sont liés un hétérocycle pouvant renfermer un second atome d'azote éventuellement substitué par un radical alkyle renfermant de 1 à 5 atomes de carbone et A est tel que l'entier n a la valeur 2 ou 3 ou A représente le groupe

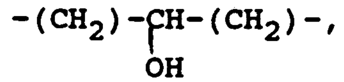

caractérisé en ce que l'on effectue la réaction de condensation sur le groupe hydroxy soit par le produit de formule (X) telle que définie à la revendication 1, soit par le produit de formule (XII) telle que définie à la revendication 1 dans laquelle A' représente un groupement $(CH_2)_n$ dans lequel n a la valeur 2 ou 3 et en ce que l'on soumet le produit de formule (XI) ou de formule (XIII) correspondant à l'action d'une amine de formule (XIV) dans laquelle R et $R_1$ ont les valeurs indiquées ci-dessus.

**Revendications pour l'Etat contractant suivant : GR**

**1.** Procédé pour préparer les produits de formule générale (I) :

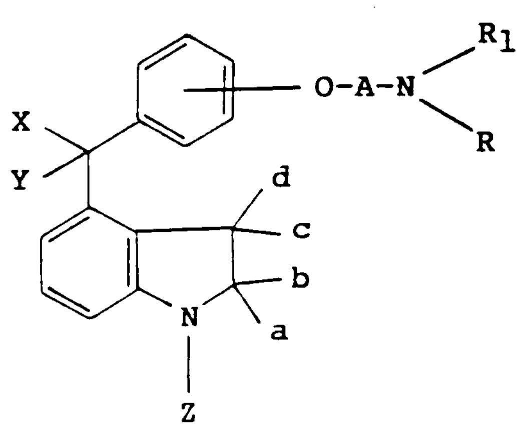

(I)

dans laquelle R et $R_1$ identiques ou différents, représentent : - soit un atome d'hydrogène,

- soit un radical alkyle, alkényle ou alkynyle linéaire ou ramifié ayant au plus 8 atomes de carbone, éventuellement substitué par un radical hydroxy,
- soit un radical cycloalkyle renfermant de 3 à 7 atomes de carbone, un radical cycloalkylalkyle renfermant de 4 à 7 atomes de carbone ou un radical arylalkyle renfermant de 7 à 14 atomes de carbone, ce dernier radical étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 5 atomes de carbone et les radicaux trifluorométhyle, méthylthio, nitro, amino, monoalkylamino ou dialkylamino,

  ou R et $R_1$ forment ensemble, avec l'atome d'azote auquel ils sont liés, un hétérocycle saturé ou insaturé pouvant renfermer un second hétéroatome choisi parmi les atomes d'oxygène, de soufre ou d'azote, ce second atome d'azote non lié à A étant éventuellement substitué par :
- soit un radical alkyle renfermant de 1 à 5 atomes de carbone,
- soit un radical phényle lui-même éventuellement substitué par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,
- soit un radical naphtyle,
- soit un radical arylalkyle ou diarylalkyle renfermant de 7 à 14 atomes de carbone, chacun de ces radicaux étant éventuellement substitué sur le noyau aryle par 1, 2 ou 3 radicaux choisis parmi le groupe constitué par le radical hydroxy, les halogènes, les radicaux alkyle ou alkyloxy linéaires ou ramifiés ayant au plus 3 atomes de carbone et les radicaux trifluorométhyle, méthylthio, nitro, amino, monoalkylamino ou dialkylamino,

  A représente :
- soit une chaîne $(CH_2)_n$ dans laquelle n peut prendre les valeurs 2, 3, 4 ou 5

- soit une chaîne

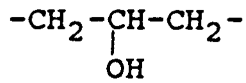

X et Y sont tels que :
- soit chacun représente un atome d'hydrogène,
- soit l'un représente un atome d'hydrogène et l'autre représente un radical hydroxy, un radical alkoxy renfermant de 1 à 4 atomes de carbone ou un radical alkyle renfermant de 1 à 4 atomes de carbone,
- soit X et Y forment ensemble un radical oxo, un radical alkylidène ayant de 1 à 4 atomes de carbone ou un radical $=N-OR_5$ dans lequel $R_5$ représente un atome d'hydrogène ou un radical alkyle renfermant de 1 à 4 atomes de carbone,

les substituants a, b, c, d sont tels que :
- soit ils représentent chacun un atome d'hydrogène
- soit a et b forment ensemble une fonction oxo et c et d représentent chacun un atome d'hydrogène
- soit l'un de a ou b forme avec l'un de c ou d une double liaison et les autres substituants représentent chacun un atome d'hydrogène,

Z représente un atome d'hydrogène, un radical alkyle, alkényle ou alkynyle, chacun de ces radicaux linéaire ou ramifié ayant au plus 5 atomes de carbone, un radical cycloalkylalkyle renfermant de 4 à 7 atomes de carbone, un radical arylalkyle renfermant de 7 à 14 atomes de carbone, chacun de ces radicaux étant éventuellement substitué par 1, 2 ou 3 radicaux choisis parmi le groupe constitué par le radical hydroxy, les halogènes, les radicaux alkyle ou alkyloxy linéaires ou ramifiés ayant au plus 5 atomes de carbone,

ou Z représente un groupe aminoalkyle de formule $-R_2-N-(R_3)(R_4)$ dans laquelle $R_3$ et $R_4$, identiques ou différents, représentent un atome d'hydrogène, un radical alkyle linéaire ou ramifié renfermant au plus 5 atomes de carbone,

et $R_2$ est un radical alkylène renfermant de 2 à 5 atomes de carbone,

lesdits composés de formule (I) pouvant être sous toutes les formes énantiomères, racémiques ou diastéréoisomères possibles et sous forme de sels d'addition avec les acides minéraux ou organiques, caractérisé en ce que l'on soumet un produit de formule (II) :

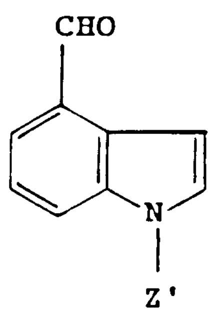

(II)

dans laquelle Z' représente :

soit les valeurs indiquées ci-dessus pour le radical Z, à l'exception de la valeur hydrogène,

soit lesdites valeurs dans lesquelles les fonctions réactives sont protégées,

soit un groupe protecteur,

à une réaction de condensation avec un organo-métallique de formule (III) :

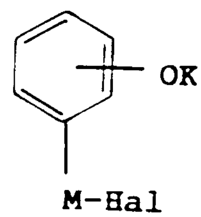

(III)

dans laquelle K représente un groupe protecteur du radical hydroxy, M représente un atome de lithium ou de magnésium, Hal représentant un atome d'halogène, pour obtenir un produit de formule (IV) :

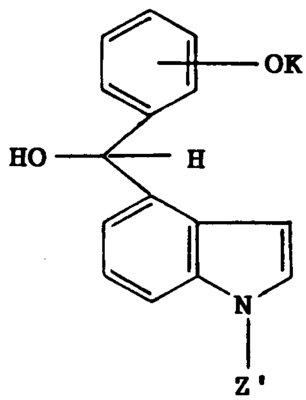

(IV)

que l'on soumet à une réaction d'oxydation pour obtenir le produit de formule (V) :

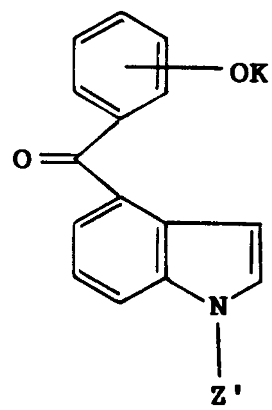

(V)

dont on libère sélectivement le groupe hydroxy pour obtenir le produit de formule (VI) :

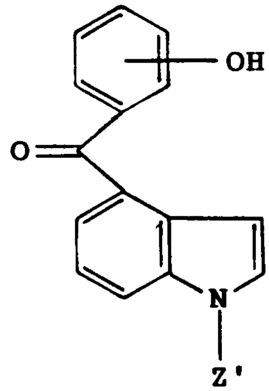

(VI)

et, si désiré, ou bien l'on soumet le produit de formule (VI) à une réaction de réduction de la fonction oxo pour obtenir le produit de formule (VII) :

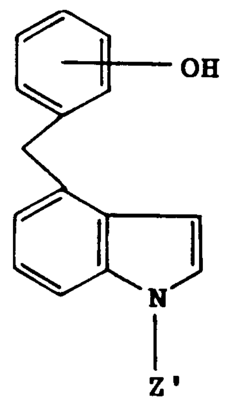

(VII)

ou bien l'on soumet le produit de formule (VI) à l'action d'un réactif organométallique puis à l'action d'un agent de déshydratation pour obtenir un produit de formule (VIII) :

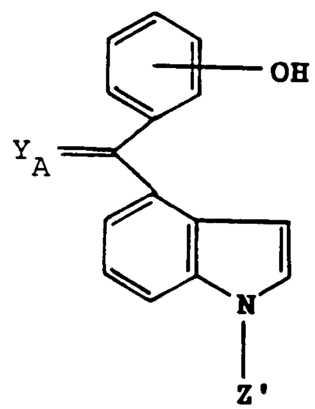

(VIII)

dans laquelle $Y_A$ représente un radical alkylidène renfermant de 1 à 4 atomes de carbone, produit de formule (VIII) que l'on hydrogène si désiré pour obtenir un produit de formule (VIII') :

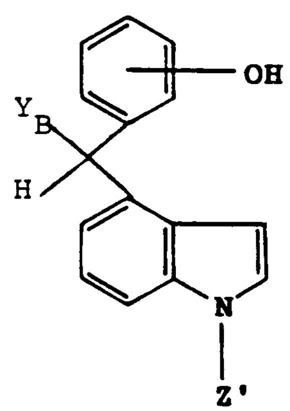

(VIII')

dans laquelle $Y_B$ représente un radical alkyle renfermant de 1 à 4 atomes de carbone,
ou bien l'on transforme le produit de formule (VI) en l'oxime correspondante de formule (IX) :

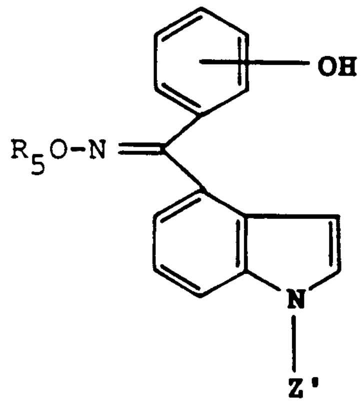

(IX)

dans laquelle $R_5$ a la signification indiquée précédemment, produits de formule (VI), (VII), (VIII), (VIII') ou (IX) que l'on soumet à une réaction de condensation sur le groupe hydroxy,

- soit par le produit de formule (X) :

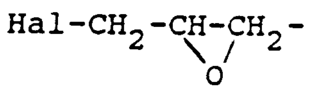

(X)

pour obtenir le produit de formule (XI) :

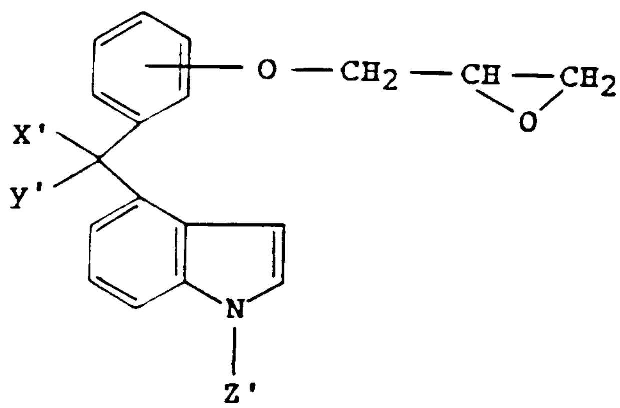

(XI)

dans laquelle X' et Y' représentent un atome d'hydrogène ou forment ensemble une fonction oxo, un radical alkylidène ou un radical $=N\text{-}OR_5$, ou l'un représente un atome d'hydrogène et l'autre représente un radical alkyle renfermant de 1 à 4 atomes de carbone,

- soit par le produit de formule (XII) :

Hal-A'-Hal (XII)

dans laquelle A' représente $-(CH_2)_n$ où n a la signification indiquée précédemment pour obtenir le produit de formule (XIII) :

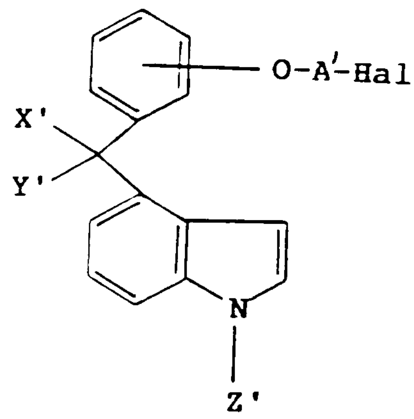

(XIII)

dans laquelle A', Z', X' et Y' ont la signification donnée ci-dessus et soumet les produits de formule (XI) ou (XIII) à une réaction d'addition par l'amine de formule (XIV) :

$NH-(R)(R_1)$ (XIV)

dans laquelle R et $R_1$ ont la signification indiquée ci-dessus, pour obtenir les produits de formule (XV) :

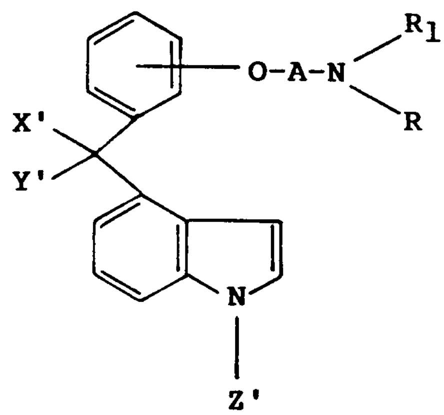

(XV)

que l'on soumet, si nécessaire et si désiré, à l'une ou plusieurs des réactions suivantes, dans un ordre quelconque :

a) coupure éventuelle de Z' ou des groupes protecteurs que porte Z' si nécessaire pour obtenir Z,

b) réduction de la fonction oxo formée par X' et Y' en une fonction alcool, suivie si nécessaire et si désiré d'une alkylation de la fonction hydroxy formée par X ou Y pour obtenir les produits de formule (I) dans laquelle X ou Y représente un radical alkoxy,

c) réduction de la double liaison du noyau pyrrole du radical indole afin d'obtenir les produits de formule (I) dans laquelle a, b, c et d représentent chacun un atome d'hydrogène,

d) halogénation en béta de l'atome d'azote sur le noyau pyrrole du radical indole suivie d'une hydrolyse en milieu acide afin d'obtenir les produits de formule (I) dans laquelle a et b forment une fonction oxo,

e) dédoublement des produits racémiques par des procédés classiques pour obtenir les produits optiquement actifs et salification, si désiré, des produits de formule (I) pour obtenir les sels correspondants.

**2.** Procédé selon la revendication 1 pour préparer des produits de formule (I) telle que définie à la revendication 1 répondant à la formule (I') :

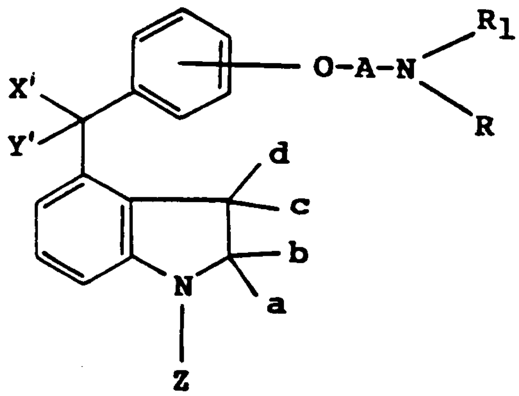

(I')

dans laquelle R, $R_1$, A, a, b, c, d et Z ont la signification indiquée à la revendication 1 et X' et Y' sont tels que :

- soit chacun représente un atome d'hydrogène,
- soit l'un représente un atome d'hydrogène et l'autre représente un radical hydroxy,
- soit X et Y forment ensemble un radical oxo,
  caractérisé en ce que l'on soumet le produit de formule (VI) ou de formule (VII) telle que définie à la revendication 1 à une réaction de condensation sur le groupe hydroxy,
- soit par le produit de formule (X) :

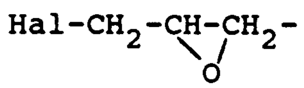

(X)

pour obtenir le produit de formule (XI') :

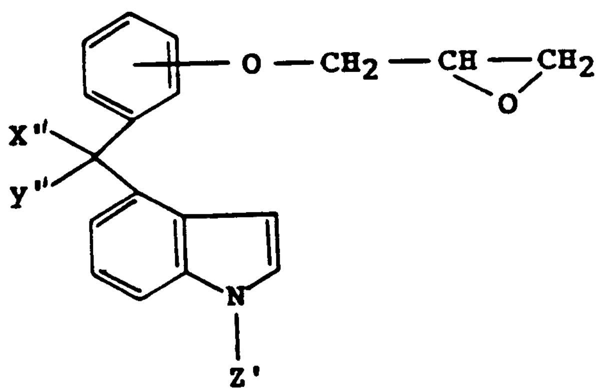

(XI')

dans laquelle X'' et Y'' représentent un atome d'hydrogène ou forment ensemble une fonction oxo,

- soit par le produit de formule (XII) :

Hal-A'-Hal (XII)

dans laquelle A' représente $-(CH_2)_n$ pour obtenir le produit de formule (XIII') :

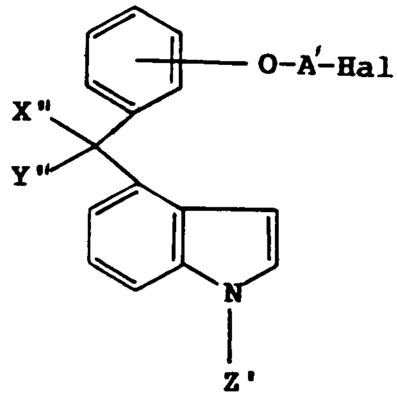

(XIII')

dans laquelle A', Z', X' et Y' ont la signification donnée précédemment et soumet les produits de formule (XI) ou (XIII) à une réaction d'addition par l'amine de formule (XIV) :

NH-(R)($R_1$) (XIV)

dans laquelle R et $R_1$ ont la signification indiquée ci-dessus, pour obtenir les produits de formule (XV') :

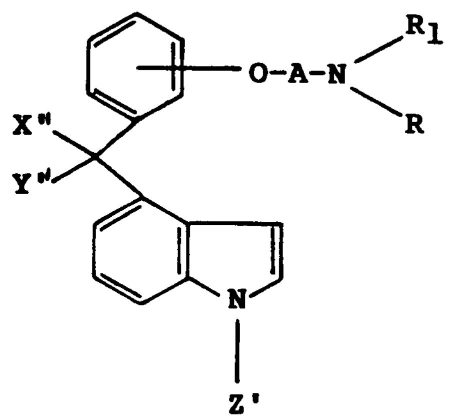

(XV')

que l'on soumet, si nécessaire et si désiré, à l'une ou plusieurs des réactions suivantes, dans un ordre quelconque :
a) coupure de Z' ou des groupes protecteurs que porte Z' pour obtenir Z,
b) réduction de la fonction oxo formée par X' et Y' en une fonction alcool,
c) réduction de la double liaison du noyau pyrrole du radical indole afin d'obtenir les produits de formule (I) dans laquelle a, b, c et d représentent chacun un atome d'hydrogène,
d) halogénation en béta de l'atome d'azote sur le noyau pyrrole du radical indole suivie d'une hydrolyse en milieu acide afin d'obtenir les produits de formule (I) dans laquelle a et b forment une fonction oxo,
e) dédoublement des produits racémiques par des procédés classiques pour obtenir les produits optiquement actifs et salification, si désiré, des produits de formule (I) pour obtenir les sels correspondants.

**3.** Procédé selon la revendication 1 caractérisé en ce que l'on utilise au départ un produit de formule (II) telle que définie à la revendication 1 dans laquelle Z' représente un groupement protecteur ou un radical alkyle renfermant de 1 à 4 atomes de carbone et en ce que l'on soumet le produit de formule (XV) correspondant, le cas échéant à une réaction de coupure de Z'.

**4.** Procédé selon la revendication 1 pour la préparation des produits de formule (I) telle que définie à la revendication 1 dans laquelle A est un atome d'hydrogène, $R_1$ représente un radical alkyle renfermant de 1 à 4 atomes de carbone ou un radical cycloalkyle renfermant de 3 à 7 atomes de carbone ou R et

$R_1$ forment ensemble avec l'atome d'azote auquel ils sont liés un hétérocycle pouvant renfermer un second atome d'azote éventuellement substitué par un radical alkyle renfermant de 1 à 5 atomes de carbone et A est tel que l'entier n a la valeur 2 ou 3 ou A représente le groupe

$$-(CH_2)-\underset{OH}{\underset{|}{CH}}-(CH_2)-,$$

caractérisé en ce que l'on effectue la réaction de condensation sur le groupe hydroxy soit par le produit de formule (X) telle que définie à la revendication 1, soit par le produit de formule (XII) telle que définie à la revendication 1 dans laquelle A' représente un groupement $(CH_2)_n$ dans lequel n a la valeur 2 ou 3 et en ce que l'on soumet le produit de formule (XI) ou de formule (XIII) correspondant à l'action d'une amine de formule (XIV) dans laquelle R et $R_1$ ont les valeurs indiquées ci-dessus.

**5.** Procédé selon la revendication 1 caractérisé en ce que l'on prépare des produits de formule (I) telle que définie à la revendication 1, dans laquelle a, b, c, d sont tels que l'un de a ou b forme avec l'un de c ou d une seconde liaison carbone-carbone et les autres représentent un atome d'hydrogène et dans laquelle a et b forment ensemble un radical oxo et c et d représentent chacun un atome d'hydrogène.

**6.** Procédé selon la revendication 1, caractérisé en ce que l'on prépare l'un quelconque des produits de formule (I) telle que définie à la revendication 1 dont les noms suivent :
- le [2-[3-[(1,1-diméthyl éthyl) amino] 2-hydroxy propoxy] phényl] (1H-indol-4-yl) méthanone,
- le [2-[3-[(1,1-diméthyl éthyl) amino] propoxy] phényl] (1H-indol-4-yl) méthanone,
- le 1-[(1,1-diméthyl éthyl) amino] 3-[2-[(1H-indol-4-yl) méthyl] phénoxy] 2-propanol,
- le (±) [2-[3-[(1,1-diméthyléthyl) amino] 2-hydroxypropoxy] phényl] (1-méthyl 1H-indol-4-yl) méthanone,
- le 1,3-dihydro 4-[2-[3-[(1,1-diméthyléthyl) amino] 2-hydroxypropoxy] benzoyl] 2H-indol-2-one,
- le (±) 3-[(1,1-diméthyléthyl) amino] 1-[[2-(1H-indol-4-yl) éthényl] phénoxy] 2-propanol,
- le N-(1,1-diméthyléthyl) 3-[2-[1-(1H-indol-4-yl) éthényl] phénoxy] propanamine,
- l'alpha [2-[3-[(1,1-diméthyléthyl) amino] 2-hydroxypropoxy] phényl 1H-indol-4-méthanol,

ainsi que leurs sels d'addition avec les acides minéraux ou organiques.

**7.** Procédé selon la revendication 1, caractérisé en ce que l'on prépare l'un quelconque des produits de formule (I') telle que définie à la revendication 2 dont les noms suivent :
- le [2-[3-[(1,1-diméthyl éthyl) amino] 2-hydroxy propoxy] phényl] (1H-indol-4-yl) méthanone,
- le [2-[3-[(1,1-diméthyl éthyl) amino] propoxy] phényl] (1H-indol-4-yl) méthanone,
- le 1-[(1,1-diméthyl éthyl) amino] 3-[2-[(1H-indol-4-yl) méthyl] phénoxy] 2-propanol,
- l'alpha [2-[3-[(1,1-diméthyléthyl) amino] 2-hydroxypropoxy] phényl 1H-indol-4-méthanol,

ainsi que leurs sels d'addition avec les acides minéraux ou organiques.

**8.** Procédé de préparation de compositions pharmaceutiques, caractérisé en ce que l'on met à titre de principe actif l'un au moins des produits de formule (I) telle que définie à la revendication 1 ou l'un au moins de leurs sels d'addition avec les acides minéraux ou organiques pharmaceutiquement acceptables sous une forme destinée à cet usage.

**9.** Procédé de préparation de compositions pharmaceutiques, caractérisé en ce que l'on met à titre de principe actif l'un au moins des produits de formule (I') telle que définie à la revendication 2 ou l'un au moins de leurs sels d'addition avec les acides minéraux ou organiques pharmaceutiquement acceptables sous une forme destinée à cet usage.

**10.** Procédé de préparation de compositions pharmaceutiques, caractérisé en ce que l'on met à titre de principe actif l'un au moins des produits de formule (I) telle que définie à la revendication 8 ou l'un au moins de leurs sels d'addition avec les acides minéraux ou organiques pharmaceutiquement acceptables sous une forme destinée à cet usage.

**11.** Les produits intermédiaires répondant aux formules (IV), (V), (VI), (VIII), (VIII'), (IX), (XI) et (XIII) tels que définis à la revendication 9.

## Claims

**Claims for the following Contracting States : AT, BE, CH, DE, DK, FR, GB, IT, LI, LU, NL, SE**

**1.** The products of general formula (I):

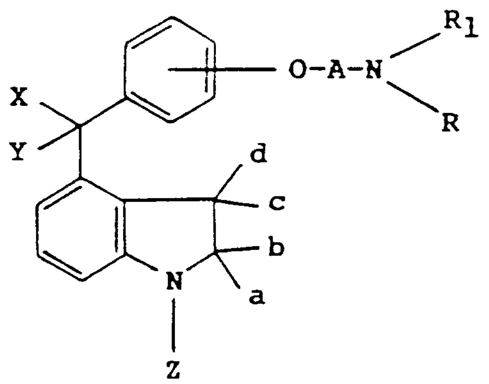

(I)

in which R and $R_1$, identical or different, represent:

- either a hydrogen atom,
- or a linear or branched alkyl, alkenyl or alkynyl radical having at most 8 carbon atoms, optionally substituted by a hydroxy radical,
- or a cycloalkyl radical containing 3 to 7 carbon atoms, a cycloalkylalkyl radical containing 4 to 7 carbon atoms or an arylalkyl radical containing 7 to 14 carbon atoms, the latter radical being optionally substituted on the aryl nucleus by 1, 2 or 3 radicals chosen from the group constituted by the hydroxy radical, halogens, linear or branched alkyl or alkyloxy radicals having at most 5 carbon atoms and trifluoromethyl, methylthio, nitro, amino, monoalkylamino or dialkylamino radicals,
- or R and $R_1$ form together with the nitrogen atom to which they are linked a saturated or unsaturated heterocycle which can contain a second heteroatom chosen from oxygen, sulphur or nitrogen atoms, this second nitrogen atom not linked to A being optionally substituted by:
- either an alkyl radical containing 1 to 5 carbon atoms,
- or a phenyl radical itself optionally substituted by 1, 2 or 3 radicals chosen from the group constituted by the hydroxy radical, halogens, linear or branched alkyl or alkyloxy radicals having at most 3 carbon atoms and trifluoromethyl, methylthio, nitro, amino, monoalkylamino or dialkylamino radicals,
- or a naphthyl radical,
- or an arylalkyl or diarylalkyl radical containing 7 to 14 carbon atoms, each of these radicals being optionally substituted on the aryl nucleus by 1, 2 or 3 radicals chosen from the group constituted by the hydroxy radical, halogens, linear or branched alkyl or alkyloxy radicals having at most 3 carbon atoms and trifluoromethyl, methylthio, nitro, amino, monoalkylamino or dialkylamino radicals,

A represents:

- either a $(CH_2)_n$ chain in which n can take the values 2, 3, 4 or 5
- or a

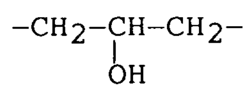

chain

X and Y are such that:

- either each represents a hydrogen atom,
- or one represents a hydrogen atom and the other represents a hydroxy radical, an alkoxy radical containing 1 to 4 carbon atoms or an alkyl radical containing 1 to 4 carbon atoms,
- or X and Y together form an oxo radical, an alkylidene radical having 1 to 4 carbon atoms or an $=N\text{-}OR_5$ radical in which $R_5$ represents a hydrogen atom or an alkyl radical containing 1 to 4

carbon atoms, the substituents a, b, c, d are such that:
- either they each represent a hydrogen atom
- or a and b together form an oxo function and c and d each represent a hydrogen atom
- or one of a or b forms with one of c or d a double bond and the other substituents each represent a hydrogen atom,

Z represents a hydrogen atom, an alkyl, alkenyl or alkynyl radical, each of these linear or branched radicals having at most 5 carbon atoms, a cycloalkylalkyl radical containing 4 to 7 carbon atoms, an arylalkyl radical containing 7 to 14 carbon atoms, each of these radicals being optionally substituted by 1, 2 or 3 radicals chosen from the group constituted by the hydroxy radical, halogens, linear or branched alkyl or alkyloxy radicals having at most 5 carbon atoms,

or Z represents an aminoalkyl group of formula $-R_2-N-(R_3)(R_4)$ in which $R_3$ and $R_4$, identical or different, represent a hydrogen atom, a linear or branched alkyl radical containing at most 5 carbon atoms,

and $R_2$ is an alkylene radical containing 2 to 5 carbon atoms, the said compounds of formula (I) being able to be in all the possible enantiomeric, racemic or diastereoisomeric isomer forms and in the form of addition salts with mineral or organic acids.

**2.** The products of formula (I) as defined in claim 1, in which Z is a hydrogen atom or an alkyl radical containing 1 to 4 carbon atoms, the said compounds of formula (I) being able to be in the form of addition salts with mineral or organic acids.

**3.** The products of formula (I) as defined in any one of claims 1 and 2, in which X and Y represent a hydrogen atom or together form an oxo radical or an alkylidene radical, the said compounds of formula (I) being able to be in the form of addition salts with mineral or organic acids.

**4.** The products of formula (I) as defined in any one of claims 1 to 3, in which R is a hydrogen atom, $R_1$ represents an alkyl radical containing 1 to 4 carbon atoms or a cycloalkyl radical containing 3 to 7 carbon atoms or R and $R_1$ form together with the nitrogen atom to which they are linked a heterocycle which can contain a second nitrogen atom optionally substituted by an alkyl radical containing 1 to 5 carbon atoms and A is such that the integer n has the value 2 or 3 or A represents the group

$$-(CH_2)-\underset{\displaystyle OH}{\underset{|}{CH}}-(CH_2)-$$

the said compounds of formula (I) being able to be in the form of addition salts with mineral or organic acids.

**5.** The products of formula (I) as defined in claims 1 to 4, in which a, b, c, d are such that one of a or b forms with one of c or d a second carbon-carbon bond and the others represent a hydrogen atom, and those in which a and b together form an oxo radical and c and d each represent a hydrogen atom, the said compounds of formula (I) being able to be in the form of addition salts with mineral or organic acids.

**6.** The derivatives of formula (I) as defined in claim 1, of which the names follow:
- [2-[3-[(1,1-dimethyl ethyl) amino] 2-hydroxy propoxy] phenyl] (1H-indol-4-yl) methanone,
- [2-[3-[(1,1-dimethyl ethyl) amino] propoxy] phenyl] (1H-indol-4-yl) methanone,
- 1-[(1,1-dimethyl ethyl) amino] 3-[2-[(1H-indol-4-yl) methyl] phenoxy] 2-propanol,
- (±) [2-[3-[(1,1-dimethylethyl) amino] 2-hydroxypropoxy] phenyl] (1-methyl 1H-indol-4-yl) methanone,
- 1,3-dihydro 4-[2-[3-[(1,1-dimethylethyl) amino] 2-hydroxypropoxy] benzoyl] 2H-indol-2-one,
- (±) 3-[(1,1-dimethylethyl) amino] 1-[[2-(1H-indol-4-yl) ethenyl] phenoxy] 2-propanol,
- N-(1,1-dimethylethyl) 3-[2-[1-(1H-indol-4-yl) ethenyl] phenoxy] propanamine,
- alpha [2-[3-[(1,1-dimethylethyl) amino] 2-hydroxypropoxy] phenyl 1H-indol-4-methanol,

as well as their addition salts with mineral or organic acids.

**7.** Preparation process for the products of formula (I) as defined in claim 1, characterized in that a product of formula (II):

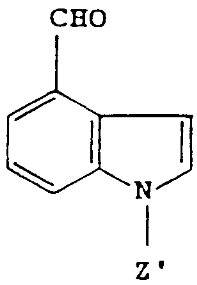

(II)

in which Z' represents:
either the values indicated above for the Z radical, with the exception of the hydrogen value,
or the said values in which the reactive functions are protected,
or a protector group,
is subjected to a condensation reaction with an organometallic compound of formula (III):

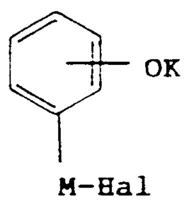

(III)

in which K represents a protector group of the hydroxy radical, M represents a lithium or magnesium atom, Hal representing a halogen atom, in order to obtain a product of formula (IV):

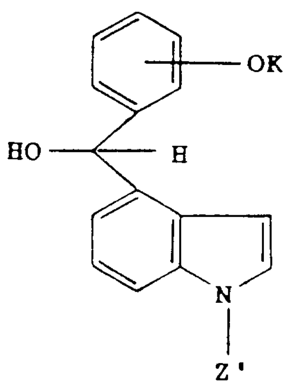

(IV)

which is subjected to an oxidation reaction in order to obtain the product of formula (V):

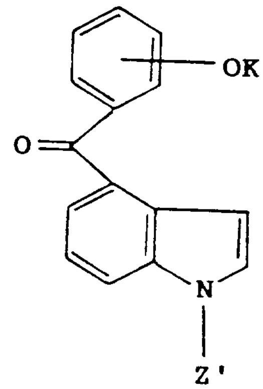

(V)

the hydroxy group of which is selectively released in order to obtain the product of formula (VI):

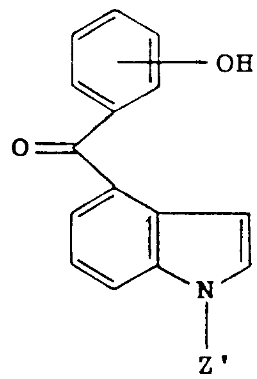

(VI)

and, if desired, either the product of formula (VI) is subjected to a reduction reaction of the oxo function in order to obtain the product of formula (VII):

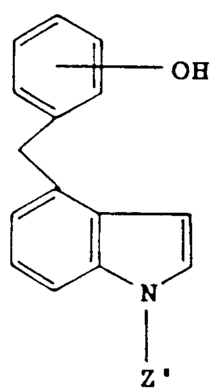

(VII)

or the product of formula (VI) is subjected to the action of an organometallic reagent then to the action of a dehydration agent in order to obtain a product of formula (VIII):

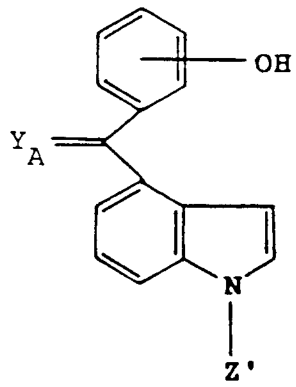

(VIII)

in which $Y_A$ represents an alkylidene radical containing 1 to 4 carbon atoms, which product of formula (VIII) is hydrogenated if desired in order to obtain a product of formula (VIII'):

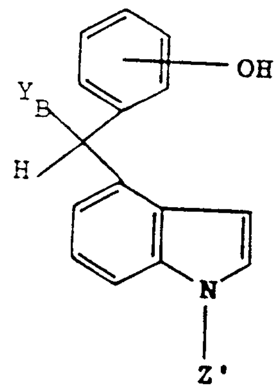

(VIII')

in which $Y_B$ represents an alkyl radical containing 1 to 4 carbon atoms,
<u>or</u> the product of formula (VI) is converted into the corresponding oxime of formula (IX):

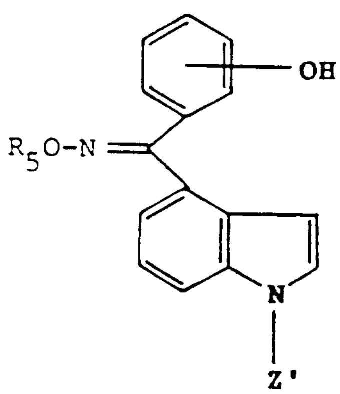

(IX)

in which $R_5$ has the meaning indicated previously, which products of formulae (VI), (VII), (VIII), (VIII') or (IX) are subjected to a condensation reaction on the hydroxy group,

- either by the product of formula (X):

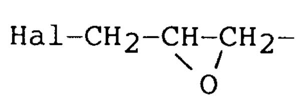

(X)

in order to obtain the product of formula (XI):

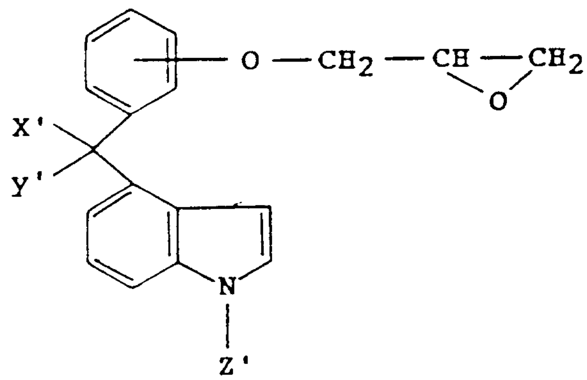

(XI)

in which X' and Y' represent a hydrogen atom or together form an oxo function, an alkylidene radical or an $=N\text{-}OR_5$ radical, or one represents a hydrogen atom and the other represents an alkyl radical containing 1 to 4 carbon atoms,

- or by the product of formula (XII):

Hal-A'-Hal (XII)

in which A' represents $-(CH_2)_n$ where n has the meaning indicated in claim 1, in order to obtain the product of formula (XIII):

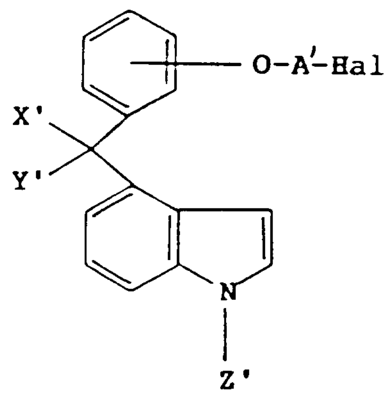

(XIII)

in which A', Z', X' and Y' have the meaning given above and the products of formula (XI) or (XIII) are subjected to an addition reaction by the amine of formula (XIV):

$NH\text{-}(R)(R_1)$ (XIV)

in which R and $R_1$ have the meaning indicated above, in order to obtain the products of formula (XV):

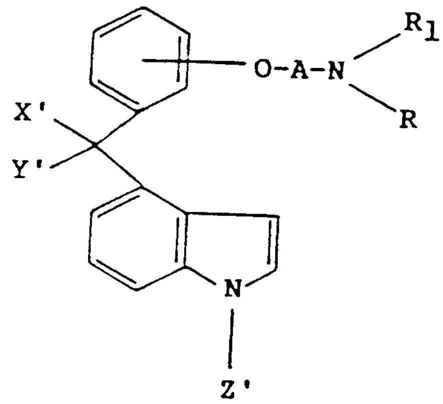

(XV)

which are subjected, if necessary and if desired, to one or more of the following reactions, in any order:

a) optional cleavage of Z' or of the protector groups carried by Z' if necessary to obtain Z,

b) reduction of the oxo function formed by X' and Y' into an alcohol function, followed if necessary and if desired by an alkylation of the hydroxy function formed by X or Y in order to obtain the products of formula (I) in which X or Y represents an alkoxy radical,

c) reduction of the double bond of the pyrrole nucleus of the indole radical in order to obtain the products of formula (I) in which a, b, c and d each represent a hydrogen atom,

d) halogenation in beta position of the nitrogen atom on the pyrrole nucleus of the indole radical followed by a hydrolysis in an acid medium in order to obtain the products of formula (I) in which a and b form an oxo function,

e) resolution of the racemic products by standard processes in order to obtain the optically active products and salification, if desired, of the products of formula (I) in order to obtain the corresponding salts.

**8.** As medicaments, the products of formula (I) as defined in any one of claims 1 to 5, as well as their addition salts with pharmaceutically acceptable acids.

**9.** As medicaments, the products defined in claim 6, as well as their addition salts with pharmaceutically acceptable acids.

**10.** The pharmaceutical compositions containing as active ingredient at least one of the medicaments as defined by claim 8 or 9.

**11.** The intermediate products corresponding to formulae (IV), (V), (VI), (VIII), (VIII'), (IX), (XI) and (XIII) as defined in claim 9.

**Claims for the following Contracting State : ES**

**1.** Process for preparing the products of general formula (I):

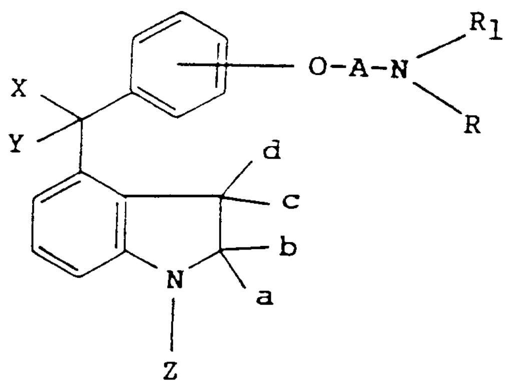

(I)

in which R and $R_1$, identical or different, represent:

- either a hydrogen atom,
- or a linear or branched alkyl, alkenyl or alkynyl radical having at most 8 carbon atoms, optionally substituted by a hydroxy radical,
- or a cycloalkyl radical containing 3 to 7 carbon atoms, a cycloalkylalkyl radical containing 4 to 7 carbon atoms or an arylalkyl radical containing 7 to 14 carbon atoms, the latter radical being optionally substituted on the aryl nucleus by 1, 2 or 3 radicals chosen from the group constituted by the hydroxy radical, halogens, linear or branched alkyl or alkyloxy radicals having at most 5 carbon atoms and trifluoromethyl, methylthio, nitro, amino, monoalkylamino or dialkylamino radicals,
- or R and $R_1$ form together with the nitrogen atom to which they are linked a saturated or unsaturated heterocycle which can contain a second heteroatom chosen from oxygen, sulphur or nitrogen atoms, this second nitrogen atom not linked to A being optionally substituted by:
- either an alkyl radical containing 1 to 5 carbon atoms,
- or a phenyl radical itself optionally substituted by 1, 2 or 3 radicals chosen from the group constituted by the hydroxy radical, halogens, linear or branched alkyl or alkyloxy radicals having at most 3 carbon atoms and trifluoromethyl, methylthio, nitro, amino, monoalkylamino or dialkylamino radicals,
- or a naphthyl radical,
- or an arylalkyl or diarylalkyl radical containing 7 to 14 carbon atoms, each of these radicals being optionally substituted on the aryl nucleus by 1, 2 or 3 radicals chosen from the group constituted by the hydroxy radical, halogens, linear or branched alkyl or alkyloxy radicals having at most 3 carbon atoms and trifluoromethyl, methylthio, nitro, amino, monoalkylamino or dialkylamino radicals,

A represents:

- either a $(CH_2)_n$ chain in which n can take the values 2, 3, 4 or 5
- or a

$$-CH_2-\underset{\displaystyle OH}{\underset{|}{CH}}-CH_2-$$

chain

X and Y are such that:

- either each represents a hydrogen atom,
- or one represents a hydrogen atom and the other represents a hydroxy radical, an alkoxy radical containing 1 to 4 carbon atoms or an alkyl radical containing 1 to 4 carbon atoms,
- or X and Y together form an oxo radical, an alkylidene radical having 1 to 4 carbon atoms or an $=N\text{-}OR_5$ radical in which $R_5$ represents a hydrogen atom or an alkyl radical containing 1 to 4 carbon atoms, the substituents a, b, c, d are such that:

- either they each represent a hydrogen atom
- or a and b together form an oxo function and c and d each represent a hydrogen atom
- or one of a or b forms with one of c or d a double bond and the other substituents each represent a hydrogen atom,

Z represents a hydrogen atom, an alkyl, alkenyl or alkynyl radical, each of these linear or branched radicals having at most 5 carbon atoms, a cycloalkylalkyl radical containing 4 to 7 carbon atoms, an arylalkyl radical containing 7 to 14 carbon atoms, each of these radicals being optionally substituted by 1, 2 or 3 radicals chosen from the group constituted by the hydroxy radical, halogens, linear or branched alkyl or alkyloxy radicals having at most 5 carbon atoms,

or Z represents an aminoalkyl group of formula $-R_2-N-(R_3)(R_4)$ in which $R_3$ and $R_4$, identical or different, represent a hydrogen atom, a linear or branched alkyl radical containing at most 5 carbon atoms,

and $R_2$ is an alkylene radical containing 2 to 5 carbon atoms, the said compounds of formula (I) being able to be in all the possible enantiomeric, racemic or diastereoisomeric isomer forms and in the form of addition salts with mineral or organic acids, characterized in that a product of formula (II):

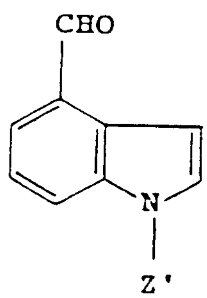

(II)

in which Z' represents:

either the values indicated above for the Z radical, with the exception of the hydrogen value,

or the said values in which the reactive functions are protected,

or a protector group,

is subjected to a condensation reaction with an organometallic compound of formula (III):

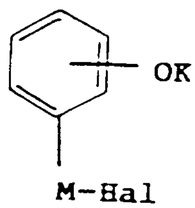

(III)

in which K represents a protector group of the hydroxy radical, M represents a lithium or magnesium atom, Hal representing a halogen atom, in order to obtain a product of formula (IV):

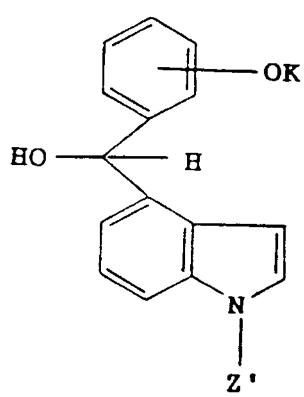

(IV)

which is subjected to an oxidation reaction in order to obtain the product of formula (V):

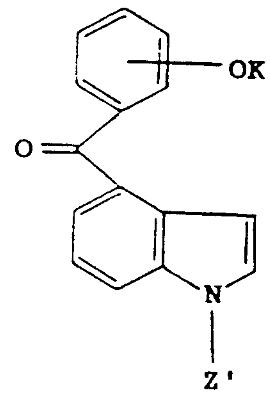

(V)

the hydroxy group of which is selectively released in order to obtain the product of formula (VI):

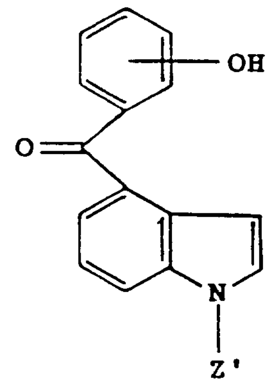

(VI)

and, if desired, either the product of formula (VI) is subjected to a reduction reaction of the oxo function in order to obtain the product of formula (VII):

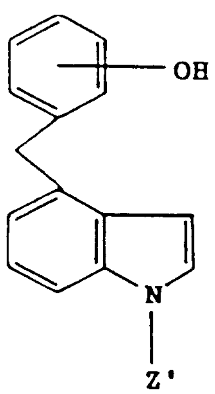

(VII)

or the product of formula (VI) is subjected to the action of an organometallic reagent then to the action of a dehydration agent in order to obtain a product of formula (VIII):

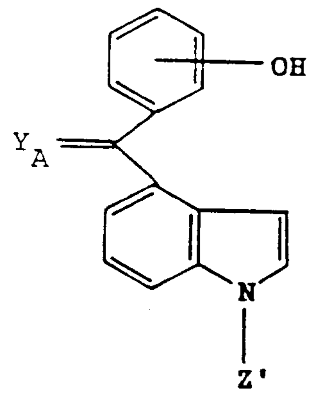

(VIII)

in which $Y_A$ represents an alkylidene radical containing 1 to 4 carbon atoms, which product of formula (VIII) is hydrogenated if desired in order to obtain a product of formula (VIII'):

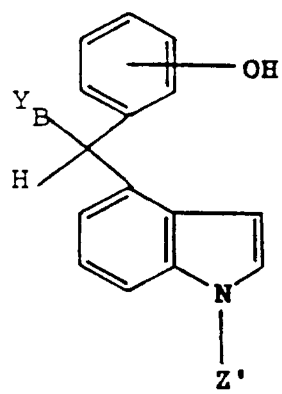

(VIII')

in which $Y_B$ represents an alkyl radical containing 1 to 4 carbon atoms,
or the product of formula (VI) is converted into the corresponding oxime of formula (IX):

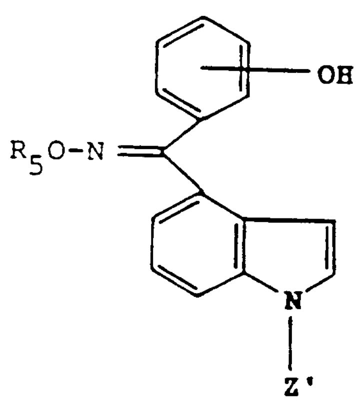

(IX)

in which $R_5$ has the meaning indicated previously, which products of formulae (VI), (VII), (VIII), (VIII') or (IX) are subjected to a condensation reaction on the hydroxy group, - either by the product of formula (X):

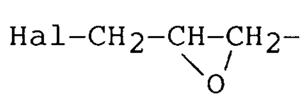

(X)

in order to obtain the product of formula (XI):

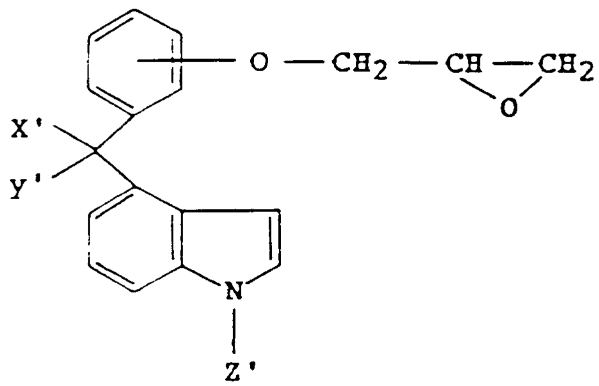

(XI)

in which X' and Y' represent a hydrogen atom or together form an oxo function, an alkylidene radical or an $=N-OR_5$ radical, or one represents a hydrogen atom and the other represents an alkyl radical containing 1 to 4 carbon atoms,

- or by the product of formula (XII):

Hal-A'-Hal (XII)

in which A' represents $-(CH_2)_n$ where n has the meaning indicated previously, in order to obtain the product of formula (XIII):

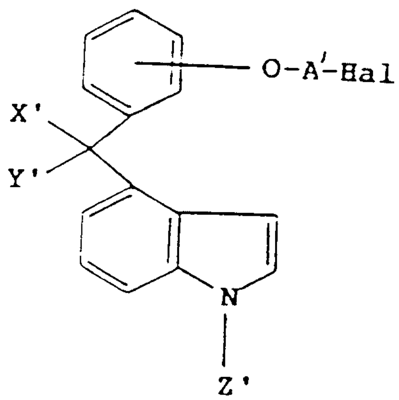

(XIII)

in which A', Z', X' and Y' have the meaning given above and the products of formula (XI) or (XIII) are subjected to an addition reaction by the amine of formula (XIV):

$NH-(R)(R_1)$ (XIV)

in which R and $R_1$ have the meaning indicated above, in order to obtain the products of formula (XV):

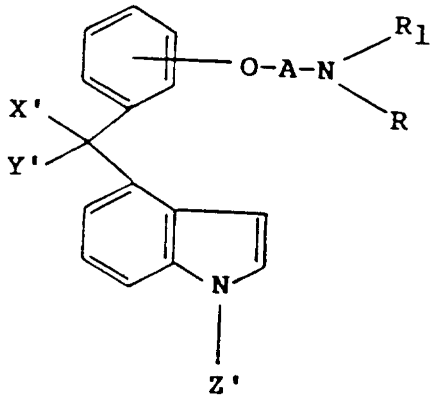

(XV)

which are subjected, if necessary and if desired, to one or more of the following reactions, in any order:

a) optional cleavage of Z' or of the protector groups carried by Z' if necessary to obtain Z,

b) reduction of the oxo function formed by X' and Y' into an alcohol function, followed if necessary and if desired by an alkylation of the hydroxy function formed by X or Y in order to obtain the products of formula (I) in which X or Y represents an alkoxy radical,

c) reduction of the double bond of the pyrrole nucleus of the indole radical in order to obtain the products of formula (I) in which a, b, c and d each represent a hydrogen atom,

d) halogenation in beta position of the nitrogen atom on the pyrrole nucleus of the indole radical followed by a hydrolysis in an acid medium in order to obtain the products of formula (I) in which a and b form an oxo function,

e) resolution of the racemic products by standard processes in order to obtain the optically active products and salification, if desired, of the products of formula (I) in order to obtain the corresponding salts.

**2.** Process according to claim 1 for preparing the products of formula (I) as defined in claim 1 corresponding to formula (I'):

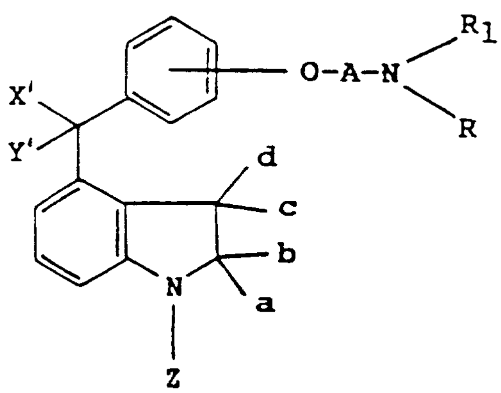

(I')

in which R, $R_1$, A, a, b, c, d and Z having the meaning indicated in claim 1 and X' and Y' are such that:

- either each represents a hydrogen atom,
- or one represents a hydrogen atom and the other represents a hydroxy radical,
- or X and Y together form an oxo radical,

characterized in that the product of formula (VI) or of formula (VII) as defined in claim 1 is subjected to a condensation reaction on the hydroxy group,

- either by the product of formula (X):

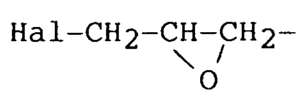

(X)

in order to obtain the product of formula (XI'):

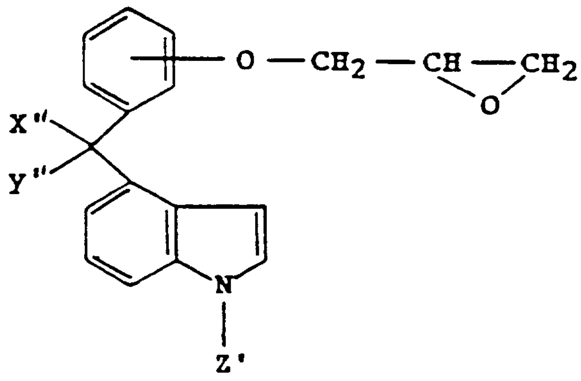

(XI')

in which X'' and Y'' represent a hydrogen atom or together form an oxo function,

- or by the product of formula (XII):

Hal-A'-Hal (XII)

in which A' represents $-(CH_2)_n$, in order to obtain the product of formula (XIII'):

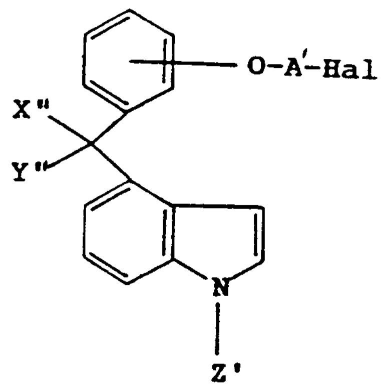

(XIII')

in which A', Z', X' and Y' have the meaning given previously, and the products of formula (XI) or (XIII) are subjected to an addition reaction by the amine of formula (XIV):

$NH-(R)(R_1)$ (XIV)

in which R and $R_1$ have the meaning indicated above, in order to obtain the products of formula (XV'):

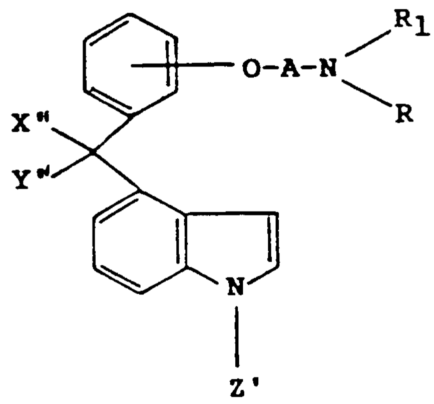

(XV')

which are subjected, if necessary and if desired, to one or more of the following reactions, in any order:

a) cleavage of Z' or of the protector groups carried by Z' in order to obtain Z,

b) reduction of the oxo function formed by X' and Y' into an alcohol function,

c) reduction of the double bond of the pyrrole nucleus of the indole radical in order to obtain the products of formula (I) in which a, b, c and d each represent a hydrogen atom,

d) halogenation in beta position of the nitrogen atom on the pyrrole nucleus of the indole radical followed by a hydrolysis in an acid medium in order to obtain the products of formula (I) in which a and b form an oxo function,

e) resolution of the racemic products by standard processes in order to obtain the optically active products and salification, if desired, of the products of formula (I) in order to obtain the corresponding salts.

**3.** Process according to claim 1 characterized in that a product of formula (II) as defined in claim 1 is used at the start in which Z' represents a protector group or an alkyl radical containing 1 to 4 carbon atoms and in that the corresponding product of formula (XV) is subjected, if appropriate, to a cleavage reaction of Z'.

**4.** Process according to claim 1 for the preparation of the products of formula (I) as defined in claim 1 in which R is a hydrogen atom, $R_1$ represents an alkyl radical containing 1 to 4 carbon atoms or a cycloalkyl radical containing 3 to 7 carbon atoms or R and $R_1$ form together with the nitrogen atom to which they are linked a heterocycle which can contain a second nitrogen atom optionally substituted by an alkyl radical containing 1 to 5 carbon atoms and A is such that the integer n has the value 2 or 3 or A represents the group

$$-(CH_2)-\underset{\displaystyle OH}{\underset{|}{CH}}-(CH_2)-,$$

characterized in that the condensation reaction on the hydroxy group is carried out either by the product of formula (X) as defined in claim 1, or by the product of formula (XII) as defined in claim 1 in which A' represents a $(CH_2)_n$ group in which n has the value 2 or 3 and in that the corresponding product of formula (XI) or of formula (XIII) is subjected to the action of an amine of formula (XIV) in which R and $R_1$ have the values indicated above.

**Claims for the following Contracting State : GR**

**1.** Process for preparing the products of general formula (I):

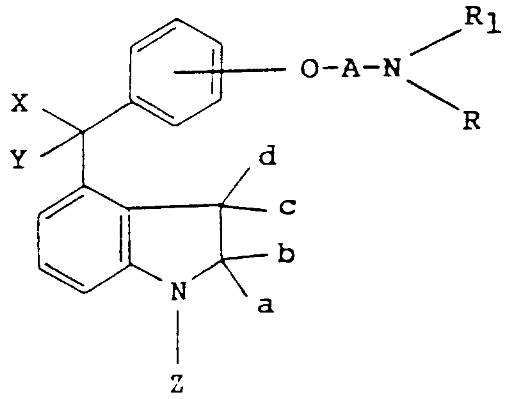

(I)

in which R and $R_1$, identical or different, represent:

- either a hydrogen atom,
- or a linear or branched alkyl, alkenyl or alkynyl radical having at most 8 carbon atoms, optionally substituted by a hydroxy radical,
- or a cycloalkyl radical containing 3 to 7 carbon atoms, a cycloalkylalkyl radical containing 4 to 7 carbon atoms or an arylalkyl radical containing 7 to 14 carbon atoms, the latter radical being optionally substituted on the aryl nucleus by 1, 2 or 3 radicals chosen from the group constituted by the hydroxy radical, halogens, linear or branched alkyl or alkyloxy radicals having at most 5 carbon atoms and trifluoromethyl, methylthio, nitro, amino, monoalkylamino or dialkylamino radicals,
- or R and $R_1$ form together with the nitrogen atom to which they are linked a saturated or unsaturated heterocycle which can contain a second heteroatom chosen from oxygen, sulphur or nitrogen atoms, this second nitrogen atom not linked to A being optionally substituted by:
- either an alkyl radical containing 1 to 5 carbon atoms,
- or a phenyl radical itself optionally substituted by 1, 2 or 3 radicals chosen from the group constituted by the hydroxy radical, halogens, linear or branched alkyl or alkyloxy radicals having at most 3 carbon atoms and trifluoromethyl, methylthio, nitro, amino, monoalkylamino or dialkylamino radicals,
- or a naphthyl radical,
- or an arylalkyl or diarylalkyl radical containing 7 to 14 carbon atoms, each of these radicals being optionally substituted on the aryl nucleus by 1, 2 or 3 radicals chosen from the group constituted by the hydroxy radical, halogens, linear or branched alkyl or alkyloxy radicals having at most 3 carbon atoms and trifluoromethyl, methylthio, nitro, amino, monoalkylamino or dialkylamino radicals,

A represents:

- either a $(CH_2)_n$ chain in which n can take the values 2, 3, 4 or 5
- or a

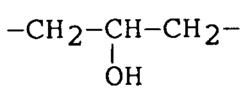

chain

X and Y are such that:

- either each represents a hydrogen atom,
- or one represents a hydrogen atom and the other represents a hydroxy radical, an alkoxy radical containing 1 to 4 carbon atoms or an alkyl radical containing 1 to 4 carbon atoms,

- or X and Y together form an oxo radical, an alkylidene radical having 1 to 4 carbon atoms or an =N-$OR_5$ radical in which $R_5$ represents a hydrogen atom or an alkyl radical containing 1 to 4 carbon atoms, the substituents a, b, c, d are such that:
- either they each represent a hydrogen atom
- or a and b together form an oxo function and c and d each represent a hydrogen atom
- or one of a or b forms with one of c or d a double bond and the other substituents each represent a hydrogen atom,

Z represents a hydrogen atom, an alkyl, alkenyl or alkynyl radical, each of these linear or branched radicals having at most 5 carbon atoms, a cycloalkylalkyl radical containing 4 to 7 carbon atoms, an arylalkyl radical containing 7 to 14 carbon atoms, each of these radicals being optionally substituted by 1, 2 or 3 radicals chosen from the group constituted by the hydroxy radical, halogens, linear or branched alkyl or alkyloxy radicals having at most 5 carbon atoms,

or Z represents an aminoalkyl group of formula -$R_2$-N-($R_3$)($R_4$) in which $R_3$ and $R_4$, identical or different, represent a hydrogen atom, a linear or branched alkyl radical containing at most 5 carbon atoms,

and $R_2$ is an alkylene radical containing 2 to 5 carbon atoms, the said compounds of formula (I) being able to be in all the possible enantiomeric, racemic or diastereoisomeric isomer forms and in the form of addition salts with mineral or organic acids, characterized in that a product of formula (II):

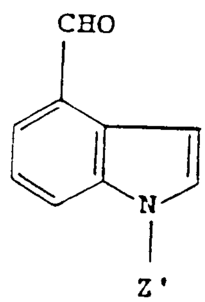

(II)

in which Z' represents:

either the values indicated above for the Z radical, with the exception of the hydrogen value,

or the said values in which the reactive functions are protected,

or a protector group,

is subjected to a condensation reaction with an organometallic compound of formula (III):

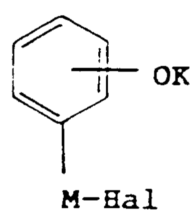

(III)

in which K represents a protector group of the hydroxy radical, M represents a lithium or magnesium atom, Hal representing a halogen atom, in order to obtain a product of formula (IV):

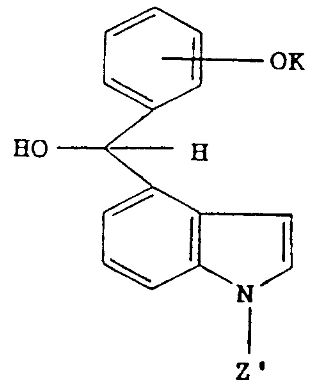

(IV)

which is subjected to an oxidation reaction in order to obtain the product of formula (V):

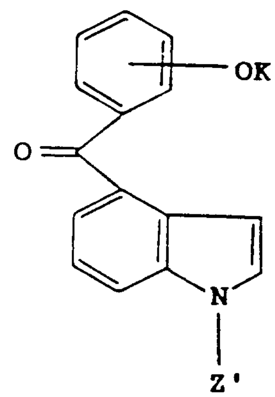

(V)

the hydroxy group of which is selectively released in order to obtain the product of formula (VI):

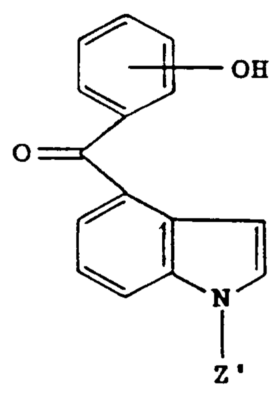

(VI)

and, if desired, either the product of formula (VI) is subjected to a reduction reaction of the oxo function in order to obtain the product of formula (VII):

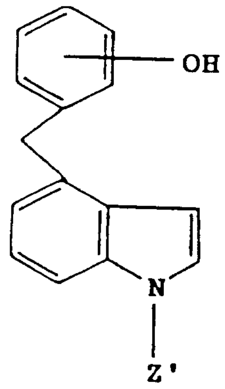

(VII)

or the product of formula (VI) is subjected to the action of an organometallic reagent then to the action of a dehydration agent in order to obtain a product of formula (VIII):

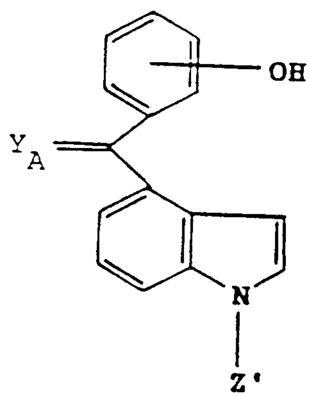

(VIII)

in which $Y_A$ represents an alkylidene radical containing 1 to 4 carbon atoms, which product of formula (VIII) is hydrogenated if desired in order to obtain a product of formula (VIII'):

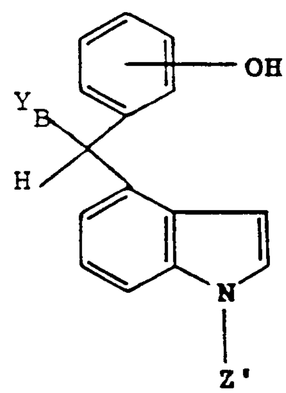

(VIII')

in which $Y_B$ represents an alkyl radical containing 1 to 4 carbon atoms,
or the product of formula (VI) is converted into the corresponding oxime of formula (IX):

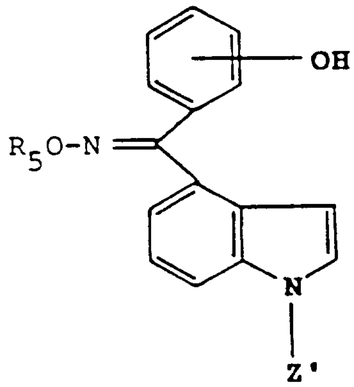

(IX)

in which $R_5$ has the meaning indicated previously, which products of formulae (VI), (VII), (VIII), (VIII') or (IX) are subjected to a condensation reaction on the hydroxy group,

- either by the product of formula (X):

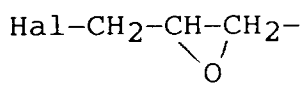

(X)

in order to obtain the product of formula (XI):

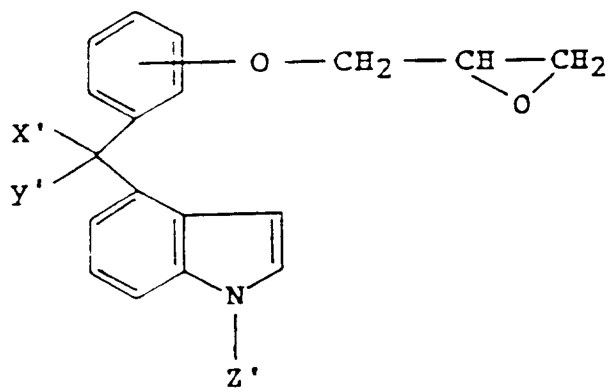

(XI)

in which X' and Y' represent a hydrogen atom or together form an oxo function, an alkylidene radical or an $=N\text{-}OR_5$ radical, or one represents a hydrogen atom and the other represents an alkyl radical containing 1 to 4 carbon atoms,

- or by the product of formula (XII):

Hal-A'-Hal (XII)

in which A' represents $-(CH_2)_n$ where n has the meaning indicated previously, in order to obtain the product of formula (XIII):

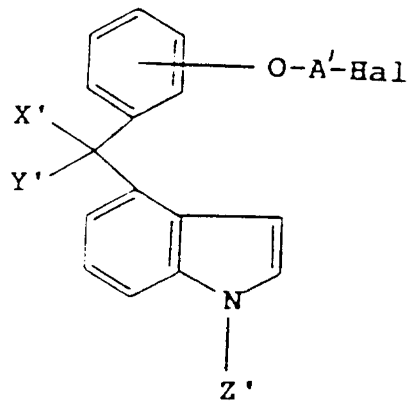

(XIII)

in which A', Z', X' and Y' have the meaning given above and the products of formula (XI) or (XIII) are subjected to an addition reaction by the amine of formula (XIV):

$NH-(R)(R_1)$ (XIV)

in which R and $R_1$ have the meaning indicated above, in order to obtain the products of formula (XV):

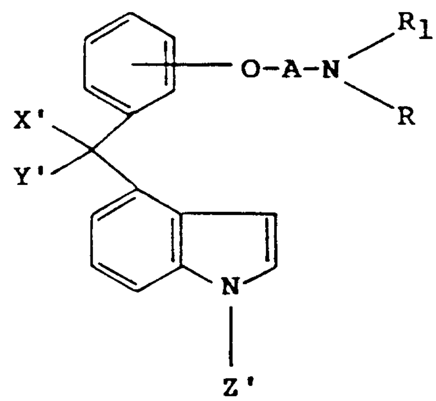

(XV)

which are subjected, if necessary and if desired, to one or more of the following reactions, in any order:
a) optional cleavage of Z' or of the protector groups carried by Z' if necessary to obtain Z,
b) reduction of the oxo function formed by X' and Y' into an alcohol function, followed if necessary and if desired by an alkylation of the hydroxy function formed by X or Y in order to obtain the products of formula (I) in which X or Y represents an alkoxy radical,
c) reduction of the double bond of the pyrrole nucleus of the indole radical in order to obtain the products of formula (I) in which a, b, c and d each represent a hydrogen atom,
d) halogenation in beta position of the nitrogen atom on the pyrrole nucleus of the indole radical followed by a hydrolysis in an acid medium in order to obtain the products of formula (I) in which a and b form an oxo function,
e) resolution of the racemic products by standard processes in order to obtain the optically active products and salification, if desired, of the products of formula (I) in order to obtain the corresponding salts.

**2.** Process according to claim 1 for preparing the products of formula (I) as defined in claim 1 corresponding to formula (I'):

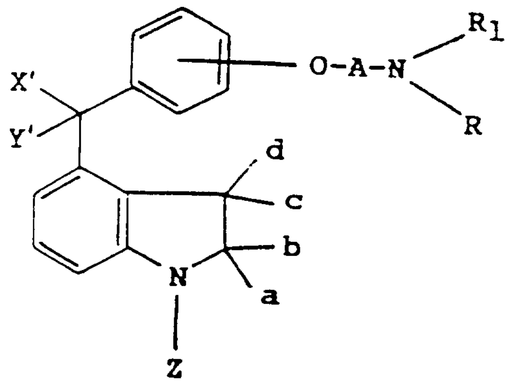

(I')

in which R, $R_1$, A, a, b, c, d and Z having the meaning indicated in claim 1 and X'and Y' are such that:

- either each represents a hydrogen atom,
- or one represents a hydrogen atom and the other represents a hydroxy radical,
- or X and Y together form an oxo radical,
  characterized in that the product of formula (VI) or of formula (VII) as defined in claim 1 is subjected to a condensation reaction on the hydroxy group,
- either by the product of formula (X):

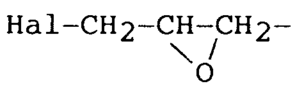

(X)

in order to obtain the product of formula (XI'):

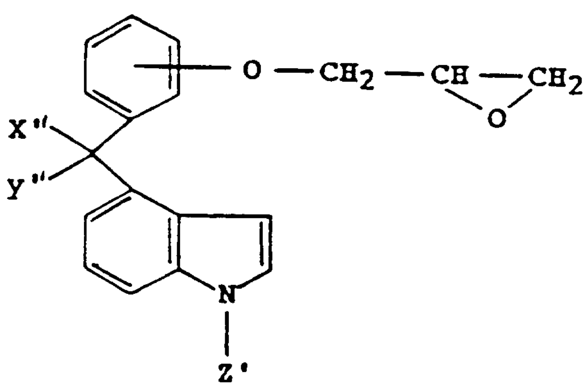

(XI')

in which X" and Y" represent a hydrogen atom or together form an oxo function,

- or by the product of formula (XII):

Hal-A'-Hal (XII)

in which A' represents $-(CH_2)_n$, in order to obtain the product of formula (XIII'):

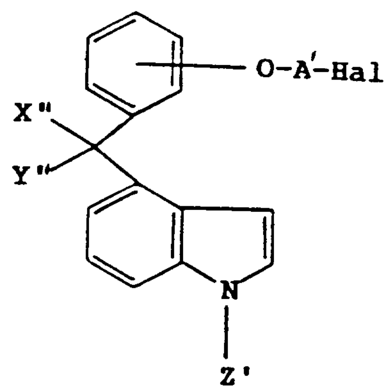

(XIII')

in which A', Z', X' and Y' have the meaning given previously, and the products of formula (XI) or (XIII) are subjected to an addition reaction by the amine of formula (XIV):

NH-(R)($R_1$) (XIV)

in which R and $R_1$ have the meaning indicated above, in order to obtain the products of formula (XV'):

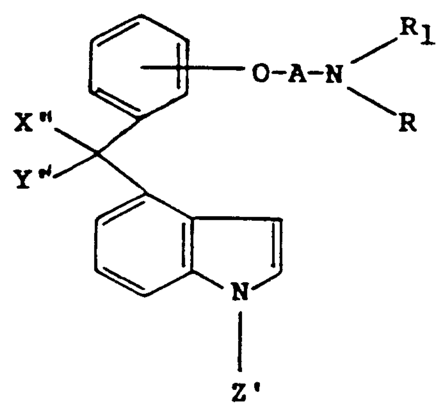

(XV')

which are subjected, if necessary and if desired, to one or more of the following reactions, in any order:
a) cleavage of Z' or of the protector groups carried by Z' in order to obtain Z,
b) reduction of the oxo function formed by X' and Y' into an alcohol function,
c) reduction of the double bond of the pyrrole nucleus of the indole radical in order to obtain the products of formula (I) in which a, b, c and d each represent a hydrogen atom,
d) halogenation in beta position of the nitrogen atom on the pyrrole nucleus of the indole radical followed by a hydrolysis in an acid medium in order to obtain the products of formula (I) in which a and b form an oxo function,
e) resolution of the racemic products by standard processes in order to obtain the optically active products and salification, if desired, of the products of formula (I) in order to obtain the corresponding salts.

**3.** Process according to claim 1 characterized in that a product of formula (II) as defined in claim 1 is used at the start in which Z' represents a protector group or an alkyl radical containing 1 to 4 carbon atoms and in that the corresponding product of formula (XV) is subjected, if appropriate, to a cleavage reaction of Z'.

**4.** Process according to claim 1 for the preparation of the products of formula (I) as defined in claim 1 in which R is a hydrogen atom, $R_1$ represents an alkyl radical containing 1 to 4 carbon atoms or a cycloalkyl radical containing 3 to 7 carbon atoms or R and $R_1$ form together with the nitrogen atom to which they are linked a heterocycle which can contain a second nitrogen atom optionally substituted by

an alkyl radical containing 1 to 5 carbon atoms and A is such that the integer n has the value 2 or 3 or A represents the group

$$-(CH_2)-\underset{\displaystyle OH}{\underset{|}{CH}}-(CH_2)-,$$

characterized in that the condensation reaction on the hydroxy group is carried out either by the product of formula (X) as defined in claim 1, or by the product of formula (XII) as defined in claim 1 in which A' represents a $(CH_2)_n$ group in which n has the value 2 or 3 and in that the corresponding product of formula (XI) or of formula (XIII) is subjected to the action of an amine of formula (XIV) in which R and $R_1$ have the values indicated above.

**5.** Process according to claim 1 characterized in that the products of formula (I) as defined in claim 1 are prepared, in which a, b, c, d are such that one of a or b forms with one of c or d a second carbon-carbon bond and the others represent a hydrogen atom and in which a and b together form an oxo radical and c and d each represent a hydrogen atom.

**6.** Process according to claim 1, characterized in that any one of the products of formula (I) as defined in claim 1 of which the names follow are prepared:
- [2-[3-[(1,1-dimethyl ethyl) amino] 2-hydroxy propoxy] phenyl] (1H-indol-4-yl) methanone,
- [2-[3-[(1,1-dimethyl ethyl) amino] propoxy] phenyl] (1H-indol-4-yl) methanone,
- 1-[(1,1-dimethyl ethyl) amino] 3-[2-[(1H-indol-4-yl) methyl] phenoxy] 2-propanol,
- (±) [2-[3-[(1,1-dimethylethyl) amino] 2-hydroxypropoxy] phenyl] (1-methyl 1H-indol-4-yl) methanone,
- 1,3-dihydro 4-[2-[3-[(1,1-dimethylethyl) amino] 2-hydroxypropoxy] benzoyl] 2H-indol-2-one,
- (±) 3-[(1,1-dimethylethyl) amino] 1-[[2-(1H-indol-4-yl) ethenyl] phenoxy] 2-propanol,
- N-(1,1-dimethylethyl) 3-[2-[1-(1H-indol-4-yl) ethenyl] phenoxy] propanamine,
- alpha [2-[3-[(1,1-dimethylethyl) amino] 2-hydroxypropoxy] phenyl 1H-indol-4-methanol,

as well as their addition salts with mineral or organic acids.

**7.** Process according to claim 1, characterized in that any one of the products of formula (I') as defined in claim 2 of which the names follow are prepared:
- [2-[3-[(1,1-dimethyl ethyl) amino] 2-hydroxy propoxy] phenyl] (1H-indol-4-yl) methanone,
- [2-[3-[(1,1-dimethyl ethyl) amino] propoxy] phenyl] (1H-indol-4-yl) methanone,
- 1-[(1,1-dimethyl ethyl) amino] 3-[2-[(1H-indol-4-yl) methyl] phenoxy] 2-propanol,
- alpha [2-[3-[(1,1-dimethylethyl) amino] 2-hydroxypropoxy] phenyl] 1H-indol-4-methanol,

as well as their addition salts with mineral or organic acids.

**8.** Preparation process for pharmaceutical compositions, characterized in that at least one of the products of formula (I) as defined in claim 1 or at least one of their addition salts with pharmaceutically acceptable mineral or organic acids is used as active ingredient in a form intended for this use.

**9.** Preparation process for pharmaceutical compositions, characterized in that at least one of the products of formula (I') as defined in claim 2 or at least one of their addition salts with pharmaceutically acceptable mineral or organic acids is used as active ingredient in a form intended for this use.

**10.** Preparation process for pharmaceutical compositions, characterized in that at least one of the products of formula (I) as defined in claim 8 or at least one of their addition salts with pharmaceutically acceptable mineral or organic acids is used as active ingredient in a form intended for this use.

**11.** The intermediate products corresponding to formulae (IV), (V), (VI), (VIII), (VIII'), (IX), (XI) and (XIII) as defined in claim 9.

**Patentansprüche**

**Patentansprüche für folgende Vertragsstaaten : AT, BE, CH, DE, DK, FR, GB, IT, LI, LU, NL, SE**

**1.** Die Produkte mit der allgemeinen Formel (I):

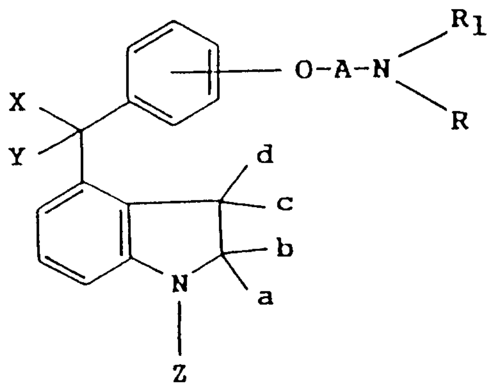

(I)

in der R und $R_1$, gleich oder verschieden, für folgendes stehen:

- entweder für ein Wasserstoffatom,
- oder für einen geradlinigen oder verzweigten Alkyl-, Alkenyl- oder Alkinylrest mit höchstens 8 Kohlenstoffatomen, der gegebenenfalls mit einem Hydroxylrest substituiert ist,
- oder für einen Cycloalkylrest mit 3 bis 7 Kohlenstoffatomen, für einen Cycloalkylalkylrest mit 4 bis 7 Kohlenstoffatomen oder für einen Arylalkylrest mit 7 bis 14 Kohlenstoffatomen, wobei dieser letzte Rest gegebenenfalls am Arylkern mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 5 Kohlenstoffatomen und den Resten Trifluormethyl, Methylthio, Nitro, Amino, Monoalkylamino oder Dialkylamino,

oder R und $R_1$ zusammen mit dem Stickstoffatome, an das sie gebunden sind, einen gesättigten oder ungesättigten Heterozyklus bilden, der ein zweites Heteroatom enthalten kann, ausgewählt aus dem Sauerstoff-, Schwefel- oder Stickstoffatom, wobei dieses zweite Stickstoffatom, das nicht an A gebunden ist, gegebenenfalls substituiert ist mit:

- entweder einem Alkylrest mit 1 bis 5 Kohlenstoffatomen,
- oder einem Phenylrest, der selbst gegebenenfalls mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 3 Kohlenstoffatomen und den Resten Trifluormethyl, Methylthio, Nitro, Amino, Monoalkylamino oder Dialkylamino,
- oder einem Naphthylrest,
- oder einem Arylalkyl- oder Diarylalkylrest mit 7 bis 14 Kohlenstoffatomen, wobei jeder dieser Reste gegebenenfalls am Arylkern mit 1, 2 oder 3 Resten substituiert ist, die aus der aus folgenden Resten bestehenden Gruppe ausgewählt sind: dem Hydroxylrest, den Halogenen, den linearen oder verzweigten Alkyl- oder Alkoxyresten mit höchstens 3 Kohlenstoffatomen und den Resten Trifluormethyl, Methylthio, Nitro, Amino, Monoalkylamino oder Dialkylamino,

A für folgendes steht:

- entweder für eine $(CH_2)_n$-Kette, in der n die Werte 2, 3, 4 oder 5 annehmen kann,
- oder für eine

$$-CH_2-\underset{OH}{CH}-CH_2-$$

Kette,

X und Y so beschaffen sind, daß

- entweder jedes für ein Wasserstoffatom steht,
- oder eines für ein Wasserstoffatom steht und das andere für einen Hydroxylrest, einen Alkoxyrest mit 1 bis 4 Kohlenstoffatomen oder einen Alkylrest mit 1 bis 4 Kohlenstoffatomen steht,

- oder X und Y zusammen einen Oxorest, einen Alkylidenrest mit 1 bis 4 Kohlenstoffatomen oder einen =N-$OR_5$-Rest bilden, in dem $R_5$ für ein Wasserstoffatom oder einen Alkylrest mit 1 bis 4 Kohlenstoffatomen steht,

die Substituenten a, b, c, d so beschaffen sind, daß
- entweder jedes für ein Wasserstoffatom steht,
- oder a und b zusammen eine Oxofunktion bilden und c und d jeweils für ein Wasserstoffatom stehen,
- oder eines von a oder b mit einem von c oder d eine Doppelbindung bildet und die anderen Substituenten jeweils für ein Wasserstoffatom stehen,

Z für folgende Reste steht: ein Wasserstoffatom, einen Alkyl-, Alkenyl- oder Alkinylrest, wobei jeder dieser geradlinigen oder verzweigten Reste höchstens 5 Kohlenstoffatome aufweist, einen Cycloalkylalkylrest mit 4 bis 7 Kohlenstoffatomen, einen Arylalkylrest mit 7 bis 14 Kohlenstoffatomen, wobei jeder dieser Reste gegebenenfalls mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 5 Kohlenstoffatomen,

oder Z für eine Aminoalkylgruppe mit der Formel -$R_2$-N-($R_3$)($R_4$) steht, in der $R_3$ und $R_4$, gleich oder verschieden, für ein Wasserstoffatom, einen geradlinigen oder verzweigten Alkylrest mit höchstens 5 Kohlenstoffatomen stehen,

und $R_2$ ein Alkylenrest mit 2 bis 5 Kohlenstoffatomen ist,

wobei besagte Verbindungen mit der Formel (I) in allen möglichen enantiomeren, racemischen oder diastereoisomeren Formen vorliegen können und in Form von Additionssalzen mit den Mineralsäuren oder organischen Säuren.

**2.** Die Produkte mit der Formel (I) wie in Anspruch 1 definiert, in der Z ein Wasserstoffatom oder ein Alkylrest mit 1 bis 4 Kohlenstoffatomen ist, wobei besagte Verbindungen mit der Formel (I) in Form von Additionssalzen mit den Mineralsäuren oder den organischen Säuren vorliegen können.

**3.** Die Produkte mit der Formel (I) wie in irgendeinem der Ansprüche 1 und 2 definiert, in der X und Y für ein Wasserstoffatom stehen oder in der sie zusammen einen Oxorest oder einen Alkylidenrest bilden, wobei besagte Verbindungen mit der Formel (I) in Form von Additionssalzen mit den Mineralsäuren oder den organischen Säuren vorliegen können.

**4.** Die Produkte mit der Formel (I) wie in irgendeinem der Ansprüche 1 bis 3 definiert, in der R ein Wasserstoffatom ist, $R_1$ für einen Alkylrest mit 1 bis 4 Kohlenstoffatomen oder für einen Cycloalkylrest mit 3 bis 7 Kohlenstoffatomen steht oder R und $R_1$ zusammen mit dem Stickstoffatom, an das sie gebunden sind, einen Heterozyklus bilden, der ein zweites Stickstoffatom enthalten kann, das gegebenenfalls mit einem Alkylrest mit 1 bis 5 Kohlenstoffatomen substituiert ist und A so gestaltet ist, daß die ganze Zahl n den Wert 2 oder 3 hat oder A für die Gruppe

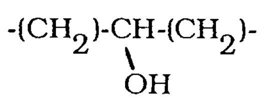

steht,

wobei besagte Verbindungen mit der Formel (I) in Form von Additionssalzen mit den Mineralsäuren oder den organischen Säuren vorliegen können.

**5.** Die Verbindungen mit der Formel (I) wie in den Ansprüchen 1 bis 4 definiert, in der a, b, c, d so beschaffen sind, daß einer von a oder b mit einem von c oder d eine zweite Kohlenstoff-Kohlenstoff-Bindung bilden und die anderen für ein Wasserstoffatom stehen, und diejenigen, in denen a und b zusammen einen Oxorest bilden und c und d jeweils für ein Wasserstoffatom stehen, wobei besagte Verbindungen mit der Formel (I) in Form von Additionssalzen mit den Mineralsäuren oder den organischen Säuren vorliegen können.

**6.** Die Derivate mit der Formel (I) wie in Anspruch 1 definiert mit folgenden Namen:
- das [2-[3-[(1,1-Dimethylethyl)-amino]-2-hydroxypropoxy]-phenyl]-(1H-indol-4-yl)-methanon,
- das [2-[3-[(1,1-Dimethylethyl)-amino]-propoxy]-phenyl]-(1H-indol-4-yl)-methanon,

- das 1-[(1,1-Dimethylethyl)-amino]-3-[2-[(1H-indol-4-yl)-methyl]-phenoxy]-2-propanol,
- das (±)-[2-[3-[(1,1-Dimethylethyl)-amino]-2-hydroxypropoxy]-phenyl]-(1-methyl-1H-indol-4-yl)-methanon,
- das 1,3-Dihydro-4-[2-[3-[(1,1-Dimethylethyl)-amino]-2-hydroxypropoxy]-benzoyl]-2H-indol-2-on,
- das (±)-3-[(1,1-Dimethylethyl)-amino]-1-[[2-(1H-indol-4-yl]-ethenyl]-phenoxy]-2-propanol,
- das N-(1,1-Dimethylethyl)-3-[2-[1-(1H-indol-4-yl)-ethenyl]-phenoxy]-propanamin,
- das α-[2-[3-[(1,1-Dimethylethyl)-amino]-2-hydroxypropoxy]-phenyl-1H-indol-4-methanol,

sowie ihre Additionssalze mit den Mineralsäuren oder den organischen Säuren.

**7.** Verfahren zur Herstellung der Produkten mit der Formel (I) wie in Anspruch 1 definiert, dadurch gekennzeichnet, daß man ein Produkt mit der Formel (II)

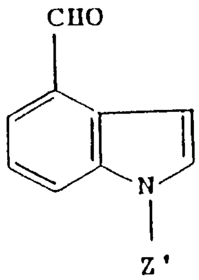

(II)

in der Z' für folgendes steht:
entweder für die oben für den Rest Z angegebenen Bedeutungen mit Ausnahme der Bedeutung Wasserstoff,
oder für besagte Bedeutungen, in denen die reaktiven Gruppen geschützt sind, oder für eine Schutzgruppe
einer Kondensationsreaktion mit einer Organometallverbindung mit folgender Formel (III) unterzieht:

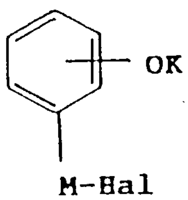

(III)

in der K für eine Schutzgruppe für den Hydroxylrest steht, M für ein Lithium- oder Magnesiumatom steht, wobei Hal für ein Halogenatom steht, wodurch man ein Produkte mit folgender Formel (IV) erhält:

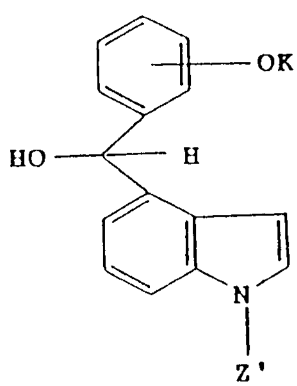

(IV)

das man einer Oxidationsreaktion unterzieht, wodurch man das Produkt mit folgender Formel (V) erhält:

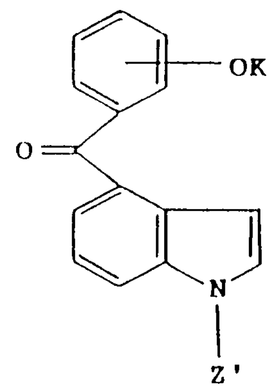

(V)

aus dem man selektiv die Hydroxylgruppe freisetzt, wodurch man das Produkt mit folgender Formel (VI) erhält:

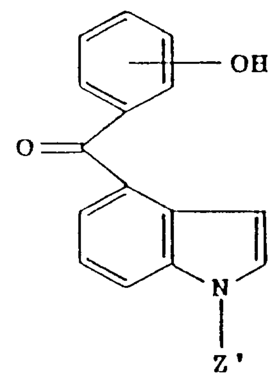

(VI)

und daß man, wenn erwünscht, das Produkt mit der Formel (VI) entweder einer Reduktionsreaktion der Oxofunktion unterzieht, wodurch man das Produkt mit folgender Formel (VII) erhält:

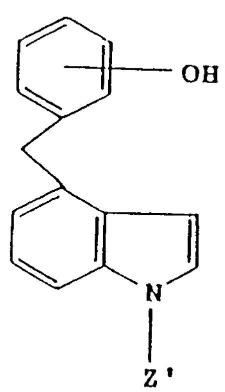

(VII)

oder aber daß man das Produkt mit der Formel (VI) der Einwirkung eines Organometallreagenzes unterzieht, dann der Einwirkung eines Dehydratisierungsmittels,wodurch man ein Produkt mit folgender Formel (VIII) erhält:

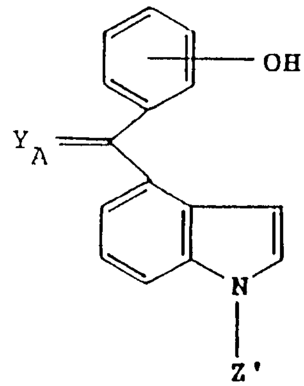

(VIII)

in der $Y_A$ für einen Alkylidenrest mit 1 bis 4 Kohlenstoffatomen steht, das Produkt mit der Formel (VIII), das man hydriert, wenn erwünscht, wodurch man ein Produkt mit folgender Formel (VIII') erhält:

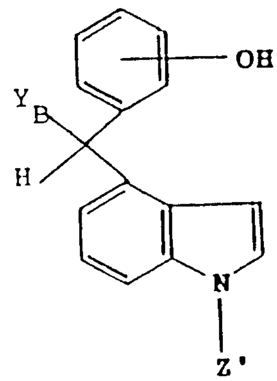

(VIII')

in der $Y_B$ für einen Alkylrest mit 1 bis 4 Kohlenstoffatomen steht,
oder aber daß man das Produkt mit der Formel (VI) in das entsprechende Oxim mit der Formel (IX) überführt:

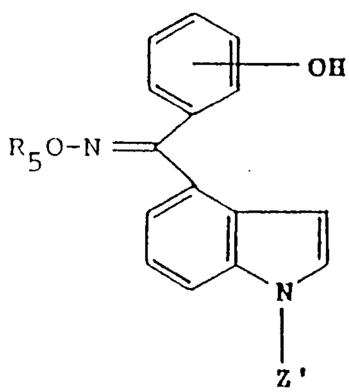

(IX)

in der $R_5$ die vorher angegebene Bedeutung hat,
Produkte mit der Formel (VI), (VII), (VIII), (VIII') oder (IX), die man einer Kondensationsreaktion an der Hydroxylgruppe unterzieht, und zwar

- entweder mit dem Produkt mit der Formel (X):

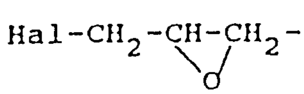

(X)

wodurch man das Produkt mit der Formel (XI) erhält:

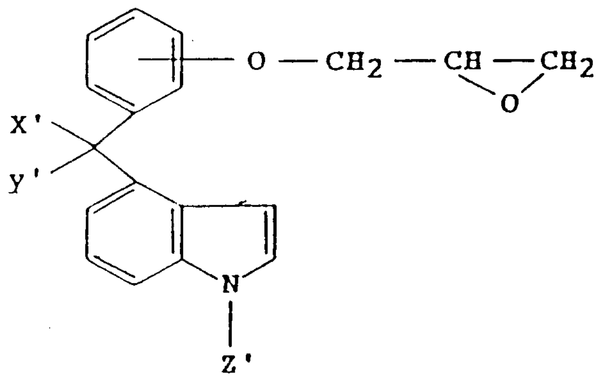

(XI)

in der X' und Y' für ein Wasserstoffatom stehen oder in der sie zusammen eine Oxofunktion, einen Alkylidenrest oder einen $=N\text{-}OR_5$-Rest bilden oder in der eines für ein Wasserstoffatom steht und das andere für einen Alkylrest mit 1 bis 4 Kohlenstoffatomen steht,

- oder mit dem Produkt mit der Formel (XII):

Hal-A'-Hal (XII)

in der A' für $-(CH_2)_n$ steht, wo n die in Anspruch 1 angegebene Bedeutung hat, wodurch man das Produkt mit der Formel (XIII) erhält:

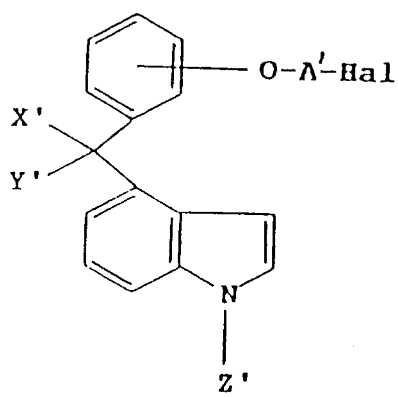

(XIII)

in der A', Z', X' und Y' die oben angegebene Bedeutung haben, und daß man die Produkte mit der Formel (XI) oder (XIII) einer Additionsreaktion mit einem Amin mit der Formel (XIV) unterzieht:

$NH\text{-}(R)(R_1)$ (XIV)

in der R und $R_1$ die oben angegebene Bedeutung haben, wodurch man die Produkte mit folgender Formel (XV) erhält:

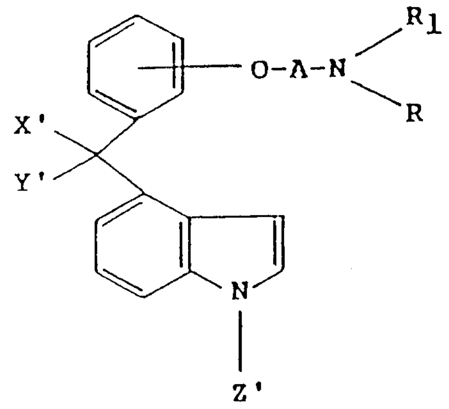

(XV)

die man, wenn nötig und wenn erwünscht, in beliebiger Reihenfolge einer oder mehrerer der folgenden Reaktionen unterzieht:

a) etwaige Abtrennung von Z' oder der Schutzgruppen, die Z', wenn nötig trägt, um Z zu erhalten,

b) Reduktion der Oxofunktion, die von X' und Y' gebildet wird, zu einer Alkoholfunktion, gefolgt von, wenn nötig und wenn erwünscht, einer Alkylierung der Hydroxylfunktion, die von X oder Y gebildet wird, wodurch man die Produkte mit der Formel (I) erhält, in der X oder Y für einen Alkoxyrest stehen,

c) Reduktion der Doppelbindung des Pyrrolkerns des Indolrests, um Produkte mit der Formel (I) zu erhalten, in der a, b, c und d jeweils für ein Wasserstoffatom stehen,

d) Halogenierung in $\beta$-Stellung des Stickstoffatoms am Pyrrolkern des Indolrests, gefolgt von einer Hydrolyse im sauren Milieu, um die Produkte mit der Formel (I) zu erhalten, in der a und b eine Oxofunktion bilden,

e) Auftrennung der racemischen Produkte mittels klassischer Verfahren, um die optisch aktiven Produkte zu erhalten, und, wenn erwünscht, Salzbildung der Produkte mit der Formel (I), um die entsprechenden Salze zu erhalten.

**8.** Die Produkte mit der Formel (I) wie in einem beliebigen der Ansprüche 1 bis 5 definiert, sowie ihre Additionssalze mit den pharmazeutisch verwendbaren Säuren als Arzneistoffe.

**9.** Die in Anspruch 6 definierten Produkte sowie ihre Additionssalze mit den pharmazeutisch verwendbaren Salzen als Arzneistoffe.

**10.** Die pharmazeutischen Zusammensetzungen, die als Wirkstoff wenigstens einen der Arzneistoffe wie in Anspruch 8 oder 9 definiert enthalten.

**11.** Die Zwischenprodukte, die den Formein (IV), (V), (VI), (VIII), (VIII'), (IX), (XI) und (XIII) wie in Anspruch 9 definiert entsprechen.

**Patentansprüche für folgenden Vertragsstaat : ES**

1. Verfahren zur Herstellung der Produkte mit der allgemeinen Formel (I):

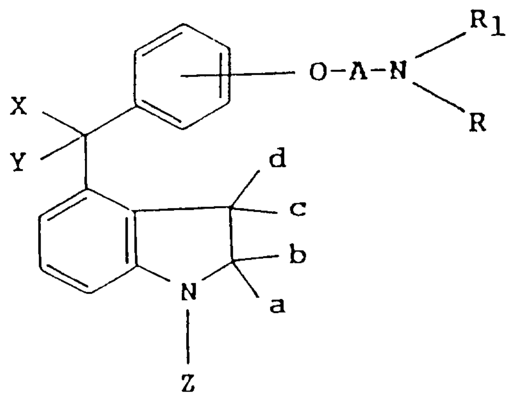

(I)

in der R und $R_1$, gleich oder verschieden, für folgendes stehen:
- entweder für ein Wasserstoffatom,
- oder für einen geradlinigen oder verzweigten Alkyl-, Alkenyl- oder Alkinylrest mit höchstens 8 Kohlenstoffatomen, der gegebenenfalls mit einem Hydroxylrest substituiert ist,
- oder für einen Cycloalkylrest mit 3 bis 7 Kohlenstoffatomen, einen Cycloalkylalkylrest mit 4 bis 7 Kohlenstoffatomen oder einen Arylalkylrest mit 7 bis 14 Kohlenstoffatomen, wobei dieser letzte Rest gegebenenfalls am Arylkern mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 5 Kohlenstoffatomen und den Resten Trifluormethyl, Methylthio, Nitro, Amino, Monoalkylamino oder Dialkylamino,

  oder R und $R_1$ zusammen mit dem Stickstoffatom, an das sie gebunden sind, einen gesättigten oder ungesättigten Heterozyklus bilden, der ein zweites Heteroatom enthalten kann, ausgewählt aus dem Sauerstoff-, Schwefel- oder Stickstoffatom, wobei dieses zweite Stickstoffatom, das nicht an A gebunden ist, gegebenenfalls substituiert ist mit:
- entweder einem Alkylrest mit 1 bis 5 Kohlenstoffatomen,
- oder einem Phenylrest, der selbst gegebenenfalls mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 3 Kohlenstoffatomen und den Resten Trifluormethyl, Methylthio, Nitro, Amino, Monoalkylamino oder Dialkylamino,
- oder einem Naphthylrest,
- oder einem Arylalkyl- oder Diarylalkylrest mit 7 bis 14 Kohlenstoffatomen, wobei jeder dieser Reste gegebenenfalls am Arylkern mit 1, 2 oder 3 Resten substituiert ist, die aus der aus folgenden Resten bestehenden Gruppe ausgewählt sind: dem Hydroxylrest, den Halogenen, den linearen oder verzweigten Alkyl- oder Alkoxyresten mit höchstens 3 Kohlenstoffatomen und den Resten Trifluormethyl, Methylthio, Nitro, Amino, Monoalkylamino oder Dialkylamino,

  A für folgendes steht:
- entweder für eine $(CH_2)_n$-Kette, in der n die Werte 2, 3, 4 oder 5 annehmen kann,
- oder für eine

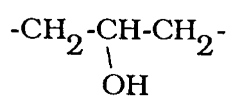

  Kette,

  X und Y so beschaffen sind, daß
- entweder jedes für ein Wasserstoffatom steht,
- oder eines für ein Wasserstoffatom steht und das andere für einen Hydroxylrest, einen Alkoxyrest mit 1 bis 4 Kohlenstoffatomen oder einen Alkylrest mit 1 bis 4 Kohlenstoffatomen steht,

- oder X und Y zusammen einen Oxorest, einen Alkylidenrest mit 1 bis 4 Kohlenstoffatomen oder einen $=N-OR_5$-Rest bilden, in dem $R_5$ für ein Wasserstoffatom oder einen Alkylrest mit 1 bis 4 Kohlenstoffatomen steht,

die Substituenten a, b, c, d so beschaffen sind, daß
- entweder jedes für ein Wasserstoffatom steht,
- oder a und b zusammen eine Oxofunktion bilden und c und d jeweils für ein Wasserstoffatom stehen,
- oder eines von a oder b mit einem von c oder d eine Doppelbindung bildet und die anderen Substituenten jeweils für ein Wasserstoffatom stehen,

Z für folgende Reste steht: ein Wasserstoffatom, einen Alkyl-, Alkenyl- oder Alkinylrest, wobei jeder dieser geradlinigen oder verzweigten Reste höchstens 5 Kohlenstoffatome aufweist, einen Cycloalkylalkylrest mit 4 bis 7 Kohlenstoffatomen, einen Arylalkylrest mit 7 bis 14 Kohlenstoffatomen, wobei jeder dieser Reste gegebenenfalls mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 5 Kohlenstoffatomen,
oder Z für eine Aminoalkylgruppe mit der Formel $-R_2-N-(R_3)(R_4)$ steht, in der $R_3$ und $R_4$, gleich oder verschieden, für ein Wasserstoffatom, einen geradlinigen oder verzweigten Alkylrest mit höchstens 5 Kohlenstoffatomen stehen und $R_2$ ein Alkylenrest mit 2 bis 5 Kohlenstoffatomen ist,
wobei besagte Verbindungen mit der Formel (I) in allen möglichen enantiomeren, racemischen oder diastereoisomeren Formen vorliegen können und in Form von Additionssalzen mit den Mineralsäuren oder organischen Säuren, dadurch gekennzeichnet, daß man ein Produkt mit der Formel (II):

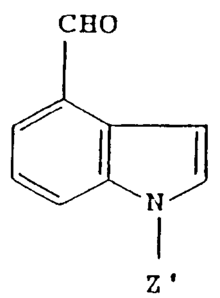

(II)

in der Z' für folgendes steht:
entweder für die oben für den Rest Z angegebenen Bedeutungen mit Ausnahme der Bedeutung Wasserstoff,
oder für besagte Bedeutungen, in denen die reaktiven Gruppen geschützt sind, oder für eine Schutzgruppe
einer Kondensationsreaktion mit einer Organometallverbindung mit folgender Formel (III) unterzogen wird:

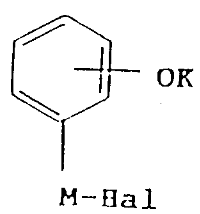

(III)

in der K für eine Schutzgruppe für den Hydroxylrest steht, M für ein Lithium- oder Magnesiumatom steht, wobei Hal für ein Halogenatom steht, wodurch man ein Produkt mit der Formel (IV) erhält:

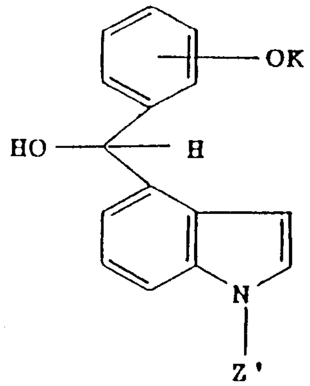

(IV)

das man einer Oxidationsreaktion unterzieht, wodurch man das Produkt mit der Formel (V) erhält:

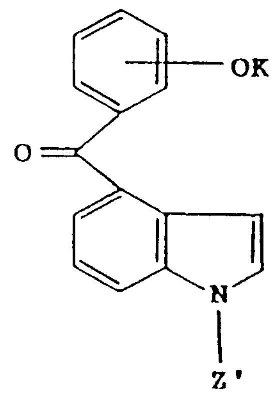

(V)

woraus man selektiv die Hydroxylgruppe freisetzt, wodurch man das Produkt mit der Formel (VI) erhält:

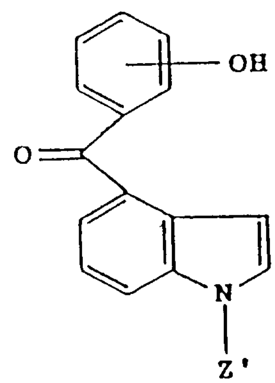

(VI)

und daß man, wenn erwünscht, das Produkt mit der Formel (VI) entweder einer Reduktionsreaktion der Oxofunktion unterzieht, wodurch man das Produkt mit der Formel (VII) erhält:

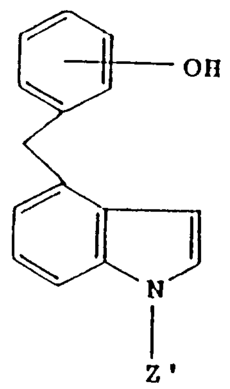

(VII)

oder aber daß man das Produkt mit der Formel (VI) der Einwirkung eines Organometallreagenzes unterzieht, dann der Einwirkung eines Dehydratisierungsmittels,wodurch man ein Produkt mit folgender Formel (VIII) erhält:

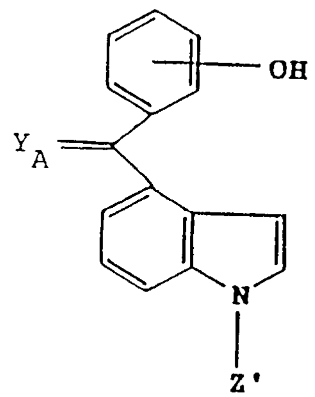

(VIII)

in der $Y_A$ für einen Alkylidenrest mit 1 bis 4 Kohlenstoffatomen steht, das Produkt mit der Formel (VIII), das man hydriert, wenn erwünscht, wodurch man ein Produkt mit folgender Formel (VIII') erhält:

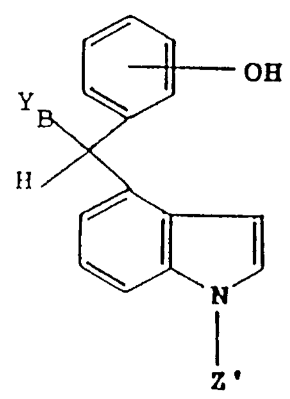

(VIII')

in der $Y_B$ für einen Alkylrest mit 1 bis 4 Kohlenstoffatomen steht,
oder aber daß man das Produkt mit der Formel (VI) in das entsprechende Oxim mit der Formel (IX) überführt:

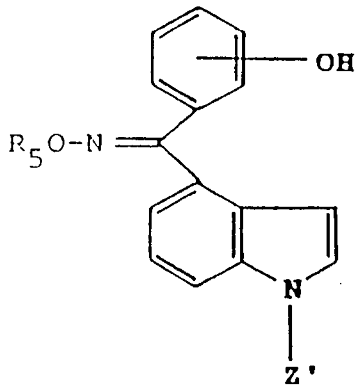

(IX)

in der $R_5$ die vorher angegebene Bedeutung hat,
Produkte mit der Formel (VI), (VII), (VIII), (VIII') oder (IX), die man einer Kondensationsreaktion an der Hydroxylgruppe unterzieht, und zwar

- entweder mit dem Produkt mit der Formel (X):

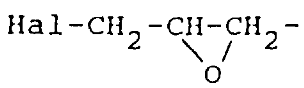

(X)

wodurch man das Produkt mit der Formel (XI) erhält:

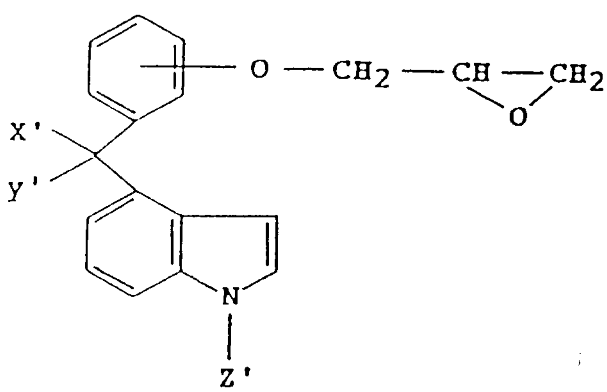

(XI)

in der X' und Y' für ein Wasserstoffatom stehen oder in der sie zusammen eine Oxofunktion, einen Alkylidenrest oder einen $=N\text{-}OR_5$-Rest bilden, oder in der eines für ein Wasserstoffatom steht und das andere für einen Alkylrest mit 1 bis 4 Kohlenstoffatomen steht.

- oder mit dem Produkt mit der Formel (XII):

Hal-A'-Hal (XII)

in der A' für $-(CH_2)_n$ steht, wo n die vorher angegebene Bedeutung hat, wodurch man das Produkt mit der Formel (XIII) erhält:

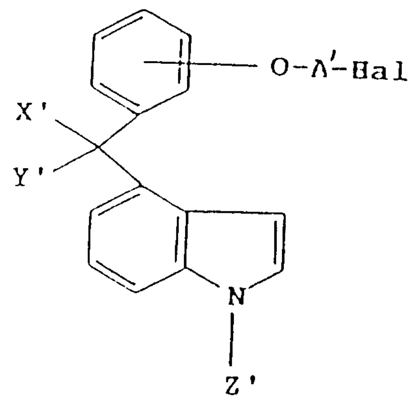

(XIII)

in der A', Z', X' und Y' die oben angegebene Bedeutung haben, und daß man die Produkte mit der Formel (XI) oder (XIII) einer Additionsreaktion mit einem Amin mit der Formel (XIV) unterzieht:

$NH$-(R)($R_1$) (XIV)

in der R und $R_1$ die oben angegebene Bedeutung haben, wodurch man die Produkte mit folgender Formel (XV) erhält:

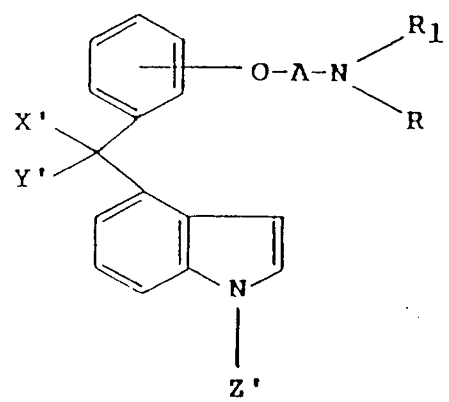

(XV)

die man, wenn nötig und wenn erwünscht, in beliebiger Reihenfolge einer oder mehrerer der folgenden Reaktionen unterzieht:
a) etwaige Abtrennung voll Z' oder der Schutzgruppen, die Z' ,wenn nötig, trägt, um Z zu erhalten,
b) Reduktion der Oxofunktion, die von X' und Y' gebildet wird, zu einer Alkoholfunktion, gefolgt von, wenn nötig und wenn erwünscht, einer Alkylierung der Hydroxylfunktion, die von X oder Y gebildet wird, wodurch man die Produkte mit der Formel (I) erhält, in der X oder Y für einen Alkoxyrest stehen,
c) Reduktion der Doppelbindung des Pyrrolkerns des Indolrests, um Produkte mit der Formel (I) zu erhalten, in der a, b, c und d jeweils für ein Wasserstoffatom stehen,
d) Halogenierung in $\beta$-Stellung des Stickstoffatoms am Pyrrolkern des Indolrests, gefolgt von einer Hydrolyse im sauren Milieu, um die Produkte mit der Formel (I) zu erhalten, in der a und b eine Oxofunktion bilden,
e) Auftrennung der racemischen Produkte mittels klassischer Verfahren, um die optisch aktiven Produkte zu erhalten, und, wenn erwünscht, Salzbildung der Produkte mit der Formel (I), um die entsprechenden Salze zu erhalten.

**2.** Verfahren nach Anspruch 1 zur Herstellung der Produkte mit der Formel (I) wie in Anspruch 1 definiert, die der Formel (I') entsprechen:

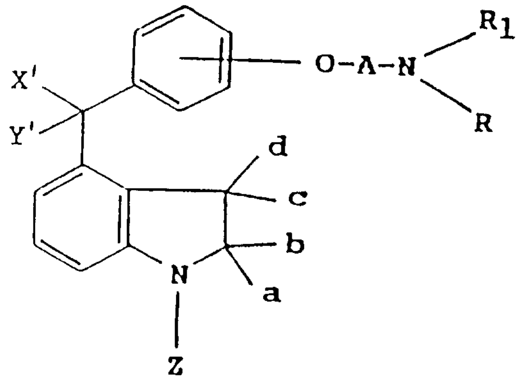

(I')

in der R, $R_1$, A, a, b, c, d und Z die in Anspruch 1 angegebene Bedeutung haben und X' und Y' so beschaffen sind, daß:

- entweder jedes für ein Wasserstoffatom steht,
- oder eines für ein Wasserstoffatom steht und das andere für einen Hydroxylrest steht,
- oder X und Y zusammen einen Oxorest bilden, dadurch gekennzeichnet, daß man das Produkt mit der Formel (VI) oder der Formel (VII) wie in Anspruch 1 definiert einer Kondensationsreaktion an der Hydroxylgruppe unterwirft, und zwar
- entweder mit dem Produkt mit der Formel (X):

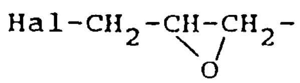

(X)

wodurch man das Produkt mit der Formel (XI') erhält:

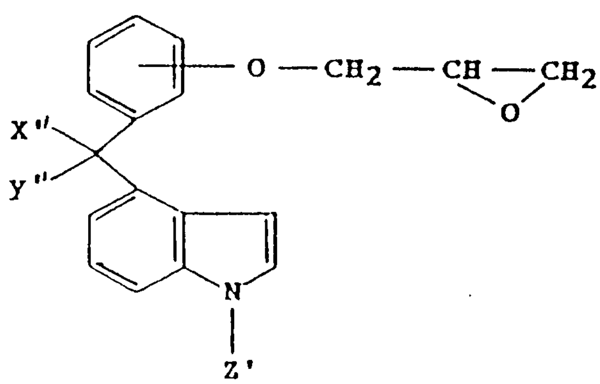

(XI')

in der X'' und Y'' für ein Wasserstoffatom stehen oder zusammen einen Oxorest bilden,

- oder mit dem Produkt mit der Formel (XII):

Hal-A'-Hal (XII)

in der A' für $-(CH_2)_n$ steht, wodurch man das Produkt mit der Formel (XIII') erhält:

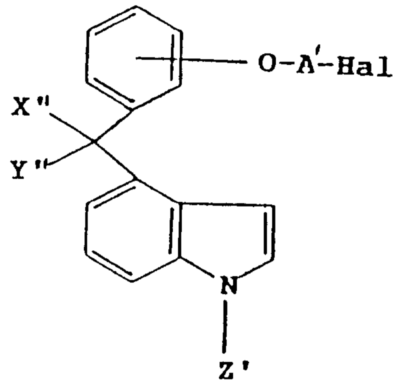

(XIII')

in der A', Z', X' und Y' die vorher angegebene Bedeutung haben, und daß man die Produkte mit der Formel (XI) oder (XIII) einer Additionsreaktion mit dem Amin mit folgender Formel (XIV) unterwirft:

$NH-(R)(R_1)$ (XIV)

in der R und $R_1$ die oben angegebene Bedeutung haben, wodurch man die Produkte mit der Formel (XV') erhält:

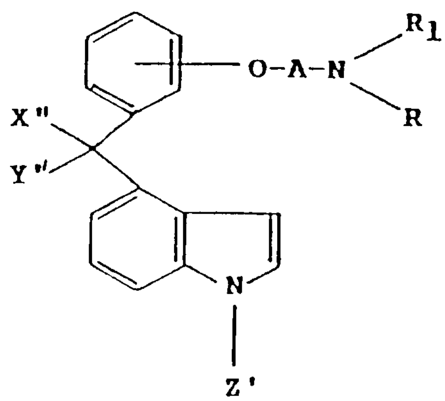

(XV')

die man, wenn nötig und wenn erwünscht, in beliebiger Reihenfolge einer oder mehreren der folgenden Reaktionen unterwirft:
a) Abtrennung von Z' oder der Schutzgruppen, die Z' trägt, um Z zu erhalten,
b) Reduktion der Oxofunktion, die von X' und Y' gebildet wird, zu einer Alkoholfunktion,
c) Reduktion der Doppelbindung des Pyrrolkerns des Indolrests, um Produkte mit der Formel (I) zu erhalten, in der a, b, c und d jeweils für ein Wasserstoffatom stehen,
d) Halogenierung in β-Stellung des Stickstoffatoms am Pyrrolkern des Indolrests, gefolgt von einer Hydrolyse im sauren Milieu, um die Produkte mit der Formel (I) zu erhalten, in der a und b eine Oxofunktion bilden,
e) Auftrennung der racemischen Produkte mittels klassischer Verfahren, um die optisch aktiven Produkte zu erhalten, und, wenn erwünscht, Salzbildung der Produkte mit der Formel (I), um die entsprechenden Salze zu erhalten.

3. Verfahren nach Anspruch 1, dadurch gekennzeichnet, daß man anfangs ein Produkt mit der Formel (II) wie in Anspruch 1 definiert verwendet, in der Z' für einer Schutzgruppe oder einen Alkylrest mit 1 bis 4 Kohlenstoffatomen steht und dadurch, daß man das entsprechende Produkt mit der Formel (XV) gegebenenfalls einer Abtrennreaktion von Z' unterwirft.

4. Verfahren nach Anspruch 1 zur Herstellung der Produkte mit der Formel (I) wie in Anspruch 1 definiert, in der R ein Wasserstoffatom ist, $R_1$ für einen Alkylrest mit 1 bis 4 Kohlenstoffatomen oder für einen

Cycloalkylrest mit 3 bis 7 Kohlenstoffatomen steht oder R und $R_1$ zusammen mit dem Stickstoffatom, an das sie gebunden sind, einen Heterozyklus bilden, der ein zweites Stickstoffatom enthalten kann, das gegebenenfalls mit einem Alkylrest mit 1 bis 5 Kohlenstoffatomen substituiert ist, und A so beschaffen ist, daß die ganze Zahl n den Wert 2 oder 3 hat oder A für die Gruppe

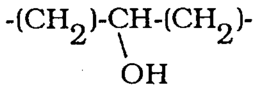

steht,
dadurch gekennzeichnet, daß man die Kondensationsreaktion an der Hydroxylgruppe entweder mit dem Produkt mit der Formel (X) wie in Anspruch 1 definiert durchführt oder mit dem Produkt mit der Formel (XII) wie in Anspruch 1 definiert, in der A' für eine $(CH_2)_n$-Gruppierung steht, in der n den Wert 2 oder 3 hat, und dadurch daß man das Produkt mit der Formel (XI) oder dem entsprechenden mit der Formel (XIII) der Einwirkung eines Amins mit der Formel (XIV) unterwirft, in der R und $R_1$ die oben angegebenen Bedeutungen haben.

**Patentansprüche für folgenden Vertragsstaat : GR**

**1.** Verfahren zur Herstellung der Produkte mit der allgemeinen Formel (I):

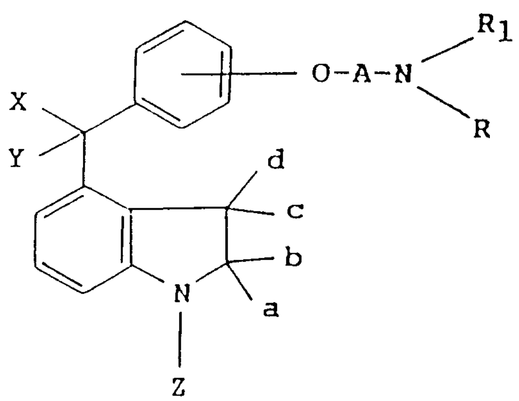

(I)

in der R und $R_1$, gleich oder verschieden, für folgendes stehen:

- entweder für ein Wasserstoffatom,
- oder für einen geradlinigen oder verzweigten Alkyl-, Alkenyl- oder Alkinylrest mit höchstens 8 Kohlenstoffatomen, der gegebenenfalls mit einem Hydroxylrest substituiert ist,
- oder für einen Cycloalkylrest mit 3 bis 7 Kohlenstoffatomen, einen Cycloalkylalkylrest mit 4 bis 7 Kohlenstoffatomen oder einen Arylalkylrest mit 7 bis 14 Kohlenstoffatomen, wobei dieser letzte Rest gegebenenfalls am Arylkern mit 1, 2 oder 3 Resten substituiert ist, die aus der aus folgenden Resten bestehenden Gruppe ausgewählt sind: dem Hydroxylrest, den Halogenen, den geradlinigen, oder verzweigten Alkyl- oder Alkoxyresten mit höchstens 5 Kohlenstoffatomen und den Resten Trifluormethyl, Methylthio, Nitro, Amino, Monoalkylamino oder Dialkylamino,

  oder R und $R_1$ zusammen mit dem Stickstoffatom, an das sie gebunden sind, einen gesättigten oder ungesättigten Heterozyklus bilden, der ein zweites Heteroatom enthalten kann, ausgewählt aus dem Sauerstoff-, Schwefel- oder Stickstoffatom, wobei dieses zweite Stickstoffatom, das nicht an A gebunden ist, gegebenenfalls substituiert ist mit:
- entweder einem Alkylrest mit 1 bis 5 Kohlenstoffatomen,
- oder einem Phenylrest, der selbst gegebenenfalls mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 3 Kohlenstoffatomen und den Resten Trifluormethyl, Methylthio, Nitro, Amino, Monoalkylamino oder Dialkylamino,
- oder einem Naphthylrest,

- oder einem Arylalkyl- oder Diarylalkylrest mit 7 bis 14 Kohlenstoffatomen, wobei jeder dieser Reste gegebenenfalls am Arylkern mit 1, 2 oder 3 Resten substituiert ist, die aus der aus folgenden Resten bestehenden Gruppe ausgewählt sind: dem Hydroxylrest, den Halogenen, den linearen oder verzweigten Alkyl- oder Alkoxyresten mit höchstens 3 Kohlenstoffatomen und den Resten Trifluormethyl, Methylthio, Nitro, Amino, Monoalkylamino oder Dialkylamino,

A für folgendes steht:
- entweder für eine $(CH_2)_n$-Kette, in der n die Werte 2, 3, 4 oder 5 annehmen kann,
- oder für eine

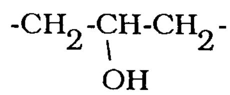

Kette,

X und Y so beschaffen sind, daß
- entweder jedes für ein Wasserstoffatom steht,
- oder eines für ein Wasserstoffatom steht und das andere für einen Hydroxylrest, einen Alkoxyrest mit 1 bis 4 Kohlenstoffatomen oder einen Alkylrest mit 1 bis 4 Kohlenstoffatomen steht,
- oder X und Y zusammen einen Oxorest, einen Alkylidenrest mit 1 bis 4 Kohlenstoffatomen oder einen $=N-OR_5$-Rest bilden, in dem $R_5$ für ein Wasserstoffatom oder einen Alkylrest mit 1 bis 4 Kohlenstoffatomen steht,

die Substituenten a, b, c, d so beschaffen sind, daß
- entweder jedes für ein Wasserstoffatom steht,
- oder a und b zusammen eine Oxofunktion bilden und c und d jeweils für ein Wasserstoffatom stehen,
- oder eines von a oder b mit einem von c oder d eine Doppelbindung bildet und die anderen Substituenten jeweils für ein Wasserstoffatom stehen,

Z für folgende Reste steht: ein Wasserstoffatom, einen Alkyl-, Alkenyl- oder Alkinylrest, wobei jeder dieser geradlinigen oder verzweigten Reste höchstens 5 Kohlenstoffatome aufweist, einen Cycloalkylalkylrest mit 4 bis 7 Kohlenstoffatomen, einen Arylalkylrest mit 7 bis 14 Kohlenstoffatomen, wobei jeder dieser Reste gegebenenfalls mit 1, 2 oder 3 Resten substituiert ist, die aus der aus folgenden Resten bestehenden Gruppe ausgewählt sind: dem Hydroxylrest, den Halogenen, den geradlinigen oder verzweigten Alkyl- oder Alkoxyresten mit höchstens 5 Kohlenstoffatomen,

oder Z für eine Aminoalkylgruppe mit der Formel $-R_2-N-(R_3)(R_4)$ steht, in der $R_3$ und $R_4$, gleich oder verschieden, für ein Wasserstoffatom, einen geradlinigen oder verzweigten Alkylrest mit höchstens 5 Kohlenstoffatomen stehen

und $R_2$ ein Alkylenrest mit 2 bis 5 Kohlenstoffatomen ist,

wobei besagte Verbindungen mit der Formel (I) in allen möglichen enantiomeren, racemischen oder diastereoisomeren Formen vorliegen können und in Form von Additionssalzen mit den Mineralsäuren oder organischen Säuren, dadurch gekennzeichnet, daß man ein Produkt mit der Formel (II):

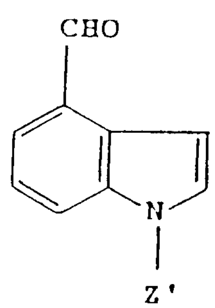

(II)

in der Z' für folgendes steht:

entweder für die oben für den Rest Z angegebenen Bedeutungen mit Ausnahme der Bedeutung Wasserstoff,

oder für besagte Bedeutungen, in denen die reaktiven Gruppen geschützt sind, oder für eine

Schutzgruppe
einer Kondensationsreaktion mit einer Organometallverbindung mit folgender Formel (III) unterzogen wird:

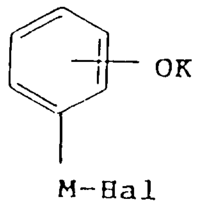

(III)

in der K für eine Schutzgruppe für den Hydroxylrest steht, M für ein Lithium- oder Magnesiumatom steht, wobei Hal für ein Halogenatom steht, wodurch man ein Produkt mit der Formel (IV) erhält:

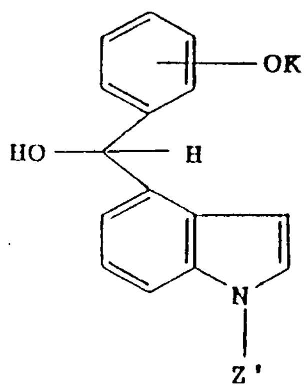

(IV)

das man einer Oxidationsreaktion unterzieht, wodurch man das Produkt mit der Formel (V) erhält:

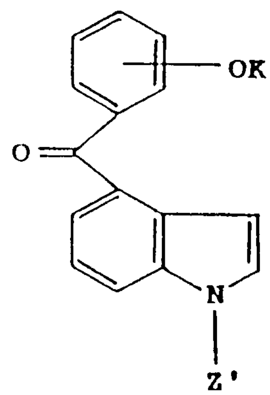

(V)

woraus man selektiv die Hydroxylgruppe freisetzt, wodurch man das Produkt mit der Formel (VI) erhält:

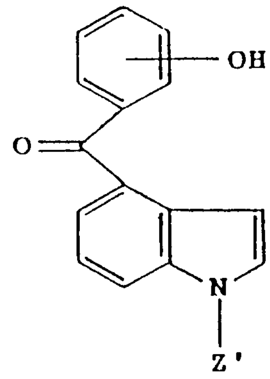

(VI)

und daß man, wenn erwünscht, das Produkt mit der Formel (VI) entweder einer Reduktionsreaktion der Oxofunktion unterzieht, wodurch man das Produkt mit der Formel (VII) erhält:

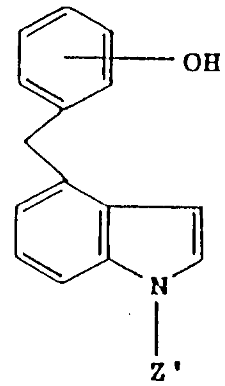

(VII)

oder aber daß man das Produkt mit der Formel (VI) der Einwirkung eines Organometallreagenzes unterzieht, dann der Einwirkung eines Dehydratisierungsmittels,wodurch man ein Produkt mit folgender Formel (XII) erhält:

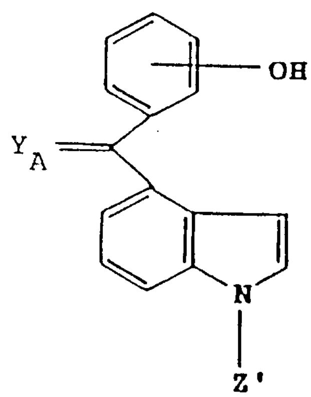

(VIII)

in der $Y_A$ für einen Alkylidenrest mit 1 bis 4 Kohlenstoffatomen steht, das Produkt mit der Formel (VIII), das man hydriert, wenn erwünscht, wodurch man ein Produkt mit folgender Formel (VIII') erhält:

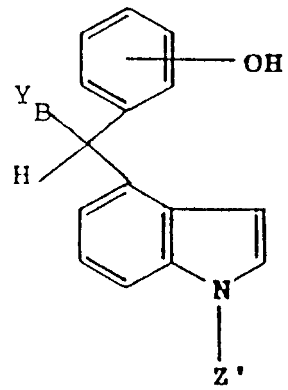

(VIII')

in der $Y_B$ für einen Alkylrest mit 1 bis 4 Kohlenstoffatomen steht,
oder aber daß man das Produkt mit der Formel (VI) in das entsprechende Oxim mit der Formel (IX) überführt:

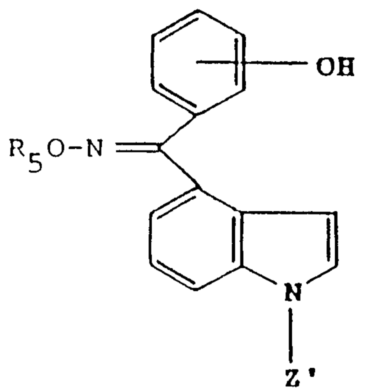

(IX)

in der $R_5$ die vorher angegebene Bedeutung hat,
Produkte mit der Formel (VI), (VII), (VIII), (VIII') oder (IX), die man einer Kondensationsreaktion an der Hydroxylgruppe unterzieht, und zwar

- entweder mit dem Produkt mit der Formel (X):

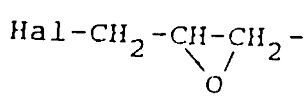

(X)

wodurch man das Produkt mit der Formel (XI) erhält:

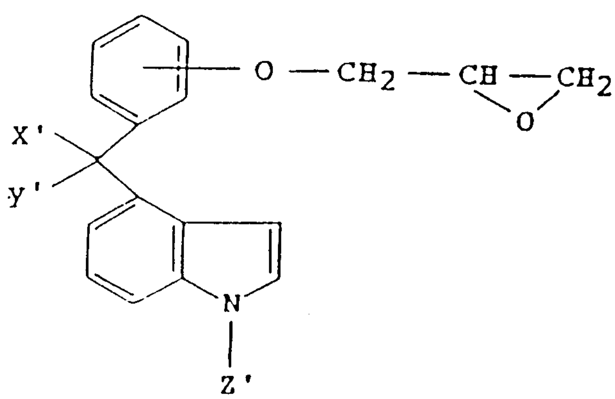

(XI)

in der X' und Y' für ein Wasserstoffatom stehen oder in der sie zusammen eine Oxofunktion, einen Alkylidenrest oder einen =N-$OR_5$-Rest bilden, oder in der eines für ein Wasserstoffatom steht und das andere für einen Alkylrest mit 1 bis 4 Kohlenstoffatomen steht,

- oder mit dem Produkt mit der Formel (XII):

Hal-A'-Hal (XII)

in der A' für $-(CH_2)_n$ steht, wo n die vorher angegebene Bedeutung hat, wodurch man das Produkt mit der Formel (XIII) erhält:

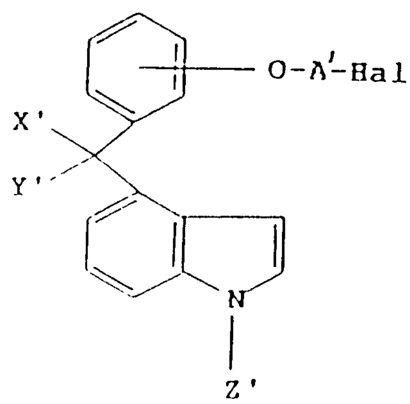

(XIII)

in der A', Z', X' und Y' die oben angegebene Bedeutung haben, und daß man die Produkte mit der Formel (XI) oder (XIII) einer Additionsreaktion mit einem Amin mit der Formel (XIV) unterzieht:

NH-(R)($R_1$) (XIV)

in der R und $R_1$ die oben angegebene Bedeutung haben, wodurch man die Produkte mit folgender Formel (XV) erhält:

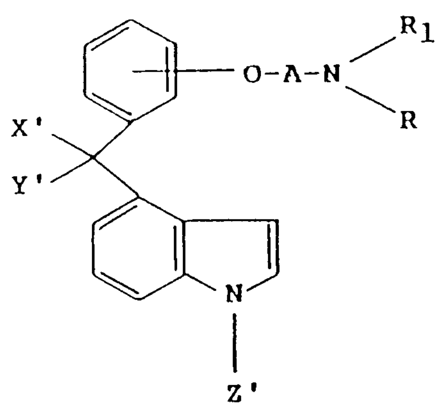

(XV)

die man, wenn nötig und wenn erwünscht, in beliebiger Reihenfolge einer oder mehrerer der folgenden Reaktionen unterzieht:

a) etwaige Abtrennung von Z' oder der Schutzgruppen, die Z',wenn nötig, trägt, um Z zu erhalten,

b) Reduktion der Oxofunktion, die von X' und Y' gebildet wird, zu einer Alkoholfunktion, gefolgt von, wenn nötig und wenn erwünscht, einer Alkylierung der Hydroxylfunktion, die von X oder Y gebildet wird, wodurch man die Produkte mit der Formel (I) erhält, in der X oder Y für einen Alkoxyrest stehen,

c) Reduktion der Doppelbindung des Pyrrolkerns des Indolrests, um Produkte mit der Formel (I) zu erhalten, in der a, b, c und d jeweils für ein Wasserstoffatom stehen,

d) Halogenierung in $\beta$-Stellung des Stickstoffatoms am Pyrrolkern des Indolrests, gefolgt von einer Hydrolyse im sauren Milieu, um die Produkte mit der Formel (I) zu erhalten, in der a und b

eine Oxofunktion bilden,

e) Auftrennung der racemischen Produkte mittels klassischer Verfahren, um die optisch aktiven Produkte zu erhalten, und, wenn erwünscht, Salzbildung der Produkte mit der Formel (I), um die entsprechenden Salze zu erhalten.

**2.** Verfahren nach Anspruch 1 zur Herstellung der Produkte mit der Formel (I) wie in Anspruch 1 definiert, die der Formel (I') entsprechen:

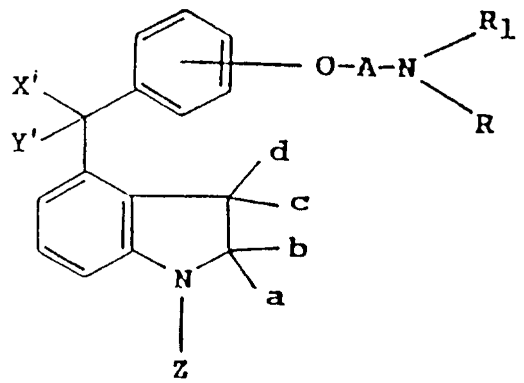

(I')

in der R, $R_1$, A, a, b, c, d und Z die in Anspruch 1 angegebene Bedeutung haben und X' und Y' so beschaffen sind, daß:

- entweder jedes für ein Wasserstoffatom steht,
- oder eines für ein Wasserstoffatom steht und das andere für einen Hydroxylrest steht,
- oder X und Y zusammen einen Oxorest bilden,

dadurch gekennzeichnet, daß man das Produkt mit der Formel (VI) oder der Formel (VII) wie in Anspruch 1 definiert einer Kondensationsreaktion an der Hydroxylgruppe unterwirft, und zwar

- entweder mit dem Produkt mit der Formel (X):

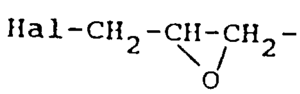

(X)

wodurch man das Produkt mit der Formel (XI') erhält:

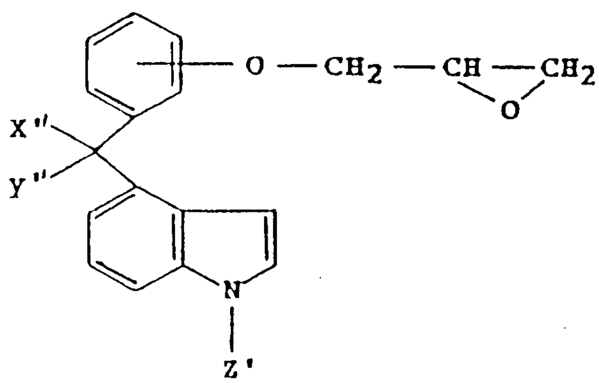

(XI')

in der X'' und Y'' für ein Wasserstoffatom stehen oder zusammen einen Oxorest bilden,

- oder mit dem Produkt mit der Formel (XII):

Hal-A'-Hal (XII)

in der A' für $-(CH_2)_n$ steht, wodurch man das Produkt mit der Formel (XIII') erhält:

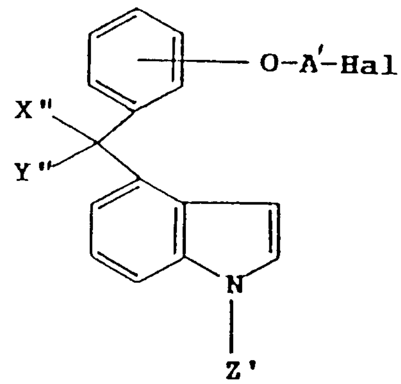

(XIII')

in der A', Z', X' und Y' die vorher angegebene Bedeutung haben, und daß man die Produkte mit der Formel (XI) oder (XIII) einer Additionsreaktion mit dem Amin mit folgender Formel (XIV) unterwirft:

$NH-(R)(R_1)$ (XIV)

in der R und $R_1$ die oben angegebene Bedeutung haben, wodurch man die Produkte mit der Formel (XV') erhält:

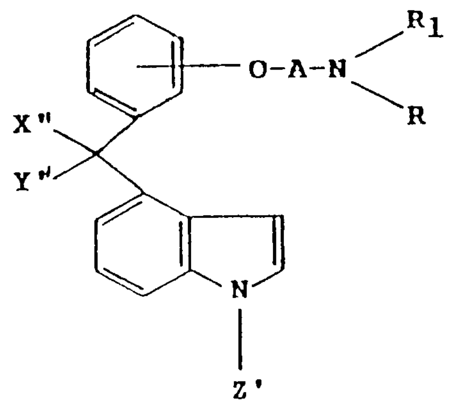

(XV')

die man, wenn nötig und wenn erwünscht, in beliebiger Reihenfolge einer oder mehreren der folgenden Reaktionen unterwirft:
a) Abtrennung von Z' oder der Schutzgruppen, die Z' trägt, um Z zu erhalten,
b) Reduktion der Oxofunktion, die von X' und Y' gebildet wird, zu einer Alkoholfunktion,
c) Reduktion der Doppelbindung des Pyrrolkerns des Indolrests, um Produkte mit der Formel (I) zu erhalten, in der a, b, c und d jeweils für ein Wasserstoffatom stehen,
d) Halogenierung in $\beta$-Stellung des Stickstoffatoms am Pyrrolkern des Indolrests, gefolgt von einer Hydrolyse im sauren Milieu, um die Produkte mit der Formel (I) zu erhalten, in der a und b eine Oxofunktion bilden,
e) Auftrennung der racemischen Produkte mittels klassischer Verfahren, um die optisch aktiven Produkte zu erhalten, und, wenn erwünscht, Salzbildung der Produkte mit der Formel (I), um die entsprechenden Salze zu erhalten.

3. Verfahren nach Anspruch 1, dadurch gekennzeichnet, daß man anfangs ein Produkt mit der Formel (II) wie in Anspruch 1 definiert verwendet, in der Z' für eine Schutzgruppe oder einen Alkylrest mit 1 bis 4 Kohlenstoffatomen steht, und dadurch, daß man das entsprechende Produkt mit der Formel (XV) gegebenenfalls einer Abtrennreaktion von Z' unterwirft.

4. Verfahren nach Anspruch 1 zur Herstellung der Produkte mit der Formel (I) wie in Anspruch 1 definiert, in der R ein Wasserstoffatom ist, $R_1$ für einen Alkylrest mit 1 bis 4 Kohlenstoffatomen oder für einen

Cycloalkylrest mit 3 bis 7 Kohlenstoffatomen steht oder R und $R_1$ zusammen mit dem Stickstoffatom, an das sie gebunden sind, einen Heterozyklus bilden, der ein zweites Stickstoffatom enthalten kann, das gegebenenfalls mit einem Alkylrest mit 1 bis 5 Kohlenstoffatomen substituiert ist, und A so beschaffen ist, daß die ganze Zahl n den Wert 2 oder 3 hat oder A für die Gruppe

$$-(CH_2)-\underset{\displaystyle \backslash \atop \displaystyle OH}{CH}-(CH_2)-$$

steht,
dadurch gekennzeichnet, daß man die Kondensationsreaktion an der Hydroxylgruppe entweder mit dem Produkt mit der Formel (X) wie in Anspruch 1 definiert durchführt, oder mit dem Produkt mit der Formel (XII) wie in Anspruch 1 definiert, in der A' für eine $(CH_2)_n$-Gruppierung steht, in der n den Wert 2 oder 3 hat, und dadurch daß man das Produkt mit der Formel (XI) oder dem entsprechenden mit der Formel (XIII) der Einwirkung eines Amins mit der Formel (XIV) unterwirft, in der R und $R_1$ die oben angegebenen Bedeutungen haben.

**5.** Verfahren nach Anspruch 1, dadurch gekennzeichnet, daß man Produkte mit der Formel (I) wie in Anspruch 1 definiert herstellt, in der a, b, c, d so beschaffen sind, daß eines von a oder b mit einem von c oder d eine zweite Kohlenstoff-Kohlenstoff-Bindung bildet und die anderen für ein Wasserstoffatom stehen und in der a und b zusammen einen Oxorest bilden und c und d jeweils für ein Wasserstoffatom stehen.

**6.** Verfahren gemaß Anspruch 1, dadurch gekennzeichnet, daß man irgendeines der Produkte mit der Formel (I) wie in Anspruch 1 definiert mit folgenden Namen herstellt:
- das [2-[3-[(1,1-Dimethylethyl)-amino]-2-hydroxypropoxy]-phenyl]-(1H-indol-4-yl)-methanon,
- das [2-[3-[(1,1-Dimethylethyl)-amino]-propoxy]-phenyl]-(1H-indol-4-yl)-methanon,
- das 1-[(1,1-Dimethylethyl)-amino]-3-[2-[(1H-indol-4-yl)-methyl]-phenoxy]-2-propanol,
- das (±)-[2-[3-[(1,1-Dimethylethyl)-amino]-2-hydroxypropoxy]-phenyl]-(1-methyl-1H-indol-4-yl)-methanon,
- das 1,3-Dihydro-4-[2-[3-[(1,1-Dimethylethyl)-amino]-2-hydroxypropoxy]-benzoyl]-2H-indol-2-on,
- das (±)-3-[(1,1-Dimethylethyl)-amino]-1-[[2-(1H-indol-4-yl]-ethenyl]-phenoxy]-2-propanol,
- das N-(1,1-Dimethylethyl)-3-[2-[1-(1H-indol-4-yl)-ethenyl]-phenoxy]-propanamin,
- das α-[2-[3-[(1,1-Dimethylethyl)-amino]-2-hydroxypropoxy]-phenyl-1H-indol-4-methanol,

sowie ihre Additionssalze mit den Mineralsäuren oder den organischen Säuren.

**7.** Verfahren gemäß Anspruch 1, dadurch gekennzeichnet, daß mit ein beliebiges Produkt mit der Formel (I') wie in Anspruch 2 definiert, mit folgenden Namen herstellt:
- das [2-[3-[(1,1-Dimethylethyl)-amino]-2-hydroxypropoxy]-phenyl]-(1H-indol-4-yl)-methanon,
  das [2-[3-[(1,1-Dimethylethyl)-amino]-propoxy]-phenyl]-(1H-indol-4-yl)-methanon,
- das 1-[(1,1-Dimethylethyl)-amino]-3-[2-[(1H-indol-4-yl)-methyl]-phenoxy]-2-propanol,
- das α-[2-[3-[(1,1-Dimethylethyl)-amino]-2-hydroxypropoxy]-phenyl-1-H-indol-4-methanol,

sowie ihre Additionssalze mit den Mineralsäuren oder den organischen Säuren.

**8.** Verfahren zur Herstellung pharmazeutischer Zusammensetzungen, dadurch gekennzeichnet, daß man als Wirkstoff wenigstens eines der Produkte mit der Formel (I) wie in Anspruch 1 definiert einsetzt oder wenigstens eines ihrer Additionssalze mit den pharmazeutisch verwendbaren Mineralsäuren oder organischen Säuren in einer für diesen Gebrauch bestimmten Form.

**9.** Verfahren zur Herstellung pharmazeutischer Zusammensetzungen, dadurch gekennzeichnet, daß man als Wirkstoff wenigstens eines der Produkte mit der Formel (I') wie in Anspruch 2 definiert einsetzt oder wenigstens eines ihrer Additionssalze mit den pharmazeutisch verwendbaren Mineralsäuren oder organischen Säuren in einer für diesen Gebrauch bestimmten Form.

**10.** Verfahren zur Herstellung pharmazeutischer Zusammensetzungen, dadurch gekennzeichnet, daß man als Wirkstoff wenigstens eines der Produkte mit der Formel (I) wie in Anspruch 8 definiert einsetzt oder wenigstens eines ihrer Additionssalze mit den pharmazeutisch verwendbaren Mineralsäuren oder

organischen Säuren in einer zu diesem Zweck bestimmten Form.

**11.** Die Zwischenprodukte, die den Formeln (IV), (V), (VI), (VIII), (VIII'), (IX), (XI) und (XIII) wie in Anspruch 9 definiert entsprechen.